# EUROPEAN PATENT SPECIFICATION

(11) **EP 4 201 951 B1**
(45) Date of publication and mention of the grant of the patent: **22.11.2023**
(21) Application number: 21216608.6
(22) Date of filing: 21.12.2021
(51) Int. Cl.: C07K 1/10, C07K 5/103, C07K 7/06

(54) **METHOD FOR PEPTIDE SYNTHESIS**
VERFAHREN ZUR PEPTIDSYNTHESE
PROCÉDÉ DE SYNTHÈSE DE PEPTIDES

(43) Date of publication of application: 28.06.2023
(73) Proprietor: Bachem Holding AG, 4416 Bubendorf (CH)
(72) Inventor: Kümin, Michael, 4416 Bubendorf (CH); Schönleber, Ralph O., 4416 Bubendorf (CH); Luther, Anatol, 4416 Bubendorf (CH)
(74) Representative: Jacobi, Markus Alexander

(56) References cited:
- EP-A2- 2 311 786
- WO-A1-01/29065
- US-A- 5 773 575
- US-A1- 2020 377 548
- US-A1- 2020 399 339
- RAMON SUBIRÓS-FUNOSAS ET AL: "Use of Oxyma as pH modulatory agent to be used in the prevention of base-driven side reactions and its effect on 2-chlorotrityl chloride resin", BIOPOLYMERS, JOHN WILEY, HOBOKEN, USA, vol. 98, no. 2, 31 August 2011 (2011-08-31), pages 89-97, XP071032435, ISSN: 0006-3525, DOI: 10.1002/BIP.21713

## Description

The present invention generally pertains to the field of peptide synthesis at an industrial or laboratory scale. Improved methods for the synthesis of peptides are disclosed. The present invention is directed to methods of effectively synthesizing peptides, thereby suppressing unwanted side reactions, in particular epimerization and double insertion. Additional aspects of the present invention relate to compositions for peptide synthesis.

Efficient methods for the manufacture of peptides of high purity at an industrial or laboratory scale are of high pharmaceutical and commercial interest.

Chemical peptide synthesis in general is well-known in the art. The key element of peptide synthesis is the typically iterative formation of an amide bond between a free amino group of an amino acid or peptide and a free carboxyl group of another amino acid or peptide (also referred to as coupling, coupling reaction or coupling step). Typically, the coupling step is a condensing reaction between the amino group and the carboxyl group. Said amide bond formation should be favored over competing side reactions and may typically require the activation of the carboxyl group.

Two standard approaches to chemical peptide synthesis may be distinguished, namely solid phase peptide synthesis (SPPS) and liquid phase peptide synthesis (LPPS). In addition, hybrid approaches may be utilized, wherein typically, fragments may first be synthesized by one of the above techniques, preferably SPPS, and then joined together using the other approach, preferably LPPS. This strategy may be employed for large (i.e. long) peptides with challenging sequences. An interesting variation of the above themes are forms of support-assisted peptide synthesis, where a (hydrophobic) solubility-modifying support is used to isolate the growing peptide chain from other components of the reaction mixture by means of extraction or precipitation steps. This allows to carry out the coupling reaction in a liquid phase. Hence, support-assisted peptide synthesis may be either a form of SPPS or of LPPS.

In the aforementioned support-assisted approaches, the growing peptide chains may typically not be cleaved from the support until the desired peptide sequence has been completely assembled. Hence, multiple different support-bound side products may accumulate over the course of the performed condensing steps and render the purification of the final product quite tedious and challenging.

It is therefore paramount to avoid side reactions during each of the coupling steps. In particular, the formation of side products, which are structurally similar to the final product and hence difficult to separate, is to be suppressed. Such side products may typically differ from the target peptide due to addition or omission of a single amino acid subunit or due to epimerization at the C-alpha position of an amino acid moiety. Even trace impurities (e.g., impurities comprised in an amount of less than 5%, 4%, 3%, 2%, or even 1% as compared to the target peptide) of such side products are to be avoided or reduced, since they may be hard to remove later on. Thus, the development of methods for peptide synthesis that (essentially) avoid or reduce the formation of such side products is highly desirable. A vast amount of studies and protocols address the choice of coupling reagents in the synthesis of peptides and peptide conjugates (cf., e.g., Subirós-Funosas et al. Biopolymers (Pept. Sci.) 2012, 98(2), 89-97; Shi et al., Synlett 2003, 5, 647-650).

Epimerization of a peptide is the inversion of a stereocenter of said peptide and may for example occur during the condensation step, when the stereocenter at the C-alpha carbon atom of a coupled amino acid moiety is inverted. Epimerization at the C-alpha atom may in principle occur in any coupling of a chiral amino acid moiety having one hydrogen substituent at the C-alpha atom. Herein, double-insertion is the undesired two-fold coupling of an amino acid or peptide into a peptide during a coupling step (i.e. after the desired condensation reaction, the desired product engages in another undesired condensation reaction to yield a double insertion product). Certain amino acid moieties may be particularly prone to epimerization and/or double insertion during peptide synthesis. Examples of such amino acids or amino acid moieties comprise histidine (a histidyl moiety), cysteine (a cystidyl moiety), alpha-arylglycines (an alpha-arylglycyl moiety) such as alpha-phenylglycine (an alpha-phenylglycyl moiety), and alpha-heteroarylglycines (an alpha-heteroarylglycyl moiety).

While histidine double insertion has rarely been addressed in the prior art, strategies for reducing histidine epimerization during peptide synthesis have been investigated for decades (see for example: M. Mergler et al., "Coupling Methods for Fmoc-His(Trt)-OH Resulting in Minimal Racemization", Solid Phase Synthesis & Combinatorial Libraries, 7th International Symposium & Exhibition, September 18-22, Southampton, UK, Abstract (2001)). For instance, it has been proposed to use histidine derivatives with a trityl-protected instead of urethane-protected alpha-amino group. However, these trityl derivatives couple rather slowly due to the bulkiness of the trityl group. A second approach involves histidine derivatives with a protected N^{π}-atom (in 3-position of the imidazole ring), wherein, for example, the tert-butyloxymethyl (Bum) and 4-methoxybenzyloxymethyl (MBom) protecting groups have been suggested. However, the use of these derivatives is limited by their availability and price, especially when considering industrial applications. Furthermore, the removal of the Bum and MBom protecting groups may be difficult. Pawlas reports an optimized coupling protocol for histidine using N,N'-diisopropylcarbodiimide (DIC) as coupling agent, alongside N-hydroxy-5-norbornene-2,3-dicarboxylimide (HONB) as additive (Proceedings of the 34th European Peptide Symposium, no PPI-034).

There is still an ongoing need for improved methods of peptide synthesis, where amino acid epimerization, as well as double insertion and/or omission of amino acid subunits is reduced. The present invention addresses this problem.

In one aspect, the present invention provides a method for the synthesis of a target peptide comprising a first cycle peptide P-1, wherein the method comprises the following steps:
(a) providing a first component C-1 being selected from the group consisting of an amino acid and a peptide and comprising one free primary or secondary aliphatic amino group, wherein any other primary or secondary aliphatic amino groups and any carboxyl groups in C-1 are protected,
(b) providing a second component C-2 being selected from the group consisting of a chiral alpha-amino acid and a peptide and comprising one free carboxyl group, which is the alpha-carboxyl group of a chiral alpha-amino acid moiety comprising one hydrogen-substituent bonded to the alpha-carbon atom, wherein any primary or secondary aliphatic amino groups and any other carboxyl groups in C-2 are protected, and
(c) condensing the first component C-1 of step (a) and the second component C-2 of step (b) in the presence of:
   - a carbodiimide coupling agent,
   - a first additive A-1 of formula A-I: 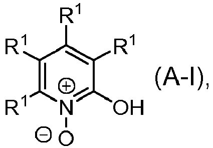 wherein in formula A-I,
      R¹ is at each occurrence independently selected from the group consisting of hydrogen, C₁-C₅-alkyl, and an electron-withdrawing substituent,
   - a second additive A-2 selected from the group consisting of ethyl 2-cyano-2-(hydroxyimino)acetate (Oxyma), 1-hydroxybenzotriazole (HOBt), 1-hydroxy-7-azabenzotriazole (HOAt), 5-(hydroxyimino)-1,3-dimethylpyrimidine-2,4,6(1H,3H,5H)-trione (Oxyma-B), and ethyl 1-hydroxy-1,2,3-triazole-4-carboxylate (HOCt), or mixtures thereof, and
   - a first cycle solvent,
   so as to form an amide bond between the free primary or secondary aliphatic amino group of the first component C-1 and the free alpha-carboxyl group of the second component C-2, to obtain a first cycle peptide P-1,
   wherein in step (c), the second additive A-2 is used in a total molar amount of 1.0 to 5.0 equivalents relative to the total molar amount of the first component C-1.

It will be understood by those skilled in the art that the first additive A-1 of formula A-I may be present as a tautomer of the structure of formula A-I, in particular as a tautomer of the following formula A-I': 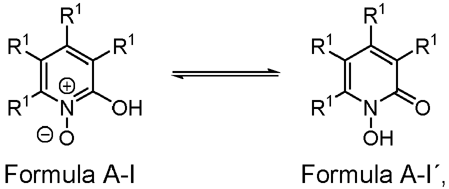 wherein, in formula A-I' the same definitions of R¹ apply as for formula A-I. In fact, said tautomerism is well-known and stating that a compound has a structure of formula A-I (i.e. is an optionally substituted 2-hydroxypyridine N-oxide) unambiguously implies that said compound may also be present as a tautomer of the formula A-I' (i.e. an optionally substituted 1-hydroxy-2-pyridone) without further notice. It will be understood that a single molecule of the first additive A-1 may (typically) only be present in a single tautomeric form at a certain point in time. Thus, when stating that said first additive A-1 may be present as a mixture of tautomers, this may refer to a population of molecules of said first additive A-1, out of which some molecules may be present in one and some molecules may be present in the other tautomeric form. For the method disclosed herein, it may not be relevant, which tautomeric form may be present in excess. In general, herein, when stating that a compound (e.g. a first component C-1, a second component C-2, a carbodiimide, a first additive A-1, or a second additive A-2, and the like) is of a certain chemical structure, this may embrace tautomers of said certain chemical structure, unless indicated differently.

In the context of the first additive A-1, the term "electron-withdrawing substituent" may refer to any substituent which decreases the electron-density in the pyridine ring of the first additive A-1 of formula A-I. Electron-withdrawing substituents (also referred to as deactivating substituents) for aromatic or hetroaromatic residues such as pyridine form part of the common knowledge of those skilled in the art, who know that such substituents will typically display a negative inductive effect (-I effect). Thus, such electron-withdrawing substituents will typically comprise at least one atom of an element having an electronegativity larger than that of carbon. Since atoms of such elements typically have one or more non-bonding electron pairs (also referred to as lone pairs), they may typically act as so-called +M substituents, i.e. induce a positive mesomeric effect when being directly bonded to an aromatic or heteroaromatic ring. Thus, as known to those skilled in the art, in practice, the positive mesomeric effect (increasing electron density of the aromatic or heteroaromatic residue or ring) and the negative inductive effect (decreasing electron density of the aromatic or heteroaromatic residue or ring) compete with each other. A person skilled in the art knows that, typically, for halogen substituents, in particular F-, C!-, and Br-substituents, the negative inductive effect outweighs the positive mesomeric effect, so that these substituents are examples of electron-withdrawing substituents which may be used as R¹ in formula A-I. Halogens may however not only be bonded directly to the aromatic or heteroaromatic residue to achieve a reduction of electron density. As known to those skilled in the art, halogenated, in particular fluorinated, hydrocarbon substituents such as e.g. the frifluoromethyl or the pentafluoroethyl group are well-established examples of electron-withdrawing substituents and may also be used as R¹ in formula A-I. For other heteroatoms such as oxygen and nitrogen, the positive mesomeric effect will typically outweigh the negative inductive effect, so that electron-withdrawing substituents typically do not bond to the aromatic or heteroaromatic ring via oxygen or nitrogen. In fact, a person skilled in the art knows that heteroatoms other than the aforementioned halogens may best be bonded to a carbon atom which is directly attached to the aromatic or heteroaromatic ring. Thus, further example, electron-withdrawing substituents which may be used as R¹ in formula A-I may be ester or amide groups attached via the carboxyl-carbon atom, acyl groups attached via the carbonyl-carbon atom, and nitrile groups attached via the carbon atom. A well-known exception is the nitro group, which is attached via nitrogen, but still an electron-withdrawing substituents which may be used as R¹ in formula A-I.

In some embodiments, R¹ of formula A-I is at each occurrence independently selected from the group consisting of hydrogen, C₁-C₅-alkyl, F, Cl, Br, -CF₃, -C₂F₅, -CN, -C(=O)OR^{EWG}, -C(O)N(R^{EWG})₂, -C(O)^{REWG}, and -NO₂, wherein R^{EWG} is at each occurrence independently selected from the group consisting of hydrogen, C₁-C₅-alkyl and phenyl.

In some embodiments, R¹ of formula A-I is at each occurrence independently selected from the group consisting of hydrogen, C₁-C₅-alkyl, F, Cl, Br, -CF₃, -C₂F₅, -CN, -C(=O)OR^{EWG}, -C(O)N(R^{EWG})₂, -C(O)R^{EWG}, and -NO₂, wherein R^{EWG} is at each occurrence independently selected from the group consisting of hydrogen, C₁-C₅-alkyl and phenyl, with the proviso that at least two residues R¹, preferably at least three residues R¹, are hydrogen.

In some embodiments, R¹ of formula A-I is at each occurrence independently selected from the group consisting of hydrogen, C₁-C₅-alkyl, F, Cl, Br, -CF₃, -C₂F₅, -CN, -C(=O)OR^{EWG}, -C(O)N(R^{EWG})₂, -C(O)R^{EWG}, and -NO₂, wherein R^{EWG} is at each occurrence independently C₁-C₅-alkyl, with the proviso that at least two residues R¹, preferably at least three residues R¹, are hydrogen.

In some embodiments, R1 of formula A-I is at each occurrence independently selected from the group consisting of hydrogen, C₁-C₅-alkyl, F, Cl, Br, -CF₃, -CN, -C(=O)OR^{EWG}, -C(O)N(R^{EWG})₂, and -C(O)R^{EWG}, wherein R^{EWG} is at each occurrence independently selected from the group consisting of -CH₃ (methyl, Me) and -CH₂CH₃ (ethyl, Et), with the proviso that at least two residues R¹, preferably at least three residues R¹, are hydrogen.

In some embodiments, R¹ of formula A-I is at each occurrence independently selected from the group consisting of hydrogen, C₁-C₅-alkyl, F, Cl, Br, -CF₃, -CN, and -C(=O)OR^{EWG}, wherein R^{EWG} is at each occurrence independently selected from the group consisting of -CH₃ (methyl, Me) and -CH₂CH₃ (ethyl, Et), with the proviso that at least two residues R¹, preferably at least three residues R¹, are hydrogen.

In some embodiments, R¹ of formula A-I is at each occurrence independently selected from the group consisting of hydrogen, C₁-C₅-alkyl, F, Cl, and Br, with the proviso that at least two residues R¹, preferably at least three residues R¹, are hydrogen..

In some embodiments, R¹ of formula A-I is at each occurrence independently selected from the group consisting of hydrogen, F, Cl, and Br, with the proviso that at least two residues R¹, preferably at least three residues R¹, are hydrogen.

In some embodiments, R¹ of formula A-I is at each occurrence independently selected from the group consisting of hydrogen and Br, with the proviso that at least two residues R¹, preferably at least three residues R¹, are hydrogen.

In some embodiments, R¹ of formula A-I is at each occurrence hydrogen.

As used herein, the term "peptide" may refer to a compound in which at least one amino acid is covalently bonded to another amino acid (having the same or a different chemical structure) via a peptide bond. As used herein, the term "peptide bond" may refer to an amide bond obtainable from the condensation of an amino group of one amino acid and a carboxyl group of another amino acid (having the same or a different chemical structure). An amino acid comprised in a peptide is herein also referred to as "amino acid subunit". The term "amino acid moiety" may refer to a free amino acid or to an amino acid subunit of a peptide.

The order of amino acid subunits of a peptide is herein referred to as the "amino acid sequence", "peptide sequence", or simply "sequence" of the respective peptide. As used herein, the sequence of a peptide is defined by the nature (i.e. the chemical structure) of the aligned amino acid subunits and the order in which they are aligned, wherein any protecting groups optionally bonded to the amino acid subunits are not a characterizing feature of an amino acid sequence as defined herein. This is to say that such protecting groups may or may not be present and may have the same or different chemical structures without impacting the amino acid sequence of a peptide. For example, as used herein, a peptide being covalently linked to a support, which may be perceived as a permanent protecting group, and having one or more protecting groups at side chain residues of amino acid subunit(s) and/or the N-terminal amino group is considered to have the same sequence (i.e. amino acid sequence or peptide sequence) as a peptide which consists of the same alignment of amino acids, but does no longer comprise one or more of the protecting group(s) and/or is cleaved from the support.

A peptide may comprise 2-102 amino acid subunits in total.

A peptide may be a linear peptide. A linear peptide, as used herein, consists of two terminal amino acid moieties, which are each bonded to exactly one other amino acid moiety, and optionally one or more non-terminal amino acid moieties, which are each bonded to exactly two other amino acid moieties. Typically, one of the terminal amino acids will have an amino group, preferably an alpha-amino group, which is referred to as the N-terminal amino group or N-terminus of the peptide, and the other terminal amino acid will have a carboxyl group, preferably an alpha-carboxyl group, which is referred to as the C-terminal carboxyl group or C-terminus of the peptide. In some embodiments, all bonds between amino acid moieties of the peptides are peptide bonds. In some embodiments, a peptide may comprise only alpha-amino acid moieties and derivatives thereof. A peptide may be a peptide which comprises only alpha-amino acids, wherein these alpha-amino acids may optionally bear one or more protecting groups. In some embodiments, all peptide bonds of a peptide are constructed from alpha-amino groups and alpha-carboxyl groups of alpha-amino acid subunits. Such a peptide may have a backbone that is composed of the (linearly) aligned backbones of the amino acid subunits comprised in said peptide.

A peptide may be branched. This is to say that one or more, preferably one, functional group of a first peptide (chain) may be bonded to one or more, preferably one, functional group of another peptide (chain), so as to interconnect the two peptide chains. Hence, a branched peptide comprises at least one amino acid moiety (e.g. a Lys, Glu, or Asp moiety), which is bonded to three, other amino acid moieties. A peptide may be a cyclic peptide, which exhibits a covalent bond between two functional groups located on different parts of the peptide chain. For example, cyclic peptides may result from disulfide bridge formation or from head to tail cyclisation.

A peptide may be conjugated (i.e. a conjugated peptide or peptide conjugate) to a species that is not necessarily a peptide, in particular to another biomolecule selected from the group consisting of a carbohydrate (i.e. a mono-, oligo-, or polysaccharide) and one or more fatty acids, preferably, a fatty acid. The conjugation may for example occur at a functional group of a side chain residue of the peptide or at the C-terminal carboxyl group or the N-terminal amino group of the peptide. The conjugation may also occur via one or more linker molecules. As in liraglutide, an amino acid having a carboxyl group in its side chain (e.g. glutamic acid) may be used as such a linker molecule, for example when forming an amide bond between said side chain carboxyl group and a side chain amino group of an amino acid subunit (e.g. a lysine subunit) of the peptide. The C-terminal carboxyl and/or the N-terminal amino group (as in liraglutide) of the amino acid linker molecule (for example glutamic acid) may then engage in chemical bonding to the biomolecule that is to be conjugated, for example in form of an amide bond to a fatty acid. Besides liraglutide, examples of peptide conjugates are semaglutide or N-palmitoyl-peptides as for example used in cosmetics.

A peptide may be attached to a support. In this case, the respective peptide may be referred to as a support-bound peptide. In such cases, it may be preferred that the respective peptide is covalently linked to the support via a carboxyl group of an amino acid subunit, in particular via the alpha-carboxyl group of the C-terminal amino acid (i.e. via the C-terminal carboxyl group).

As used herein, the expression "target peptide" refers to any specific peptide which is to be synthesized according to the present invention. In other words, the "target peptide" is generally the final peptide product of the synthesis method. By contrast, the intermediate peptide products of subsequent coupling cycles are referred to as "first cycle peptide P-1", "second cycle peptide P-2" and so on, optionally up to the "(n+1)-th cycle peptide P-(n+1)" where n is an integer from 1 to 100, e.g. from 1 to 50 or from 5 to 30. Hence, the amino acid sequence of the "target peptide" will normally comprise the amino acid sequences of each of the intermediate peptide products. A target peptide may be linear, cyclic, branched and/or chemically modified. As used herein, the expression "target peptide" comprises specific variants of the target peptide, which have the same amino acid sequence, but may, e.g., be purified, be modified by protecting groups, be bound to a support, or bear specific modifications. For example, the target peptide may be modified by conjugation. Hence, the expressions "support-cleaved target peptide", "purified support-cleaved target peptide", "modified target peptide", and "purified modified target peptide" refer to certain variants of target peptides.

Typical target peptides comprise glucagon-like peptides such as exenatide, liraglutide and semaglutide, atosiban, buserelin, desmopressin, glucagon, gonadorelin, goserelin, lanreotide, leuprorelin, octreotide, somatostatin, teriparatide, Tetracosactide, Triptorelin, and Argipressin.

In some embodiments, the target peptide comprises only amino acid moieties, preferably only alpha-amino acid moieties. In some embodiments, the target peptide comprises amino acid moieties and fatty acid moieties. In some embodiments, the target peptide is a branched peptide.

The method of the invention is a "method for the synthesis of a target peptide comprising a first cycle peptide P-1". The expression "the target peptide comprises a first cycle peptide P-1" may mean that the target peptide comprises the amino acid sequence of said first cycle peptide P-1. As laid out above, in order for the target peptide to comprise (or share) the amino acid sequence of the first cycle peptide P-1, it is not required that the amino acid subunits of said amino acid sequence carry the same protecting groups in the first cycle peptide P-1 and the target peptide. For example, certain amino acid subunits (or moieties) may be protected in the first cycle peptide P-1 and may not or not all be protected in the target peptide or vice versa. It will be understood that, in case the target peptide comprises more amino acid subunits than the first cycle peptide P-1, the end groups of the shared amino acid sequence may not be identical. In this case, for example, the alpha-amino group of the N-terminal amino acid of P-1's amino acid sequence may be engaged in an amide bond (i.e. present as an amide group) in the target peptide and/or the alpha-carboxyl group of the C-terminal amino acid of P-1's amino acid sequence may be engaged in an amide bond (i.e. present as an amide group) in the target peptide. In some embodiments, the first cycle peptide P-1 and the target peptide which is to be synthesized by the method disclosed herein consist of the same amino acid sequence.

As laid out above, step (a) of the method of the present invention is: "providing a first component C-1 being selected from the group consisting of an amino acid and a peptide and comprising one free primary or secondary aliphatic amino group, wherein any other primary or secondary aliphatic amino groups and any carboxyl groups in C-1 are protected".

In some embodiments, the first component C-1 is an alpha-amino acid with a protected alpha-carboxyl group and a free alpha-amino group, wherein said alpha-amino acid may optionally bear one or more further protecting groups. Herein, unless indicated differently in specific embodiments, the term "protecting group" embraces a support as defined herein. In some embodiments, the first component C-1 is covalently linked to a solid support. In some embodiments, the first component C-1 is a peptide as defined herein above. In other words: all of the previous explanations relating to a peptide in general are applicable to the first component C-1 in as far as it is a peptide.

As used herein, the term "amino acid" may refer to a molecule comprising at least one carboxyl group and at least one amino group, preferably a primary or secondary amino group, more preferably a primary or secondary aliphatic amino group, wherein all of these groups may be protonated, deprotonated or protected (i.e. bonded to a suitable protecting group, including a support). In general, the term "amino acid" forms part of the common knowledge of those skilled in the art and is understood without further explanation. Unless indicated differently in the context of specific embodiments, the term "amino acid" herein embraces for example the so called- alpha-, beta-, gamma-, and delta-amino acids, wherein alpha-amino acids are most common and herein most preferred. In some embodiments, an amino acid is an alpha-amino acid. Alpha-amino acids are characterized in that a carboxyl group (the so-called alpha-carboxyl group) and an amino group (the so-called alpha-amino group) are both attached to the same carbon atom, which is referred to as the alpha-carbon (atom), or simply C-alpha. In alpha-amino acids, said alpha-carbon atom bears two further residues that are typically neither an amino group nor a carboxyl group, for example one hydrogen substituent and another residue, commonly referred to as the "side chain", or two hydrogen substituents (in the case of glycine).

As used herein, unless indicated differently in the context of specific embodiments, the term "amino acid" embraces all stereoisomeric forms of amino acids, in particular L-amino acids, and D-amino acids. Furthermore, herein, unless indicated differently in the context of specific embodiments, the term "amino acid" embraces naturally occurring amino acids, in particular the proteinogenic amino acids, unnatural amino acids, and derivatives of the aforementioned amino acids. The term "derivative" as used herein may be understood in the broadest sense and may refer to a compound that is derived from a parent compound by substitution or chemical modification or introduction or removal of an atom or atom group. Thus, the term "derivative" may typically refer to a compound which can be obtained from a parent compound by one or more, typically very few, or even exactly one, chemical reaction(s). As a result, a derivative may for example differ from the parent compound by the presence or absence of one or more substituents. The term "derivative" when referring to amino acids or peptides in particular embraces compounds that differ from the parent amino acid or peptide by comprising or not comprising one or more protecting groups, wherein again, a support may be regarded as a permanent protecting group. In some embodiments, an amino acid is an alpha-amino acid which may bear one or more protecting groups.

Examples of proteinogenic alpha-amino acids comprise in particular glycine (Gly, G), alanine (Ala, A), arginine (Arg, R), asparagine (Asn, N), aspartic acid (Asp, D), cysteine (Cys, C), glutamine (Gln, Q), glutamic acid (Glu, E), histidine (His, H), leucine (Leu, L), isoleucine (Ile, I), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V).

Examples of non-proteinogenic alpha-amino acids comprise, inter alia, alpha-aminoglycine (Agl), alpha,gamma-diaminobutyric acid (Dab), ornithine (Orn), alpha, beta-diaminopropionic acid (Dap), 2,6-diamino-4-hexynoic acid, beta-(1-piperazinyl)alanine, 4,5-dehydrolysine, delta-hydroxylysine (Hyl), omega, omega-dimethylarginine, omega,omega'-dimethylarginine, omega-methylarginine (NMA), 4-aminopiperidine-4-carboxylic acid, beta-(2-quinolyl)-alanine, alpha-methylhistidine, 1-methylhistidine (His(1-Me)), 3-methylhistidine (His(3-Me)), spinacine, 4-aminophenylalanine (Aph), 3-aminotyrosine, beta-(2-pyridyl)-alanine (2-Pal), beta-(3-pyridyl)-alanine (3-Pal), dehydroalanine (Dha), beta-fluoroalanine, beta-chloroalanine, beta-iodoalanine, alpha-aminobutyric acid (Abu), alpha-aminoisobutyric acid (Aib), beta-cyclopropylalanine, azetidine-2-carboxylic acid (Aze), alpha-allylglycine propargylglycine (Pra), tert-butylalanine (Tba), beta-(2-thiazolyl)alanine, thiaproline (Thz), 3,4-dehydroproline (Dhp), tert-butylglycine (Tle), beta-cyclopentylalanine (Cpa), beta-cyclohexylalanine (Cha), alpha-methylproline, norvaline (Nva), alpha-methylvaline, penicillamine (Pen), beta,beta-dicyclohexylalanine, 4-fluoroproline (Flp), 1-aminocyclopentanecarboxylic acid, pipecolic acid (Pip), 4,5-dehydroleucine, allo-isoleucine (alle), norleucine (Nle), alpha-methylleucine, cyclohexylglycine, cis-octahydroindole-2-carboxylic acid (Oic), beta-(2-thienyl)-alanine, alpha-phenylglycine (or phenylglycine, Phg), alpha-methyl-phenylalanine, homophenylalanine (Hph), 1,2,3,4-tetrahydroisoquinoline-3-carboxylic acid (Tic), β-(3-benzothienyl)-alanine (Bta), 4-nitrophenylalanine (Nph), 4-bromophenylalanine, 4-tert-butylphenylalanine, α-methyltryptophan, beta-(2-naphthyl)-alanine (2-Nal), beta-(2-naphthyl)-alanine (1-Nal), 4-lodophenylalanine, 3-fluorophenylalanine, 4-fluorophenylalanine, 4-methyltryptophan, 4-chlorophenylalanine (4-Cpa), 3,4-dichlorophenylalanine, 2,6-difluorophenylalanine, N-in-methyltryptophan, 1,2,3,4-tetrahydronorharman-3-carboxylic acid (Tpi), beta,beta-diphenylalanine (Dip), 4-methylphenylalanine, 4-phenylphenylalanine (Bip), 2,3,4,5,6-pentafluorophenylalanine, 4-benzoylphenylalanine (Bpa), beta-cyanoalanine, beta-ureidoalanine, homocysteine (Hcy), allo-threonine, pyroglutamic acid (Pyr), 2-oxothiazolidine-4-carboxylic acid (OTZ), citrulline (Cit), thiocitrulline, homocitrulline (Hci), hydroxyproline (Hyp), 3,4-dihydroxyphenylalanine (Dopa), beta-(1,2,4-triazol-1-yl)alanine, 2-mercaptohistidine, beta-(3,4-dihydroxyphenyl)serine, beta-(2-thienyl)serine, 4-azidophenylalanine (Pap), 4-cyanophenylalanine, 3-hydroxymethyltyrosine, 3-iodotyrosine, 3-nitrotyrosine, 3,5-dinitrotyrosine, 3,5-dibromotyrosine, 3,5-diiodotyrosine, 7-hydroxy-1,2,3,4-tetrahydroisoquinoline-3-carboxylic acid, 5-hydroxytryptophan, thyronine, beta-(7-methoxycoumarin-4-yl)alanine (Amp), 4-(7-hydroxy-4-coumarinyl)-aminobutyric acid (Cgl), gamma-hydroxyglutamic acid, gamma-methyleneglutamic acid, gamma-carboxyglutamic acid (Gla), alpha-aminoadipic acid (Aad), 2-aminoheptanedioic acid (Apm), alpha-aminosuberic acid (Asu), 4-carboxyphenylalanine, cysterici acid (Cya), 4-phosphonophenylalanine (Ppa), and 4-sulfomethylphenylalanine.

Herein, amino acids will typically be referred to interchangeably by either their full name (e.g. alanine), their 3-letter code according to WIPO Standard ST.25 (e.g. Ala), or their 1-letter code (e.g. A). Furthermore, an amino acid that forms part of a peptide may herein be referred to by a name derived from its full name in combination with the final syllable "-yl". This is to say that, optionally, for example, a histinide subunit of a peptide may be referred to as a histidyl subunit and a cysteine subunit of a peptide may optionally be referred to as a cystidyl subunit, and the like. Additionally, herein, the following notation may be used for amino acids and derivatives thereof, in particular for alpha-amino acids: Substituents at the (N-terminal) alpha-amino group are indicated to the left of the amino acid symbol and separated by a hyphen; Substituents at the (C-terminal) alpha-carboxyl group are indicated to the right of the amino acid symbol and separated by a hyphen; Substituents at the side chain (e.g. protecting groups) are indicated in brackets immediately to the right of the amino acid symbol. For amino acids with a free (unmodified) (N-terminal) alpha-amino group, the denoted substituent at the alpha-amino group is hydrogen and for alpha-amino acids with a free (unmodified) (C-terminal) alpha-carboxyl group, the denoted substituent at the alpha-carboxyl group is -OH. For example, H-His-OH would be used to denote histidine with a free alpha-amino group and a free alpha-carboxyl group. Along the same lines, histidine with an Fmoc-protected alpha-amino group and an alpha-carboxyl methyl ester would be denoted as Fmoc-His-OMe. As far as the enantiomeric form is not expressly specified, L-amino acids are in general referred to, unless indicated differently. It should be noted, however, that the present invention can likewise be put into practice using D-amino acids and other stereoisomers. Racemates may also be used (for example denoted as DL-His in the case of histidine), wherein the method of the present invention may still have the advantage of (essentially) avoiding or reducing double insertion. It need not be explained that when referring to an amino acid being present in a certain stereoisomeric form, such as the L-isomer, this may refer to a population of molecules of said amino acid, wherein the vast majority or even (essentially) all molecules of the population of molecules are present in the indicated isomeric form. For peptides, a similar notation may herein be used, wherein the amino acid subunits are separated by hyphens and wherein again: Substituents at the N-terminal alpha-amino group of the peptide are indicated to the left of the N-terminal amino acid symbol and separated by a hyphen; Substituents at the C-terminal alpha-carboxyl group of the peptide are indicated to the right of the C-terminal amino acid symbol and separated by a hyphen; Substituents at a side chain (e.g. protecting groups) are indicated in brackets immediately to the right of the respective amino acid symbol. For example, a dipeptide comprising Fmoc-protected histidine with a trityl protecting group in 1-positon of the side chain's imidazole ring as N-terminal amino acid and cysteine with a free alpha-carboxyl group and a trityl protecting group at the side chain's sulfur atom as C-terminal amino acid may be denoted as Fmoc-His(1-Trt)-Cys(Trt)-OH. In line with common practice, amino acids and peptides are herein denoted from the N- to the C-terminus, unless indicated differently.

An amino acid may (herein) be perceived as having a backbone and one or more, typically one, side chain. One end of said backbone is marked by an amino group, and the other end of said backbone is marked by a carboxyl group, which may (herein) be referred to as the N-terminal amino group and the C-terminal carboxyl group, respectively, in analogy to peptides. These are interconnected by one or more carbon atoms. In the case of classical alpha-amino acids, the backbone comprises the alpha-carbon atom, the alpha-carboxyl group, which may herein also be referred to as the C-terminal carboxyl group, and the alpha-amino group, which may herein also be referred to as the N-terminal amino group. In beta-amino acids, the backbone comprises a further carbon atom, and so on. Any residues other than hydrogen-substituents (or isotopes thereof) attached to a carbon atom of the backbone not forming part of the carboxyl group may herein be referred to as a side chain or side chain residue.

As used herein, the term "aliphatic amino group" may refer to a functional group comprising a nitrogen atom, wherein the nitrogen atom is only substituted by hydrogen (or an isotope thereof such as deuterium) and/or alkyl groups, with the proviso that the nitrogen atom is only engaged in single bonds and that the carbon atom with which any alkyl group bonds to said nitrogen atom is not the starting point of a chemical bond to a heteroatom other than said nitrogen atom of the amino group. In aliphatic amino groups as defined herein, the nitrogen atom may form part of an aliphatic cyclic group such as in morpholine, piperidine, pyrrolidine, and the like, but may not form part of an aromatic ring such as in indole, pyrrole, pyridine, imidazole, and the like. The amino group as substituent is bonded to the respective molecule via a direct covalent single carbon-nitrogen bond C-N. The terms "primary", "secondary", "tertiary", and "quaternary" may be used to denote the number of alkyl groups bonded to the nitrogen atom of an aliphatic amino group. In primary aliphatic amino groups, a single alkyl group is bonded to the nitrogen atom. In secondary aliphatic amino groups, two alkyl groups are bonded to the nitrogen atom. In tertiary aliphatic amino groups, three alkyl groups are bonded to the nitrogen atom. In quaternary aliphatic amino groups, four alkyl groups are bonded to the nitrogen atom. For example, the alpha-amino groups of histidine, cysteine, and 2-aminoisobutyric acid (Aib) are primary aliphatic amino groups and the alpha-amino groups of proline and N-methylalanine are secondary aliphatic amino groups. In a H-Pro-His-OH dipeptide for example, the N-terminal amino group (of proline) is still a secondary aliphatic amino group as defined herein, while the former alpha-amino group of histidine is now engaged in a peptide bond and no longer regarded as an amino group, since in a peptide bond, the nitrogen atom is directly bonded to a carbonyl group. As another example, the alpha-amino group of alpha-phenylglycine is a primary aliphatic amino group as defined herein, with the alpha-carbon representing the one alkyl substituent, in this case, a phenyl-substituted alkyl substituent. The same rationale applies to amino groups in the side chain of an amino acid. For example, the amino group in the side chain of lysine is a primary aliphatic amino group as defined herein, while the amide group in the side chain of for example glutamine and asparagine as well as the guanidino group in the side chain of for example arginine are not primary or secondary aliphatic amino groups as defined herein. Likewise, for example, the nitrogen atoms in the imidazole ring of the histidine side chain and the nitrogen atom of the indole ring in the tryptophan side chain are not considered primary or secondary aliphatic amino groups as defined herein.

It should be noted that, herein, an optionally attached protecting group is not considered when determining whether or not an amino group is a primary or secondary aliphatic amino group as defined herein. For example, proline (H-Pro-OH) and Fmoc-protected proline (Fmoc-Pro-OH) both comprise a secondary aliphatic amino group, whereat in proline, it would be a free secondary aliphatic amino group and in Fmoc-protected proline, it would be a protected secondary aliphatic amino group.

As used herein, in line with common practice, unless indicated differently in the context of specific embodiments, a "carboxyl group" may be understood as a -C(=O)OH (or -CO₂H) functional group or the deprotonated form thereof, wherein the hyphen indicates a covalent single bond attaching the carboxyl group to the respective molecule such as an amino acid. For example, the alpha-carboxyl groups of alpha-amino acids such as the proteinogenic alpha-amino acids as well as the carboxyl groups in the side chain residues of for example glutamic acid and aspartic acid are carboxyl groups as defined herein. In a H-Pro-His-OH dipeptide for example, the C-terminal carboxyl group (of histidine) is still a carboxyl group as defined herein, while the former alpha-carboxyl group of proline is now engaged in a peptide bond and no longer regarded as a carboxyl group.

Thus, in line with what has been laid out so far, when stating that the first component C-1 comprises "one free primary or secondary aliphatic amino group, wherein any other primary or secondary aliphatic amino groups and any carboxyl groups in C-1 are protected", this may mean that C-1 comprises one or more primary or secondary aliphatic amino groups as defined herein, out of which exactly one does not carry an amino protecting group as defined herein, i.e. a protecting group that prevents the respective amino group from partaking in the amide bond forming condensation reaction of step (c). Thus, the first component C-1 comprises only a single primary or secondary aliphatic amino group capable of engaging in the amide bond forming condensation reaction of step (c). Additionally, the first component C-1 may optionally comprise one or more carboxyl groups as defined herein, all of which are however protected by a carboxyl protecting group as defined herein, i.e. by a protecting group that prevents the respective carboxyl group from partaking in the amide bond forming condensation reaction of step (c). Thus, the first component C-1 does not comprises a carboxyl group capable of engaging in the amide bond forming condensation reaction of step (c).

In some embodiments, the free primary or secondary aliphatic amino group of the first component C-1 is an alpha-amino group of an alpha-amino acid moiety. As stated above, such an alpha-amino acid moiety may be an amino acid as such or an amino acid subunit of a peptide, in particular the N-terminal amino acid subunit of a peptide, since it comprises a free alpha-amino group. In some embodiments, the free primary or secondary aliphatic amino group of the first component C-1 is an alpha-amino group of an alpha-amino acid or the N-terminal amino group of a peptide.

The first component may be covalently linked to a support (i.e. bonded to a support via a covalent chemical bond). In such cases, the method of the invention may be a method for support-assisted peptide synthesis. In this context, herein, the term "support" may refer to any material or compound to which the growing peptide chains are attached, preferably throughout the entire synthesis (i.e. until the peptide has been assembled with a desired sequence). Herein, the terms "linked to a support", "attached to a support", "immobilized on a support", and "support-bound" may be used interchangeably. A support may be classified as soluble or insoluble.

Thus, herein, a support which is not soluble in the solvents used during peptide synthesis is referred to as a "solid support". Herein, the terms "solid support", "insoluble support", and "solid phase" may be used interchangeably. As used herein, a support which is soluble at least in the solvents used for the condensing, deprotecting, and cleavage steps is referred to as a "soluble support".

In some embodiments, the first component C-1 is covalently linked to a support. In some embodiments, the first component C-1 is covalently liked to a solid support. In some embodiments, the first component C-1 is covalently liked to a soluble support. The use of supports, in particular solid supports, is common practice in peptide synthesis and well-known to a person skilled in the art, who knows how to select and utilize such a support based on standard knowledge and detailed guidelines in the state of the art. In fact, any support commonly used for peptide synthesis may also be used as support in the method disclosed herein.

Non-limiting examples of solid supports suitable for use in the method of the present inventions comprise cross-linked polymer resins or gels, that may swell but not dissolve in the reaction medium. Such polymer resins may for example be based on polystyrene, polystyrene-PEG composites, PEG (polyethylene glycol), PEGA (polymers consisting of 2-acrylamidoprop-1-yl-(2-aminoprop-1-yl) polyethylene glycol₈₀₀ and dimethylacylamide cross-linked with bis 2-acrylamidoprop-1-yl polyethylene glycol₈₀₀), cross-linked ethoxylate acrylate (CLEAR), polyamides, polydimethylacrylamide, or any other support with the desired physical and chemical properties. Resins based on beaded polystyrene cross-linked with about 1% divinylbenzene are among the routinely used solid supports, typically having a size distribution of 200-400 mesh or 100-200 mesh. Polystyrene based 4-alkoxybenzyl alcohol (Wang) resin, diphenyldiazomethane (PDDM) resin, 4-(2',4'-dimethoxyphenyl-Fmoc-aminomethyl)-phenoxymethyl-polystyrene (Rink or Rink Amide) resin, 2-methoxy-4-alkoxybenzyl alcohol (Sasrin) resin, 2-chlorotrityl chloride (CTC) resin, and CTC-amidomethyl resin may for example be used in the method of the present invention and are commercially available from suppliers such as Sigma-Aldrich, Bachem, and EMD Millipore. The resins may typically be swollen in a suitable solvent such as DMF or the like. Solid supports, for use in the method of the invention, may typically be present in the form of polymer beads. A peptide synthesis during which the growing peptide chains are attached to a solid support is commonly referred to as solid phase peptide synthesis (SPPS). Thus, the method of the present invention may be a method for SPPS, if the first component C-1 is attached to a solid support.

Herein, a soluble support may also be referred to as tag, anchor molecule or carrier and may consist of a relatively simple organic molecule such as a benzylic alcohol or a benzylamine bearing large carbohydrate-based hydrophobic substituents, to which the growing peptide chain may be bonded (directly or via a linker). Such a tag may for example be selected so that it may modify the solubility of the growing peptide chain attached to it, for example to be soluble in organic solvents, but not in aqueous solutions, or even in water. The use of such a soluble support may for example allow to carry out the entire peptide synthesis in an organic medium and to remove applied reagents, excess reactants, and water-soluble byproducts or side products by means of aqueous extraction(s). A peptide synthesis during which the growing peptide chains are attached to a soluble support, may herein be referred to as tag-assisted or support assisted liquid phase peptide synthesis (tag-assisted or support assisted LPPS). Thus, the method of the present invention may be a method for tag-assisted LPPS, if the first component C-1 is attached to a soluble support. Non-limiting examples of such approaches to peptide synthesis include the Ajiphase^{™} system and the Molecular Hiving^{™} concept (see for example: D. Takahashi et al. Angew. Chem. Int. Ed. 2017, 56, 7803-7807; EP3778621A1). The soluble supports disclosed in these references may exemplarily also be used as soluble supports in the method disclosed herein.

In some embodiments, the first component C-1 is covalently linked to a support via the alpha-carboxyl group of an alpha-amino acid moiety. In some embodiment, the first component C-1 is covalently linked to a support via its C-terminal carboxyl group, which is an alpha-carboxyl group of an alpha-amino acid moiety. In some embodiments, the first component C-1 is covalently linked to a support via a side chain carboxyl group. The means of covalently linking an amino acid or a peptide such as the first component C-1 to a support form part of the common knowledge of those skilled in the art.

The term "providing a first component C-1" in step (a) of the method of the present invention may be understood in the broadest sense and may refer to any process of obtaining said first component C-1. The first component C-1 may be obtained by means of amino acid or peptide synthesis, wherein any synthetic strategies (i.e. synthetic approaches) may be applied. A person skilled in the art is familiar with methods for amino acid and peptide synthesis and knows how to apply them to the synthesis of a first component C-1. The first component C-1 may be commercially available, in particular if it is an amino acid or a support-bound amino acid. In some embodiments, the first component C-1 is a peptide obtained by means of support-assisted peptide synthesis. In some embodiments, the first component C-1 is a peptide obtained by means of SPPS. In some embodiments, the first component C-1 is a peptide obtained by means of LPPS. In some embodiments, the first component C-1 is a peptide obtained by means of support-assisted LPPS.

Step (b) of the method of the present invention is: "providing a second component C-2 being selected from the group consisting of a chiral alpha-amino acid and a peptide and comprising one free carboxyl group, which is the alpha-carboxyl group of a chiral alpha-amino acid moiety comprising one hydrogen-substituent bonded to the alpha-carbon atom, wherein any primary or secondary aliphatic amino groups and any other carboxyl groups in C-2 are protected".

In this context, the terms "free carboxyl group" and "primary or secondary aliphatic amino group" are defined as stated above in the for step (a). Thus, when stating that the second component C-2 "comprises one free carboxyl group" and that "any other carboxyl groups in C-2 are protected", this may mean that C-2 comprises one or more carboxyl groups as defined herein, out of which exactly one does not carry a carboxyl protecting group as defined herein, i.e. a protecting group that prevents the respective carboxyl group from partaking in the amide bond forming condensation reaction of step (c). When stating that in the second component C-2, "any primary or secondary aliphatic amino groups" are "protected", this may mean that C-2 optionally comprises one or more primary or secondary aliphatic amino groups as defined herein, all of which carry an amino protecting group as defined herein, i.e. a protecting group that prevents the respective amino group from partaking in the amide bond forming condensation reaction of step (c). Thus, the second component C-2 comprises only a single carboxyl group capable of engaging in the amide bond forming condensation reaction of step (c). Furthermore, the second component C-2 does not comprise any primary or secondary aliphatic amino groups capable of engaging in the amide bond forming condensation reaction of step (c).

A used herein, the term "chiral alpha-amino acid" may refer to an alpha-amino acid, wherein four different substituents (i.e. substituents with different chemical structures) are bonded to the alpha-carbon atom. It will be understood by those skilled in the art, that a chiral alpha-amino acid may optionally comprise additional stereocenters, for example in the case of the amino acids threonine and isoleucine. An "alpha-carboxyl group of a chiral alpha-amino acid moiety" may be understood as the alpha-carboxyl group of a chiral alpha-amino acid as such or of a chiral alpha-amino acid subunit comprised in a peptide, in particular the C-terminal alpha-amino acid subunit of a peptide, since it comprises a free alpha-carboxyl group. The proviso that said "chiral alpha-amino acid moiety" comprises "one hydrogen-substituent bonded to the alpha-carbon atom" implies that exactly one hydrogen substituent is bonded to the alpha-carbon atom of said alpha-amino acid moiety as it would not constitute a chiral alpha-amino acid moiety as defined herein otherwise. The presence of the hydrogen-substituent may be noteworthy for that it implies that epimerization of said chiral alpha-amino acid moiety with respect to the stereocenter at the alpha-carbon atom may for example occur through deprotonation and reprotonation.

In some embodiments, the second component C-2 is a chiral alpha-amino acid. In some embodiments, the second component C-2 is a chiral alpha-amino acid. In some embodiments, the second component C-2 is selected from the group consisting of histidine, cysteine, an alpha-arylglycine, and an alpha-heteroarylglycine. In some embodiments, the second component C-2 is selected from the group consisting of histidine, cysteine, and an alpha-arylglycine. In some embodiments, the second component C-2 is selected from the group consisting of histidine, cysteine, and alpha-phenylglycine. In some embodiments, the second component C-2 is selected from the group consisting of histidine and cysteine. In some embodiments, the second component C-2 is histidine. In some embodiments, the second component C-2 is cysteine. In some embodiments, the second component C-2 is selected from the group consisting of an alpha-arylglycine and an alpha-heteroarylglycine. In some embodiments, the second component C-2 is an alpha-arylglycine, preferably an α-phenylglycine, in particular α-phenylglycine as such. The term "alpha-arylglycine" may herein refer to any alpha-amino acid which may be derived from glycine by substituting one of the hydrogen residues of the alpha-carbon atom for an aryl residue. Likewise, the term "alpha-heteroarylglycine" may herein refer to any alpha-amino acid which may be derived from glycine by substituting one of the hydrogen residues of the alpha-carbon atom for a heteroaryl residue.

When stating that the second component C-2 is histidine, the term "histidine" refers to the proteinogenic alpha-amino acid histidine in any stereoisomeric form, wherein said amino acid may optionally bear one or more protecting groups. Such protecting groups may in particular comprise an alpha-amino protecting group such as tertbutyloxycarbonyl- (Boc), 9-fluorenylmethyloxycarbonyl (Fmoc), benzyloxycarbonyl (Cbz or Z), allyloxycarbonyl (Alloc), and related carbamate protecting groups, out of which Fmoc may be most preferred. Furthermore, for example, histidine may carry a protecting group at the N^{τ}-atom of the imidazole residue (i.e. in 1-position of the imidazole residue), preferably a Boc- or a triarylmethyl-type protecting group such as a triphenylmethyl (trityl)-type protecting group such as a trityl (Trt), chlorotrityl (Cl-Trt), methyltrityl (Mtt), and methoxytrityl (Mmt) group, out of which Trt may be most preferred. Histidine may also carry a protecting group at the N^{π}-atom of the imidazole residue (i.e. in 3-position of the imidazole residue). Non-limiting examples of histidine derivatives that may be used as the second component C-2 when stating that said second component C-2 may be or is histidine comprise: Fmoc-His(1-Trt)-OH, Fmoc-His(1-Boc)-OH, Alloc-His(1-Trt)-OH, Alloc-His(1-Boc)-OH, Boc-His(1-Trt)-OH, and Boc-His(1-Boc)-OH in all stereoisomeric forms. In some embodiments, the second component C-2 is Fmoc-His(1-Trt)-OH. A person skilled in the art is familiar with histidine protecting groups and knows how to introduce and to remove them. A person skilled in the art also knows how to select such protecting groups in light of certain desired reaction conditions, for example from F. Alberico et al., Chem. Rev. 2009., 109(6), 2455-2504 and similar publications.

When stating that the second component C-2 is cysteine, the term "cysteine" in this context refers to the proteinogenic alpha-amino acid cysteine in any stereoisomeric form, wherein said amino acid may optionally bear one or more protecting groups. Such protecting groups may in particular comprise an alpha-amino protecting group such as Boc, Fmoc, Cbz, Alloc, and related carbamate protecting groups, out of which Fmoc may be most preferred. Furthermore, the thiol group (in other words sulfhydryl group) in the cysteine side chain may preferably be protected as well, for example with a triarylmethyl-type protecting group such as a triphenylmethyl (trityl)-type protecting group such as a trityl (Trt), chlorotrityl (Cl-Trt), methyltrityl (Mtt), and methoxytrityl (Mmt) group, out of which a Trt-group may be most preferred. Further non-limiting examples of protecting groups for cysteine's side chain thiol group comprise benzyl-type protecting groups such as a benzyl (Bn) group, a *para*methylbenzyl (Meb) group, a *para*-methoxybenzyl (Mob) group, a trimethoxybenzyl (Tmob) group, and the related 2,2,4,6,7-pentamethyl-5-dihydrobenzofuranylmethyl (Pmbf) group. Additional nonlimiting examples of protecting groups for cysteine's side chain thiol group comprise the 9fluorenylmethyl (Fm) group, the Fmoc group, the acetamidomethyl (Acm) group, and many more. In some embodiments, the second component C-2 is Fmoc-Cys(Trt)-OH. A person skilled in the art is familiar with cysteine protecting groups and knows how to introduce and to remove them. A person skilled in the art also knows how to select such protecting groups in light of certain desired reaction conditions, for example from F. Alberico et al., Chem. Rev. 2009., 109(6), 2455-2504 and similar publications.

When stating that the second component C-2 is an alpha-arylglycine or an alpha-heteroarylglycine, these terms may herein refer to the respective amino acids in any stereoisomeric form, wherein said amino acids may optionally bear one or more protecting groups. Such protecting groups may in particular comprise an alpha-amino protecting group such as Boc, Fmoc, Cbz, Alloc, and related carbamate protecting groups, out of which Fmoc may be most preferred. Again, a person skilled in the art is familiar with suitable protecting groups and the means of introducing and removing them.

In some embodiments, the second component C-2 is a peptide as defined herein above. In other words, all of the previous explanations relating to a peptide in general are applicable to the second component C-2 in as far as it is a peptide.

In some embodiments, the second component C-2 is a peptide selected from the group consisting of a dipeptide and a tripeptide. As used herein, a dipeptide is a peptide comprising exactly two amino acid subunits, and a tripeptide is a peptide comprising exactly three amino acid subunits. In some embodiments, the second component C-2 is a tripeptide. In some embodiments, the second component C-2 is a dipeptide. Preferred examples of such dipeptides comprise the well-known pseudoproline dipeptides. The term "pseudoproline dipeptides" as used herein may refer to temporary proline mimics, which may be readily obtained from serine (Ser) and threonine (Thr) by oxazolidine formation and from cysteine (Cys) by thiazolidine formation. The 2,2-dimethyloxazolidines are smoothly cleaved by TFA and thus particularly suitable for Fmoc-SPPS. Non-limiting examples of dipeptides for use as the second component C-2 in the method of the present invention comprise Fmoc-Phe-Thr(Psi(Me,Me)pro)-OH, or Fmoc-Gly-Thr(Psi(Me,Me)pro)-OH, Fmoc-Thr(^{t}Bu)-Ser(Psi(Me,Me)pro)-OH, Fmoc-Val-Ser(Psi(Me,Me)pro)-OH, and Fmoc-Ser(^{t}Bu)-Ser(Psi(Me,Me)pro)-OH. Herein, the 3-letter code Psi denotes "pseudoproline".

In some embodiments, the second component C-2 is a peptide comprising a C-terminal amino acid selected from the group consisting of histidine, cysteine, an alpha-arylglycine, and an alpha-heteroarylglycine. In some embodiments, the second component C-2 is a peptide comprising a C-terminal amino acid selected from the group consisting of histidine, cysteine, and alpha-phenylglycine.

In some embodiments, the second component C-2 is selected from the group consisting of a chiral alpha-amino acid, a dipeptide, and a tripeptide. In some embodiments, the second component C-2 is selected from the group consisting of a chiral alpha-amino acid and a dipeptide.

In some embodiments, the second component C-2 comprises a primary or secondary aliphatic amino group which is protected by a fluorenylmethyloxycarbonyl-type protecting group, in particular the Fmoc group. In some embodiments, the second component C-2 comprises a primary or secondary aliphatic amino group which is protected by a fluorenylmethyloxycarbonyl-type protecting group, in particular the Fmoc group, wherein said primary or secondary aliphatic amino group is an alpha-amino group of an alpha-amino acid moiety. In some embodiments, the second component C-2 comprises exactly one primary or secondary aliphatic amino group which is protected by a fluorenylmethyloxycarbonyl-type protecting group, in particular the Fmoc group. In some embodiments, the second component C-2 comprises exactly one primary or secondary aliphatic amino group which is protected by a fluorenylmethyloxycarbonyl-type protecting group, in particular the Fmoc group, wherein said primary or secondary aliphatic amino group is an alpha-amino group of an alpha-amino acid moiety. Herein, the term "fluorenylmethyloxycarbonyl-type" protecting group may refer to any protecting group which comprises a fluorenylmethyloxycarbonyl-structure, in which optionally, one or more hydrogen residues may be substituted. Non-limiting examples of fluorenylmethyloxycarbonyl-type protecting groups comprise the 2,6-di-tert-butyl-9-fluorenylmethyloxycarbonyl protecting group, the 2,7-bis(trimethylsilyl)-9-fluorenylmethyloxycarbonyl protecting group, and the 9-fluorenylmethyloxycarbonyl protecting group Fmoc as such, which is herein most preferred.

In some embodiments, the second component C-2 is a chiral alpha-amino acid selected from the group consisting of Fmoc-His(1-Trt)-OH, Fmoc-Cys(Trt)-OH, and Fmoc-Phg-OH, wherein these amino acids may be present in any stereosomeric form. The 3-letter code Phg in this context refers to the amino acid alpha-phenylglycine (i.e. 2-phenylglycine).

In some embodiments, the population of molecules of the second component C-2 comprises essentially a single stereoisomer. In some embodiments, wherein the second component C-2 is a chiral alpha-amino acid, the population of molecules of said second component C-2 displays an enantiomeric excess (ee) equal to or larger than 95%, 96%, 97%, 98%, 99%, 99.5%, 99.6%, 99.7%, 99.8%, or 99.9%. The means of determining the enantiomeric excess of an amino acid sample form part of the common knowledge of those skilled in the art. In fact, any method commonly used to determine the enantiomeric excess of amino acids may be used, preferably (chiral) analytical HPLC using UV-detection. Alternatively, (chiral) gas chromatography in combination with mass spectrometry may be used. In the resulting chromatogram, the area underneath the peaks for both enantiomers may be obtained from integration. The ratio of each enantiomer in percent (%) may be obtained by dividing the area underneath the peak of the respective enantiomer by the sum of the areas underneath the peaks of both enantiomers. The ee in % may then be obtained by subtracting the ratio of the minor enantiomer in % from the ratio of the major (i.e. excess) enantiomer in %. For example, if the L-enantiomer has a ratio of 99% and the D-enantiomer has a ratio of 1%, the ee would be ee = 99%-1% = 98%.

In some embodiments, wherein the second component is a peptide or an amino acid with more than one stereocenter, the population of molecules of said second component C-2 displays a diastereomeric excess (de) equal to or larger than 95%, 96%, 97%, 98%, 99%, 99.5%, 99.6%, 99.7%, 99.8%, or 99.9%, wherein the de refers to the two diastereomers of C-2 that differ only in the absolute configuration at the alpha-carbon atom of the chiral alpha-amino acid moiety comprising the one free alpha-carboxyl group. The means of determining the diastereomeric excess of a peptide sample form part of the common knowledge of those skilled in the art. In fact, any method commonly used to determine the diastereomeric excess of peptides may be used, preferably (chiral) analytical HPLC using UV-detection. Alternatively, (chiral) gas chromatography in combination with mass spectrometry may be used after hydrolysis of amide bonds. In the resulting chromatogram, the area underneath the peaks for both diastereomers may be obtained from integration. The ratio of each diastereomer in percent (%) may be obtained by dividing the area underneath the peak of the respective diastereomer by the sum of the areas underneath the peaks of both diastereomers. The de in % may then be obtained by subtracting the ratio of the minor diastereomer in % from the ratio of the major (i.e. excess) diastereomer in %. For example, if the major diastereomer has a ratio of 99% and the minor diastereomer has a ratio of 1%, the de would be de = 99%-1% = 98%.

The term "providing a second component C-2" in step (b) of the method of the present invention may be understood in the broadest sense and may refer to any process of obtaining said second component C-2. The second component C-2 may be obtained by means of amino acid or peptide synthesis, wherein any synthetic strategies (i.e. synthetic approaches) known from the state of the art may be applied. A person skilled in the art is familiar with methods for amino acid and peptide synthesis and knows how to apply them to the synthesis of a second component C-2. The second component C-2 may be commercially available, in particular if it is a chiral alpha-amino acid.

In Step (c) of the method of the present invention, the first component C-1 of step (a) and the second component C-2 of step (b) are condensed "so as to form an amide bond between the free primary or secondary aliphatic amino group of the first component C-1 and the free alpha-carboxyl group of the second component C-2, to obtain a first cycle peptide P-1". The "first cycle Peptide P-1", the "free primary or secondary aliphatic amino group of the first component C-1", and the "free alpha-carboxyl group of the second component C-2" have been defined above. It is understood that said "amide bond" formed between the first component C-1 and the second component C-2 is a peptide bond as defined herein.

As used herein, a condensation reaction (or condensing reaction or condensation) is a chemical reaction during which two molecules or compounds (in step (c), the first component C-1 and the second component C-2) are connected by one or more newly formed chemical bonds (in step (c), by a newly formed amide bond) to obtain a product (in step (c), the first cycle peptide P-1) which comprises the chemical structures of both molecules or compounds which have been connected, with the exception that during the bond forming reaction one or more, typically one, small molecules such as water or a leaving group derived from a coupling agent or additive and/or one or more protons are expelled and no longer present in the product.

A person skilled in the art will understand, that in order to condense "the first component C-1 of step (a) and the second component C-2 of step (b)" in step (c), these two components need to be combined in a reaction vessel or reactor at some point during step (c), wherein the form and kind of reaction vessel or reactor is not particularly limited. In fact, any kind of reaction vessel or reactor used for peptide synthesis may be used for the method disclosed herein. For example, step (c) may be performed in an industrial reaction vessel equipped with a mechanical stirrer or in a reaction vessel of a peptide synthesizer.

According to the method of the present invention, the first component C-1 of step (a) and the second component C-2 of step (b) are condensed in the presence of a carbodiimide coupling agent, a first additive A-1, a second additive A-2, and a first cycle solvent. In this context, the term "in the presence of" may mean that at some point in time during step (c), the first component C-1, the second component C-2, the carbodiimide coupling agent, the first additive A-1, the second additive A-2, and the first cycle solvent are all comprised in the same reaction vessel or reactor. The order in which these species are added into said reaction vessel or reactor is not particularly limited, as long as they all end up in said reaction vessel or reactor.

It is known to those skilled in the art that a free carboxyl group, such as the free carboxyl group of the second component C-2, may typically need to be activated towards amide bond formation with an amino group such as the free primary or secondary aliphatic amino group of the first component C-1. Such activation may for example enable the amide bond formation at or near to room temperature (i.e. (approximately 20 °C)) and may (largely) favor it over potential side reactions. Activation of a free carboxyl group towards amide bond formation, in particular in the context of peptide synthesis, may typically be achieved by addition of one or more coupling agents. Herein, the terms "coupling agent", "coupling reagent", "condensing agent", and "condensing reagent" may be used interchangeably. Herein, a carbodiimide coupling agent is used.

A person skilled in the art is familiar with carbodiimide coupling agents and knows how to select and obtain a carbodiimide coupling agent for use according to the present invention. Any carbodiimide coupling agent used for peptide synthesis may be used in the present invention. A carbodiimide coupling agent may react with the free carboxyl group of the second component C-2, which is to be activated towards amide bond formation, to form an intermediate O-acylisourea.

Herein, unless indicated differently in specific embodiments, a "carbodiimide coupling agent" may be an organic molecule of a structure according to the following formula CDI-1: 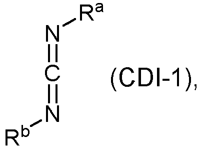 wherein in formula CDI-1,
R^{a} and R^{b} may be the same or different and may be any chemical residues.

In some embodiments, a carbodiimide coupling agent is an organic molecule of the aforementioned Formula CDI-1, wherein R^{a} and R^{b} are independently of each other selected from the group consisting of an alkyl group and a heteroalkyl group, which may be linear, branched, or cyclic. In some embodiments, a carbodiimide coupling agent is an organic molecule of the aforementioned Formula CDI-1, wherein R^{a} is an alkyl group and R^{b} is selected from the group consisting of an alkyl group and a heteroalkyl group.

In some embodiments, a carbodiimide coupling agent is an organic molecule of the aforementioned Formula CDI-1, wherein:
R^{a} is a C₁-C₆-alkyl group, and
R^{b} is selected from the group consisting of a C₁-C₆-alkyl group and a C₁-C₆-heteroalkyl group,
with the proviso that, if R^{b} is a heteroalkyl group, it comprises exactly one heteroatom, wherein said heteroatom is a nitrogen atom.

In some embodiments, a carbodiimide coupling agent is an organic molecule of the aforementioned Formula CDI-1, wherein:
R^{a} is a C₁-C₆-alkyl group, and
R^{b} is selected from the group consisting of a C₁-C₆-alkyl group and a 3-dimethylaminopropyl group.

In some embodiments, the carbodiimide coupling agent is selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof. In some embodiments, the carbodiimide coupling agent is selected from the group consisting of DIC and DCC, or a mixture thereof. In some embodiments, the carbodiimide coupling agent is selected from the group consisting of DIC and DCC. In some embodiments, the carbodiimide coupling agent is DIC. In some embodiments, the carbodiimide coupling agent is DCC.

In some embodiments, the carbodiimide coupling agent(s) may be used in free form. Alternatively, a carbodiimide coupling agent may be a salt.

The chemical structure of the first additive A-1 of the present invention has already been laid out. A preferred example of the first additive A-1 may be 2-hydroxypyridine N-oxide (2-HOPO), which is a compound of formula A-I, wherein R¹ is at each occurrence hydrogen. 2-HOPO may be present as the tautomeric 1-hydroxy-2-pyridone or a mixture of tautomers without further notice. In some embodiments, the first additive A-1 is 2-hydroxypyridine N-oxide (2-HOPO).

In some embodiments, the second additive A-2 is selected from the group consisting of Oxyma, HOBt, and HOAt, or mixtures thereof. It is understood by a person skilled in the art that Oxyma, HOBt, HOAt, Oxyma B, and HOCt may for example be added to the reaction mixture of step (c) in form of a hydrate.

In some embodiments, the first additive A-1 is 2-HOPO and the second additive A-2 is selected from the group consisting of Oxyma, HOBt, and HOAt, or mixtures thereof. In some embodiments, the first additive A-1 is 2-HOPO and the second additive A-2 is Oxyma. In some embodiments, the first additive A-1 is 2-HOPO and the second additive A-2 is HOBt. In some embodiments, the first additive A-1 is 2-HOPO and the second additive A-2 is HOAt. In some embodiments, the first additive A-1 is 2-HOPO and the second additive A-2 is Oxyma-B. In some embodiments, the first additive A-1 is 2-HOPO and the second additive A-2 is HOCt.

Herein, unless indicated differently in the context of specific embodiments, oximes such as Oxyma may be present in (*E*)- and (*Z*)-configuration with regard to the oxime's C=N double bond.

Unless indicated differently in specific embodiments, the carbodiimide coupling agent, the first additive A-1, and the second additive A-2 may each be a single compound or a mixture of two or more compounds. In some embodiments, in step (c), the carbodiimide coupling agent, the first additive A-1, and the second additive A-2 are each present as a single compound (i.e. not a mixture of compounds).

The term "fist cycle solvent" may refer to any solvent or solvent mixture capable of dissolving the first component C-1, the second component C-2, the carbodiimide coupling agent, the first additive A-1, and the second additive A-2, with the proviso that, if any of the first component C-1 and the second component C-2 is attached to an insoluble support, it need not be dissolved in the first cycle solvent. In the case that the first component C-1 and/or the second component C-2 is attached to an insoluble support, the first cycle solvent may preferably be selected, so that it is capable of swelling said insoluble support. It should be noted that, when stating that the amide bond forming condensation reaction of step (C) is performed in the presence of a first cycle solvent, this may herein mean that in step (c) the carbodiimide coupling agent, the first additive A-1, and the second additive A-2, the second component C-2, and the first component C-1 are contacted with and dissolved in said first cycle solvent. It is to be understood that in step (c) the carbodiimide coupling agent, the first additive A-1, and the second additive A-2, the second component C-2, and the first component C-1 are contacted with and dissolved in said first cycle solvent with the proviso that any solid support linked to the first component C-1 will not be dissolved. The skilled person will readily understand that said first cycle solvent is applied in an amount (or volume) sufficient to actually dissolve the utilized amounts of the first additive A-1, the second additive A-2, the carbodiimide coupling agent, and any of the first component C-1 and the second component C-2 in as much as they are not attached to an insoluble support. When stating that a solvent is capable of "dissolving" certain compounds or that certain compounds are "dissolved" in a certain solvent, this may herein preferably mean that a clear solution is obtained, upon addition of said compounds to said solvent or vice versa, which does not exclude the presence of traces of undissolved matter which are not visible as such with bare eye.

Non-limiting examples of solvents suitable for use as the first cycle solvent in step (c) comprise, e.g., N,N-dimethylformamide (DMF), N-methyl-2-pyrrolidone (NMP), N-butyl-pyrrolidone (NBP), dimethyl isosorbide (DMI), gamma-valerolactone (GVL), dihydrolevo-glucosenone (Cyrene), dimethyl sulfoxide (DMSO), tetrahydropyran (THP), tetrahydrofuran (THF), 2-methyltetrahydrofuran (Me-THF), 1,3-dioxolane, ethyl acetate (EtOAc), dichloromethane (DCM), acetonitrile (ACN), toluene, and mixtures thereof. In some embodiments, the first cycle solvent of step (c) is selected from the group consisting of N,N-dimethylformamide (DMF), N-methyl-2-pyrrolidone (NMP), N-butyl-pyrrolidone (NBP), dimethyl isosorbide (DMI), gamma-valerolactone (GVL), dihydrolevo-glucosenone (Cyrene), dimethyl sulfoxide (DMSO), tetrahydropyran (THP), tetrahydrofuran (THF), 2-methyltetrahydrofuran (Me-THF), 1,3-dioxolane, ethyl acetate (EtOAc), dichloromethane (DCM), acetonitrile (ACN), toluene, and mixtures thereof. In some embodiments, the first cycle solvent of step (c) is selected from the group consisting of N,N-dimethylformamide (DMF), N-methyl-2-pyrrolidone (NMP), dicholoromethane (DCM), ethyl acetate (EtOAc), dimethyl sulfoxide (DMSO), and mixtures thereof. In some embodiments, the first cycle solvent of step (c) is selected from the group consisting of N,N-dimethylformamide (DMF) and N-methyl-2-pyrrolidone (NMP) and a mixture thereof. In some embodiments, the first cycle solvent of step (c) is N,N-dimethylformamide (DMF).

The method of the invention is not particularly limited with regard to the temperature at which step (c) is performed, which may herein also be referred to as "reaction temperature" or "condensation temperature" or "condensing temperature". The temperature at which step (c) is performed may for example be in the range of 10-30 °C. In some embodiments, the temperature at which step (c) is performed is in a range of 10-30 °C, 15-30 °C, or 20-30 °C. Typically, for convenience, step (c) may be performed at room temperature (i.e. (approximately) 20 °C). However, the reaction time may be shorter at elevated temperatures, which may be desirable for industrial applications. The method disclosed herein is also compatible with elevated temperatures, for example in the range of 30-60 °C. In some embodiments, the temperature at which step (c) is performed is in a range of 30-60 °C, 35-55 °C, or 35-50 °. In some embodiments, the temperature at which step (c) is performed is in a range of 20-50°C. Common means of heating the reaction vessel in which step (c) is performed are known to those skilled in the art and comprise heating by using a double-walled vessel and flushing the void between the two walls with a heating medium such as water, applying microwave or infrared irradiation, and induction heating. The use of a double-walled vessel may be preferred, in particular on an industrial scale. In some embodiments, step (c) may be at least partly performed under microwave irradiation. In this case, the temperature at which step (c) is performed may be elevated, but preferably not higher than 90 °C. In some embodiments, the temperature at which step (c) is performed is in a range of 10-90 °C.

The time for which step (c) of the method of the invention is performed (i.e. the condensing time or condensation time) is not particularly limited and may for example depend on the chemical structure of the first and the second component C-1 and C-2 and on the temperature at which step (c) is performed. Typically, shorter condensing times may be required at elevated temperatures. A person skilled in the art and willing to put the method of the invention into practice will routinely optimize the condensing time and/or the condensing temperature in light of the specific first component C-1 and second component C-2 which are to be condensed. Such optimization can be achieved using standard knowledge and techniques (e.g. standard reaction monitoring to check on the conversion of reactants) and without undue experimentation. The examples provided herein may serve as guiding rail. For example, if the condensing temperature is in a range of 20-50 °C, the condensing time may be in a range of 5-360 min or 20-180 min. For example, if the condensing temperature is 35 °C, the condensing time may be in a range of 30-120 min, or 45-90 min. For example, if the condensing temperature is 50 °C, the condensing time may be in a range of 5-60 min, or 20-40 min.

In some embodiments, in step (c), a solution of the second component C-2, the first additive A-1, and the second additive A-2 in a first part of the first cycle solvent is added into a reaction vessel containing the first component C-1 and a second part of the first cycle solvent, which is directly followed by the addition of the carbodiimide coupling agent into said reaction vessel, preferably in one portion, wherein said first part and said second part of the first cycle solvent need not be identical. In some embodiments, in step (c), the carbodiimide coupling agent may be added into the reaction vessel in several portions, for example in two portions or three portions. The time interval between the addition of said carbodiimide portions may for example be in the range of a few seconds to a few hours, preferably in the range of 10 seconds to 1 hour, of 1 minute to 30 minutes, of 1 minute to 20 minutes, or of 3 minutes to 15 minutes.

In some embodiments, a so-called pre-activation is performed in the course of step (c). Such pre-activations are well-known to a person skilled in the art. For example, the second component C-2 may be preactivated, i.e. pre-incubated in solution with the carbodiimide coupling agent, and one or both of the first additive A-1 and the second additive A-2, prior to being mixed with the first component C-1. Without wishing to be bound by theory, it is believed that during preactivation, the second component C-2 reacts with the carbodiimide coupling agent and one or both of the first additive A-1 and the second additive A-2 to form an activated derivative of the second component C-2. during preactivation. Such an activated derivative of the second component C-2 may for example be an active ester formed from the second component C-2 (via its free alpha-carboxyl group) and the first additive A-1 or the second additive A-2 (via the N-OH functional group of these additives). It is therefore to be understood that step (c) of the methods of the present invention may involve in situ activation of the second component C-2 as well as pre-activation of the second component C-2.

In some embodiments, step (c) comprises the steps of:
(c-1) dissolving in any order the second component C-2, the carbodiimide coupling agent, and one or both of the first additive A-1 and the second additive A-2 in a first part of the first cycle solvent,
(c-2) incubating the solution of step (c-1) for a (so-called) pre-activation time in the range of 10 seconds to 60 minutes, 10 seconds to 35 minutes, 1-30 minutes, 3-30 minutes, 5-25 minutes 5-20 minutes, or 5-15 minutes, and
(c-3) adding the incubated solution of step (c-2) into a reaction vessel containing the first component C-1 and, if one of the first and the second additives A-1 and A-2 has not already been employed in step (c-1), said so far unemployed additive A-1 or A-2, and a second part of the first cycle solvent,
wherein said first part and said second part of the first cycle solvent need not be identical.

The first component C-1, the second component C-2, the carbodiimide coupling agent, the first additive A-1, and the second additive A-2 may optionally be employed (i.e. be provided in step (a) or (b) and/or present in step (c)) in form of their hydrates.

As laid out above, in step (c) of the method of the present invention, "the second additive A-2 is used in a total molar amount of 1.0 to 5.0 equivalents relative to the total molar amount of the first component C-1". This will be understood to mean that in step (c), for each mole of the first component C-1, equal to or more than 1.0 moles (i.e. 1.0 equivalents) but not more than 5.0 moles (i.e. 5.0 equivalents) of the second additive A-2 are used, i.e. introduced directly or indirectly into the reaction mixture of step (c).

As used herein, the term "total molar amount" refers to the overall amount of a certain species which was directly or indirectly introduced into the reaction mixture of the respective condensation reaction. For example, as laid out above, the carbodiimide coupling agent and/or the first additive A-1, and/or the second additive A-2 may be mixture of two or more compounds. In such cases, the total molar amount of the respective species is the sum of molar amounts of the two or more compounds. For example, if a mixture of HOBt and HOAt was used as second additive A-2, the total molar amount of the second additive A-2 would be the sum of the molar amounts of HOBt and HOAt. As a further example, if a species (e.g. the carbodiimide coupling agent) is added in several portions into the reaction mixture, the total molar amount of said species is the sum of the molar amounts added at each portion.

In some embodiments of the invention, in step (c), the second additive A-2 is used in a total molar amount of 1.00-5.00 equivalents, 1.50-4.00 equivalents, 2.00-4.00 equivalents, 2.50-3.50 equivalents, or 3.00 equivalents, relative to the total molar amount of the first component C-1.

In some embodiments, in step (c):
- the second component C-2 is used in a total molar amount of 1.0 to 5.0 equivalents,
- the carbodiimide coupling agent is used in a total molar amount of 1.0 to 6.0 equivalents,
- the first additive A-1 is used in a total molar amount of 1.0 to 6.0 equivalents, and
- the second additive A-2 is used in a total molar amount of 1.0 to 5.0 equivalents,
wherein all equivalents are given relative to the total molar amount of the first component C-1.

In some embodiments, in step (c):
- the second component C-2 is used in a total molar amount of 1.00-5.00 equivalents, 1.00-4.00 equivalents, 1.00-3.00 equivalents, 1.50-2.50 equivalents, or 2.00 equivalents,
- the carbodiimide coupling agent is used in a total molar amount of 1.00-6.00 equivalents, 2.00-5.00 equivalents, 3.00-5.00 equivalents, 3.50-4.50 equivalents, or 4.00 equivalents,
- the first additive A-1 is used in a total molar amount of 1.00-6.00 equivalents, 2.00-5.00 equivalents, 3.00-5.00 equivalents, 3.50-4.50 equivalents, or 4.00 equivalents, and
- the second additive A-2 is used in a total molar amount of 1.00-5.00 equivalents, 1.50-4.00 equivalents, 2.00-4.00 equivalents, 2.50-3.50 equivalents, or 3.00 equivalents,
wherein all equivalents are given relative to the total molar amount of the first component C-1.

In some embodiments, in step (c):
- the second component C-2 is used in a total molar amount of 1.00-4.00 equivalents,
- the carbodiimide coupling agent is used in a total molar amount of 2.00-5.00 equivalents,
- the first additive A-1 is used in a total molar amount of 2.00-5.00 equivalents, and
- the second additive A-2 is used in a total molar amount of 1.50-4.00 equivalents,
wherein all equivalents are given relative to the total molar amount of the first component C-1.

In some embodiments, in step (c):
- the second component C-2 is used in a total molar amount of 1.00-3.00 equivalents,
- the carbodiimide coupling agent is used in a total molar amount of 3.00-5.00 equivalents,
- the first additive A-1 is used in a total molar amount of 3.00-5.00 equivalents, 3.50-4.50 equivalents, and
- the second additive A-2 is used in a total molar amount of 2.00-4.00 equivalents,
wherein all equivalents are given relative to the total molar amount of the first component C-1.

In some embodiments, in step (c):
- the second component C-2 is used in a total molar amount of 1.50-2.50 equivalents,
- the carbodiimide coupling agent is used in a total molar amount of 3.50-4.50 equivalents,
- the first additive A-1 is used in a total molar amount of 3.50-4.50 equivalents, and
- the second additive A-2 is used in a total molar amount of 2.50-3.50 equivalents,
wherein all equivalents are given relative to the total molar amount of the first component C-1.

In some embodiments, in step (c):
- the second component C-2 is used in a total molar amount of 2.0 equivalents,
- the carbodiimide coupling agent is used in a total molar amount of 4.0 equivalents,
- the first additive A-1 is used in a total molar amount of 4.0 equivalents, and
- the second additive A-2 is used in a total molar amount of 3.0 equivalents,
wherein all equivalents are given relative to the total molar amount of the first component C-1.

As used herein, the expression "total molar amount of the first component C-1" may refer to the overall molar amount (e.g. in moles or millimoles) of said first component C-1 as employed in step (c) of the method of the invention, wherein, if C-1 is a support-bound amino acid or peptide, the total molar amount may refer to the molar amount of C-1 molecules attached to the support. In particular, said total molar amount may be identical to the total molar amount of free primary or secondary aliphatic amino groups of C-1 available for the intended amide bond forming condensation reaction of step (c), since per definition, each molecule of C-1 comprises exactly one such free primary or secondary aliphatic amino group. The means of determining the total molar amount of the first component C-1 are known to those skilled in the art, who also know that said means may depend on the nature (i.e. chemical structure) of C-1.

For example, if the first component C-1 is not bound to a support, it may simply be weighed to determine the mass (herein also referred to as weight) of employed C-1, which may then be divided by the known molar mass of C-1 to arrive at the molar amount of C-1 (e.g.- in moles or millimoles). If, e.g., the employed sample of C-1 is not of high purity, one may multiply the calculated total molar amount of C-1 with the determined purity written as decimal number (e.g. 94% = 0.94) to arrive at a more precise value for the total molar amount of the first component C-1. Analogously, the skilled person will readily apply other correction factors depending on the specific situation. For example, if the chemical content of the first component C-1 within a material used is below 100% due to the presence of water or solvent, one may multiply the calculated total molar amount of C-1 with the determined content of C-1 written as decimal number.

If the first component C-1 is bound, typically covalently linked, to a solid support, the total molar amount of the first component C-1 for step (c) may typically be determined by weighing the dry solid support carrying the C-1 molecules to be employed in step (c) and multiplying the so-determined weight with the resin load. The resin load is the molar amount of C-1 molecules per gram of the solid support carrying the C-1 molecules. The resin load may be provided by the supplier, if the solid support carrying the C-1 molecules is obtained commercially. Alternatively, the resin load may be determined by means known to those skilled in the art. For example, a sample of the resin may be weighed and the molar amount of free amino groups be determined by pH titration (to assess the amount of free amino groups at the support) or photometry. Photometric determination (quantification) relies on detecting the Fmoc chromophore liberated upon removing an Fmoc protecting group from the primary or secondary aliphatic amino group ofC-1. It may then be assumed that the amount of liberated protecting groups equals the amount of C-1 molecules attached to the support. Such a photometric determination may also be applicable to fluorenylmethyloxycarbonyl-type protecting groups other than the Fmoc group as such. Since the length of the peptide chains and hence the weight of the peptide-resin-conjugates increases with each coupling cycle, the resin load, i.e. the molar amount of the first component C-1 per gram of resin, will decrease with each coupling cycle. If the resin load of a peptide is not determined experimentally as set out above, it may be calculated based on an experimentally determined initial resin load, which is corrected to account for the weight gain caused by peptide elongation.

For calculating equivalents relative to the first component C-1, it may be assumed that the overall molar amount of growing peptide chains is constant over the duration of all coupling cycles in SPPS. Hence, if C-1 is a peptide bound to a solid support, its molar amount may be approximated to be equal to the molar amount of its first amino acid bound to the solid support. For many common protocols, if the solid support is a so-called amide resin (e.g. the Rink amide resin or the Ramage amide resin or the TAL resin) it may further be assumed that the molar amount of peptide chains is identical to the molar amount of free amino groups, which are present on the resin prior to coupling the first amino acid. Hence, if C-1 is a peptide bound to an amide resin, its molar amount may often be approximated to be equal to the molar amount of free amino groups, which are present on the resin prior to coupling the first amino acid.

As laid out herein, the first component C-1 may also be bound, typically covalently linked, to a soluble support. Soluble supports for peptide synthesis may typically be organic molecules (i.e. tags), wherein each such tag may typically carry a defined amount of molecules of C-1. For some soluble supports, each tag may carry exactly one molecule of C-1. In other cases, each tag may carry more than one, e.g. 2 or 3, molecules of C-1. Since the structure of the tag carrying a molecule of C-1 may typically be known, the total molar amount of C-1 molecules for step (c) may typically be determined by weighing the sample of support-bound C-1 molecules which is to be used in step (c), dividing the so-obtained mass (i.e. weight amount) by the known molar mass of the respective C-1-support-conjugate, and multiplying by a factor reflecting the number of molecules of C-1 per tag. For calculating equivalents relative to the first component C-1, it may be assumed that the molar amount of peptide chains is constant over the duration of all coupling cycles over the course of the tag-assisted LPPS. As outlined above and depending on the specific situation, the skilled person will readily apply any correction factors needed, e.g. to reflect the chemical content or purity of the sample of support-bound C-1 molecules, which has been weighed.

In some embodiments:
- the first component C-1 is covalently linked to a support,
- the second component C-2 is selected from the group consisting of histidine, cysteine, an alpha-arylglycine, and an alpha-heteroarylglycine,
- the second component C-2 comprises a primary or secondary aliphatic amino group which is protected by a fluorenylmethyloxycarbonyl-type protecting group,
- the carbodiimide coupling agent of step (c) is selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof,
- the first additive A-1 is 2-hydroxypyridine N-oxide (2-HOPO),
- in step (c):
   - the second component C-2 is used in a total molar amount of 1.0 to 5.0 equivalents,
   - the carbodiimide coupling agent is used in a total molar amount of 1.0 to 6.0 equivalents,
   - the first additive A-1 is used in a total molar amount of 1.0 to 6.0 equivalents, and
   - the second additive A-2 is used in a total molar amount of 1.0 to 5.0 equivalents,
      wherein all equivalents are given relative to the total molar amount of the first component C-1,
- the temperature at which step (c) is performed is in a range of 10-90 °C, and
- optionally, the time for which step (c) is performed (i.e. the condensing time) is in a range of 5-360 min.

In some embodiments:
- the first component C-1 is covalently linked to a support,
- the free primary or secondary aliphatic amino group of the first component C-1 is an alpha-amino group of an alpha-amino acid moiety,
- the second component C-2 is selected from the group consisting of histidine, cysteine, an alpha-arylglycine, and an alpha-heteroarylglycine,
- the second component C-2 comprises a primary or secondary aliphatic amino group which is protected by a fluorenylmethyloxycarbonyl-type protecting group,
- the carbodiimide coupling agent of step (c) is selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof,
- the first additive A-1 is 2-hydroxypyridine N-oxide (2-HOPO),
- in step (c):
   - the second component C-2 is used in a total molar amount of 1.0 to 5.0 equivalents,
   - the carbodiimide coupling agent is used in a total molar amount of 1.0 to 6.0 equivalents,
   - the first additive A-1 is used in a total molar amount of 1.0 to 6.0 equivalents, and
   - the second additive A-2 is used in a total molar amount of 1.0 to 5.0 equivalents,
      wherein all equivalents are given relative to the total molar amount of the first component C-1,
- the temperature at which step (c) is performed is in a range of 10-90 °C, and
- optionally, the time for which step (c) is performed (i.e. the condensing time) is in a range of 5-360 min.

In some embodiments:
- the first component C-1 is covalently linked to a support,
- the second component C-2 is selected from the group consisting of histidine, cysteine, an alpha-arylglycine, and an alpha-heteroarylglycine,
- the second component C-2 comprises a primary or secondary aliphatic amino group which is protected by a fluorenylmethyloxycarbonyl-type protecting group,
- the carbodiimide coupling agent of step (c) is selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof,
- the first additive A-1 is 2-hydroxypyridine N-oxide (2-HOPO),
- in step (c):
   - the second component C-2 is used in a total molar amount of 1.0 to 5.0 equivalents,
   - the carbodiimide coupling agent is used in a total molar amount of 1.0 to 6.0 equivalents,
   - the first additive A-1 is used in a total molar amount of 1.0 to 6.0 equivalents, and
   - the second additive A-2 is used in a total molar amount of 1.0 to 5.0 equivalents,
      wherein all equivalents are given relative to the total molar amount of the first component C-1,
- the temperature at which step (c) is performed is in a range of 20-50 °C, e.g. 35-50 °C, and
- optionally, the time for which step (c) is performed (i.e. the condensing time) is in a range of 20-180 min.

In some embodiments:
- the first component C-1 is covalently linked to a support,
- the free primary or secondary aliphatic amino group of the first component C-1 is an alpha-amino group of an alpha-amino acid moiety,
- the second component C-2 is selected from the group consisting of histidine, cysteine, an alpha-arylglycine, and an alpha-heteroarylglycine,
- the second component C-2 comprises a primary or secondary aliphatic amino group which is protected by a fluorenylmethyloxycarbonyl-type protecting group,
- the carbodiimide coupling agent of step (c) is selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof,
- the first additive A-1 is 2-hydroxypyridine N-oxide (2-HOPO),
- in step (c):
   - the second component C-2 is used in a total molar amount of 1.0 to 5.0 equivalents,
   - the carbodiimide coupling agent is used in a total molar amount of 1.0 to 6.0 equivalents,
   - the first additive A-1 is used in a total molar amount of 1.0 to 6.0 equivalents, and
   - the second additive A-2 is used in a total molar amount of 1.0 to 5.0 equivalents,
      wherein all equivalents are given relative to the total molar amount of the first component C-1,
- the temperature at which step (c) is performed is in a range of 20-50 °C, e.g. 35-50 °C, and
- the time for which step (c) is performed (i.e. the condensing time) is in a range of 20-180 min.

In each of the four embodiments above, the second component C-2 may comprise a primary or secondary aliphatic amino group, which is protected by a fluorenylmethyloxycarbonyl type protecting group, preferably the Fmoc group.

Hence, in some embodiments:
- the first component C-1 is covalently linked to a support,
- the free primary or secondary aliphatic amino group of the first component C-1 is an alpha-amino group of an alpha-amino acid moiety,
- the second component C-2 is selected from the group consisting of histidine, cysteine, and alpha-phenylglycine,
- the second component C-2 comprises a primary or secondary aliphatic amino group which is protected by a Fmoc protecting group,
- the carbodiimide coupling agent of step (c) is selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof,
- the first additive A-1 is 2-hydroxypyridine N-oxide (2-HOPO),
- in step (c):
   - the second component C-2 is used in a total molar amount of 1.0 to 5.0 equivalents,
   - the carbodiimide coupling agent is used in a total molar amount of 1.0 to 6.0 equivalents,
   - the first additive A-1 is used in a total molar amount of 1.0 to 6.0 equivalents, and
   - the second additive A-2 is used in a total molar amount of 1.0 to 5.0 equivalents,
      wherein all equivalents are given relative to the total molar amount of the first component C-1,
- the temperature at which step (c) is performed is in a range of 20-50 °C, e.g. 35-50 °C, and
- the time for which step (c) is performed (i.e. the condensing time) is in a range of 20-180 min.

Herein, the terms "residue", "moiety", "substituent", and "group" may be used interchangeably. All of them form part of the common knowledge of those skilled in the art. In line with common practice, the names of substituents may herein typically be derived from the chemical name and indicated by the last syllable "-yl". However, herein, this wording may be used interchangeably with the chemical name of the respective chemical atom or atom group as if it was not a substituent, with common abbreviations, and with the element symbol(s) themselves. For example, a CH₃-substituent may also be referred to as methyl or Me, a Cl-substituent may also be referred to as chlorine or simply Cl, and a phenyl substituent may also be denoted as Ph. Furthermore, when denoting substituents in form of their element symbol(s), hyphens may herein occasionally be used to indicate which atom of an atom group constitutes the binding site of the respective substituent. The hyphen does herein not imply the presence of an additional chemical bond that is not already depicted in the generic formula, that bears the respective residue. Herein, unless stated differently in the context of specific embodiments, when referring to a specific substituent in general terms such as "butyl", this wording embraces all isomers having a chemical structure falling under the respective general substituent name such as "butyl" or "pyridinyl", including for example regioisomers (such as n-butyl, *sec*-butyl, *tert*-butyl, isobutyl or 2-pyridinyl, 3-pyridinyl, 4-pyridinyl, and the like), diastereomers, and enantiomers, where applicable.

As used throughout the present invention, unless indicated differently in the context of specific embodiments, the term "alkyl" may be understood in the broadest sense and may be used to denote any aliphatic group (in other words: residue, moiety or substituent) that may be composed of atoms of the chemical elements carbon (C) and hydrogen (H), but does not comprise any heteroatoms. Additionally, an alkyl group as defined herein may be characterized in that it comprises at least one carbon atom and in that the one or more carbon atoms may only be bonded to each other via direct single bonds. Preferably, unless indicated differently in the context of specific embodiments, an alkyl group as defined herein may comprise 1 to 20, more preferably 1 to 6, in particular 1 to 5 carbon atoms. Unless indicated differently in the context of specific embodiments, the term "alkyl" may generally embrace unbranched, branched, and cyclic groups. Particularly preferred examples of alkyl groups comprise; methyl, ethyl, *n*-propyl (propane-1-yl), isopropyl (propane-2-yl), *n*-butyl (butan-1-yl), *sec*-butyl (butan-2-yl), *tert*-butyl (2-methylpropan-2-yl), isobutyl (2-methylpropan-1-yl), cyclopentyl, and cyclohexyl.

As used throughout the present invention, unless indicated differently in the context of specific embodiments, the term "heteroalkyl" may be understood in the broadest sense and may be used to denote any alkyl group in which one or more carbon or hydrogen atom is substituted by a heteroatom. A heteroatom in this context may be any atom of a chemical element other than carbon and hydrogen, and preferably may be an atom that is at each occurrence independently selected from the group consisting of N (nitrogen), O, (oxygen), S (sulfur), P (phosphorus), F (fluorine), Cl (chlorine), Br (bromine), I (iodine), and Si (silicon). Preferably, unless indicated differently in the context of specific embodiments, a heteroalkyl group as defined herein may comprise 1 to 19, more preferably 1 to 6, in particular 1 to 5, or even 1 to 4 carbon atoms. Unless stated differently, the term "heteroalkyl" may generally embrace unbranched, branched, and cyclic groups. Particularly preferred examples of heteroalkyl groups comprise halogenated alkyl groups such as trifluoromethyl groups, oxygenated alkyl groups (i.e. alkyloxy groups), aminated alkyl groups (i.e. alkylamine groups), and cyclic groups such as e.g. pyrrolidine groups, piperidine groups, or morpholine groups. A heteroalkyl substituent may typically be bonded via one or more of its carbon atoms.

As used throughout the present invention, unless indicated differently in the context of specific embodiments, the term "aryl" may be understood in the broadest sense and may be used to denote any mono- or polycyclic aromatic group, with the proviso that all aromatic ring atoms are carbon atoms. Unless indicated differently in the context of specific embodiments, an aryl group as defined herein may preferably comprise 6 to 30, more preferably 6 to 20, even more preferably 6 to 15, in particular 6 aromatic carbon atoms. Benzene (i.e. the phenyl group, Ph) may be a particularly preferred example of a monocyclic aromatic (i.e. aryl) group. It will be understood by those skilled in the art that the expression "polycyclic" in this regard may refer to condensed aromatic ring systems in which two or more aromatic rings are fused together. This is to say that the condensed aromatic rings share at least one bond between aromatic carbon atoms, so that this shared bond as well as the carbon atoms forming the bond are part of two or more monocyclic aromatic groups that build up the polycyclic aromatic group. Preferred examples of polycyclic aromatic (i.e. aryl) groups are naphthalene (i.e. napthyl), anthracene (i.e. anthracenyl), and phenanthrene (i.e. phenanthryl).

As used throughout the present invention, unless indicated differently in the context of specific embodiments, the term "heteroaryl" may be understood in the broadest sense and may be used to denote any mono- or polycyclic heteroaromatic group, that differs from an aromatic (i.e. aryl) group in that at least one, preferably 1 to 5, more preferably 1 to 3 aromatic ring atoms are heteroatoms. A heteroatom in this context is any atom of a chemical element other than carbon and hydrogen, and preferably may be an atom that is at each occurrence independently selected from the group consisting of N (nitrogen), O, (oxygen), and S (sulfur). Preferred examples of monocyclic heteroaromatic (i.e. heteroaryl) groups comprise pyridine (i.e. pyridyl or pyridinyl), pyridazine (i.e. pyridazinyl), pyrimidine (i.e. pyrimidinyl), pyrazine (i.e. pyrazinyl), 1,2,3- triazine (i.e. 1,2,3-triazinyl), 1,2,4- triazine (i.e. 1,2,4-triazinyl), 1,3,5- triazine (i.e. 1,3,5-triazinyl), 1,2,3,4- tetrazine (i.e. 1,2,3,4-tetrazinyl), 1,2,4,5-tetrazine (i.e. 1,2,4,5-tetrazinyl), pyrrole (i.e. pyrrolyl), pyrazole (i.e. pyrazolyl), imidazole (i.e. imidazolyl), 1,2,3-triazole (i.e. 1,2,3-triazolyl), 1,2,4-triazole (i.e. 1,2,4-triazolyl), thiophene (i.e. thiophenyl), and furan (i.e. furanyl). Preferred examples of polycyclic heteroaromatic (i.e. heteroaryl) groups comprise quinoline (i.e. quinolinyl), quinazoline (i.e. quinazolinyl), quinoxaline (i.e. quinoxalinyl), benzofuran (i.e. benzofuranyl), benzothiophene (i.e. benzothiophenyl), benzimidazole (i.e. benzimidazolyl), benzothiazole (i.e. benzothiazolyl), benzoxazole (i.e. benzoxazolyl), dibenzofuran (i.e. dibenzofuranyl), and acridine (i.e. acridinyl).

Throughout the present invention, the number of carbon atoms of residues (i.e. groups or moieties or substituents) is occasionally indicated in the form of subscript numbers, for example in the form of C₁-C₅-alkyl. These subscript numbers refer to the range of allowable numbers of carbon atoms for the specific residue and in the context of the specific embodiment. When referring to aryl or heteroaryl residues, these subscript numbers refer to aromatic ring carbon atoms.

It has already been laid out that the target peptide which is to be synthesized by the method of the invention may structurally differ from the first cycle peptide P-1 obtained in step (c), as long as it comprises the first cycle peptide P-1, which, as explained above, may herein mean that the target peptide comprises the amino acid sequence (in other words peptide sequence or sequence, see above) of said first cycle peptide P-1. In order to obtain the target peptide from the first cycle peptide P-1, one or more protecting groups may for example be removed from P-1 and/or one or more additional amino acids (in other words amino acid subunits) may for example be added to the amino acid sequence of P-1.

In some embodiments, the method of the present invention further comprises the following step (d):
(d) deprotecting one protected primary or secondary aliphatic amino group in the first cycle peptide P-1 obtained in step (c), to obtain a first cycle peptide P-1' comprising one free primary or secondary aliphatic amino group.

In step (d), the amino protecting group may be removed by any means known from the state of the art, which will depend on the structure of the respective protecting group. When stating that "one protected primary or secondary aliphatic amino group in the first cycle peptide P-1" is deprotected, this may mean that in the course of step (d), exactly one primary or secondary aliphatic amino group of P-1 is deprotected, and thereafter available in free form (see above).

In some embodiments, the one primary or secondary aliphatic amino group which is deprotected in step (d) is an alpha-amino group of an alpha-amino acid moiety. In some embodiments, the one primary or secondary aliphatic amino group which is deprotected in step (d) is the N-terminal amino group of P-1 (i.e. the alpha-amino group of the N-terminal alpha-amino acid subunit of P-1). It will be understood that, since the first cycle peptide P-1 and the first cycle peptide P-1' still comprise the same amino acid subunits in the same order and since they only differ with regard to the protecting groups, they both comprise the same amino acid sequence (in other words peptide sequence or sequence) as defined herein.

In some embodiments, the method of the present invention further comprises the following step (d):
(d) deprotecting one protected primary or secondary aliphatic amino group in the first cycle peptide P-1 obtained in step (c), to obtain a first cycle peptide P-1' comprising one free primary or secondary aliphatic amino group, wherein said free primary or secondary aliphatic amino group originates from the second component C-2.

The chemical structure of the first cycle peptide P-1 is composed of one part which is derived from the first component C-1 and another part which is derived from the second component C-2, both of which are connected via the amide bond formed in step (c) of the method of the invention. When stating that the "free primary or secondary aliphatic amino group originates from the second component C-2", this may mean that said amino group was already comprised in the second component C-2 of step (b) and is consequently comprised in the part of the first cycle-peptide P-1 which is derived from the second component C-2.

In some embodiments, wherein the method of the invention comprises the aforementioned step (d), the target peptide comprises a (n+1)-th cycle peptide P-(n+1) derived from the first cycle peptide P-1' obtained in step (d) by performing n iterations of further condensing cycles, wherein n is an integer from 1 to 100, and wherein each further condensing cycle comprises the following steps (e) to (g):
(e) providing a further component C-(n+2) selected from the group consisting of an amino acid and a peptide comprising one free carboxyl group or one activated carboxyl group, and comprising at least one protected primary or secondary aliphatic amino group, wherein any primary or secondary aliphatic amino groups and any other carboxyl groups in C-(n+2) are protected,
(f) condensing the first cycle peptide P-1' obtained in step (d) or, if one or more iterations of further condensing cycles have already been performed, the (n+1)th cycle peptide P-(n+1)' obtained in step (g) of the preceding condensing cycle, and the further component C(n+2) of step (e) under suitable conditions, to form an amide bond between the free primary or secondary aliphatic amino group of the first cycle peptide P-1' of step (d) or the (n+1)-th cycle peptide P-(n+1)' of step (g) of the preceding condensing cycle and the free or activated carboxyl group of the further component C-(n+2) of step (e), to obtain an (n+1)-th cycle peptide P-(n+1), and
(g) deprotecting one protected primary or secondary aliphatic amino group in the (n+1)-th cycle peptide P-(n+1) obtained in step (f), to obtain a (n+1)-th cycle peptide P-(n+1)' comprising one free primary or secondary aliphatic amino group,
wherein, the further component C-(n+2) is selected independently for each iteration n of further condensing cycles, and
wherein in the final condensing cycle, step (g) may be omitted.

In this context, the expression "the target peptide comprises a (n+1)-th cycle peptide P-(n+1)" may be understood as laid out above for a "target peptide comprising a first cycle peptide P-1". Thus, when stating that "the target peptide comprises a (n+1)-th cycle peptide P-(n+1)" this may mean that the target peptide comprises the amino acid sequence (in other words peptide sequence or sequence, see above) of said (n+1)-th cycle peptide P-(n+1). As laid out above, in order for the target peptide to comprise (or share) the amino acid sequence of the (n+1)-th cycle peptide P-(n+1), it is herein not required that the amino acid subunits of said amino acid sequence carry the same protecting groups in the (n+1)-th cycle peptide P-(n+1) and the target peptide. Furthermore, the target peptide may for example comprise more amino acid subunits than the (n+1)-th cycle peptide P-(n+1), in which case one or both of the terminal amino acids of the shared sequence may not be terminal amino acid(s) of the target peptide (see above).

In some embodiments, the (n+1)-th cycle peptide P-(n+1) and the target peptide which is to be synthesized by the method disclosed herein consist of the same amino acid sequence.

Unless indicated differently in the context of specific embodiments, the (n+1)-th cycle peptide P-(n+1) of the invention is a peptide as defined herein. This is to say that, unless indicated differently, all of the previous explanations relating to a peptide in general are applicable. In some embodiments, the (n+1)-th cycle peptide P-(n+1) of the invention is a linear peptide.

Step (e) of the present method is: "providing a further component C-(n+2) selected from the group consisting of an amino acid and a peptide comprising one free carboxyl group or one activated carboxyl group, and comprising at least one protected primary or secondary aliphatic amino group, wherein any primary or secondary aliphatic amino groups and any other carboxyl groups in C-(n+2) are protected". In this context, the terms "amino acid", "peptide", "free carboxyl group", "primary or secondary aliphatic amino group", and "protected" may be understood as laid out above.

The term "activated carboxyl group" may herein refer to a carboxyl group, which is activated towards amide bond formation, in the context of step (e) activated towards amide bond formation (and thus condensation) in step (f) with the free primary or secondary aliphatic amino group of the first cycle peptide P-1' of step (d) or the (n+1)-th cycle peptide P-(n+1)' of step (g) of the preceding condensing cycle. Thus, the term "activated carboxyl group" may in the herein refer to a functional group derived from a carboxyl group that is more reactive towards amide bond forming reactions, in particular peptide bond forming reactions, than the free carboxyl group itself. A person skilled in the art is familiar with the means of activating a carboxyl group for amide bond formation. Preferred examples of activated carboxyl groups comprise so-called active esters formed from the carboxyl group and an -OH functional group of another species which forms a good leaving group. Preferred examples of active esters comprise active esters formed from the further component C-(n+2) (via its free carboxyl group) and the first additive A-1 or the second additive A-2 (via the N-OH functional group of these additives) or related (coupling) additives used in peptide synthesis such as N-hydroxysuccinimide (HOSu) or N-hydroxy-5-norbornene-2,3-dicarboximide (HONB). The term "activated carboxyl group" may e.g. also comprise an acyl azide functional group which may for example be obtained by converting the free carboxyl group of the further component C-(n+2) into an alkyl ester, followed by reaction with hydrazine, followed by treatment with a nitirt such as sodium nitrit, isoamyl nitrit or tert-butyl nitrit. In fact, any activated carboxyl groups known from and used in the context of peptide synthesis may be employed as the activated carboxyl group of a further component C-(n+2).

The requirement that "any primary or secondary aliphatic amino groups and any other carboxyl groups in C-(n+2) are protected" is self-explaining based on what has been laid out so far. Briefly, a further component C-(n+2) comprises only a single carboxyl group (which is a free carboxyl group or an activated carboxyl group) capable of engaging in an amide bond forming condensation reaction under the conditions of step (f), and does not comprise a primary or secondary aliphatic amino group capable of engaging in an amide bond forming condensation reaction under the conditions of step (f).

In some embodiments, the one free or the one activated carboxyl group of each further component C-(n+2) is an alpha-carboxyl group of an alpha-amino acid moiety. In some embodiments, a further component C-(n+2) comprises one free carboxyl group and does not comprise an activated carboxyl group. In some embodiments, a further component C-(n+2) comprises one free carboxyl group, which is an alpha-carboxyl group of an alpha-amino acid moiety, and does not comprise an activated carboxyl group.

In some embodiments, in at least one iteration of a further condensing cycle, the further component C-(n+2) is an amino acid as defined herein. In some embodiments, in at least one iteration of a further condensing cycle, the further component C-(n+2) is an alpha-amino acid as defined herein. In some embodiments, in each iteration of a further condensing cycle, the further component C-(n+2) is an amino acid as defined herein. In some embodiments, in each iteration of a further condensing cycle, the further component C-(n+2) is an alpha-amino acid as defined herein. In some embodiments, in at least one iteration of a further condensing cycle, the further component C-(n+2) is a peptide as defined herein. In some embodiments, in each iteration of a further condensing cycle, the further component C-(n+2) is a peptide as defined herein. In some embodiments, in at least one iteration of a further condensing cycle, the further component C-(n+2) is a peptide selected from the group consisting of a dipeptide and a tripeptide. In some embodiments, in each iteration of a further condensing cycle, the further component C-(n+2) is a peptide selected from the group consisting of a dipeptide and a tripeptide. In some embodiments, in at least one iteration of a further condensing cycle, the further component C-(n+2) selected from the group consisting of an alpha-amino acid, a dipeptide, and a tripeptide. In some embodiments, in each iteration of a further condensing cycle, the further component C-(n+2) is a peptide selected from the group consisting of an alpha-amino acid, a dipeptide, and a tripeptide. In some embodiments, in at least one iteration of a further condensing cycle, the further component C-(n+2) selected from the group consisting of an alpha-amino acid and a dipeptide. In some embodiments, in each iteration of a further condensing cycle, the further component C-(n+2) is selected from the group consisting of an alpha-amino acid and a dipeptide. Preferred examples of such dipeptides comprise the well-known pseudoproline dipeptides (see above).

When stating that "the further component C-(n+2) is selected independently for each iteration n of further condensing cycles", this may mean that the further component C-(n+2) provided in an iteration of step (e) and subsequently condensed in a respective step (f) may have the same or a different chemical structure as compared to any other further component C-(n+2) that may optionally be provided in another iteration of step (e) and subsequently be condensed in a respective step (f).

The term "providing a further component C-(n+2)" in step (e) may be understood in the broadest sense and may refer to any process of obtaining said second further component C-(n+2). The further component C-(n+2) may be obtained by means of amino acid or peptide synthesis, wherein any synthetic strategies may be applied. A person skilled in the art is familiar with methods for amino acid and peptide synthesis and knows how to apply them to the synthesis of a further component C-(n+2). The further component C-(n+2) may be commercially available, in particular if it is an alpha-amino acid.

Step (f) is: "condensing the first cycle peptide P-1' obtained in step (d) or, if one or more iterations of further condensing cycles have already been performed, the (n+1)-th cycle peptide P-(n+1)' obtained in step (g) of the preceding condensing cycle, and the further component C(n+2) of step (e) under suitable conditions, to form an amide bond between the free primary or secondary aliphatic amino group of the first cycle peptide P-1' of step (d) or the (n+1)-th cycle peptide P-(n+1)' of step (g) of the preceding condensing cycle and the free or activated carboxyl group of the further component C-(n+2) of step (e), to obtain an (n+1)-th cycle peptide P-(n+1)".

The meaning of a condensation reaction has been laid out above. Likewise, the "first cycle peptide P-1' obtained in step (d)", the "further component C-(n+2) of step (e)", the "(n+1)-th cycle peptide P-(n+1)" as well as the meaning of a "free primary or secondary aliphatic amino group" and a "free or activated carboxyl group" have been explained above. The "(n+1)-th cycle peptide P-(n+1)' obtained in step (g)" will be explained for step (g). Each of the first cycle peptide P-1' obtained in step (d) and the (n+1)-th cycle peptide P-(n+1)' obtained in step (g) comprises exactly one primary or secondary aliphatic amino group, preferably an alpha-amino group of an alpha-amino acid moiety, which is capable of engaging- in the amide bond forming condensation reaction of step (f) with the respective further component C-(n+2) of step (e). Additionally, neither the first cycle peptide P-1' obtained in step (d) nor the (n+1)-th cycle peptide P-(n+1)' obtained in step (g) comprise a free or activated carboxyl group. Furthermore, a further component C-(n+2) comprises a single carboxyl group (the one free or the one activated carboxyl group) which is capable of engaging in the amide bond forming condensation reaction of step (f) with the free primary or secondary aliphatic amino group of either P-1' or P-(n+1)'. Thus, the amide bond forming condensation reaction of step (f) may typically only take place between the indicated functional groups.

The term "under suitable conditions" of step (f) may herein, unless indicated differently in the context of specific embodiments, refer to any reaction conditions that allow for the formation of the desired amide bond between the free primary or secondary aliphatic amino group of the first cycle peptide P-1' of step (d) or the (n+1)-th cycle peptide P-(n+1)' of step (g) of the preceding condensing cycle and the free or activated carboxyl group of the further component C-(n+2) of step (e), to obtain an (n+1)-th cycle peptide P-(n+1). Such conditions will obviously involve the combination of the further component C(n+2) of step (e) and either the first cycle peptide P-1' of step (d) or the (n+1)-th cycle peptide P-(n+1)' of step (g) of the preceding condensing cycle in a reaction vessel or reactor wherein the form and kind of reaction vessel or reactor is not particularly limited. In fact, any kind of reaction vessel or reactor used for peptide synthesis in the state of the art may be used for the method disclosed herein. For example, step (f) of the invention may be performed in an industrial reaction vessel equipped with a mechanical stirrer or in a reaction vessel of a peptide synthesizer. Typically the "suitable conditions" (i.e. the reaction conditions of the amide bond forming condensation reaction of step (f)) will also involve the dissolution of the further component C(n+2) of step (e) and either the first cycle peptide P-1' of step (d) or the (n+1)-th cycle peptide P-(n+1)' of step (g) of the preceding condensing cycle in a solvent or a solvent mixture, wherein the examples for the first cycle solvent are also valid examples of a solvent or solvent mixture for a step (f) of the method disclosed herein, in particular DMF may be used. Furthermore, the term "suitable conditions" of step (f) may include the addition of a suitable coupling agent, in particular a carbodiimide coupling agent as defined herein and/or additives (e.g. a first additive A-1 and/or a second additive A-2 as defined herein or any other additive used for peptide synthesis), wherein such a coupling agent and/or additives may typically only be added if the respective further component C(n+2) comprises a free carboxyl group and not an activated carboxyl group. Furthermore, an organic base such as triethylamine (TEA) or diisopropylethylamine (DIPEA) (which constitute well-known non-nucleophilic amine bases) may be added.

Non-limiting examples of suitable combinations of coupling agents and/or additives which may be used in step (f), if the further component C-(n+2) comprises a free carboxyl group (and not an activated carboxyl group) comprise: DIC plus Oxyma, DIC plus 2-HOPO, DIC plus HOAt, DIC plus HOBt, and
- (benzotriazolyl)tetramethyluronium tetrafluoroborate (TBTU) plus DIPEA,
- 3-(diethoxy-phosphoryloxy)-3*H*-benzo[d][1,2,3]triazin-4-one (DEPBT) plus DIPEA,
- N-[(7-Aza-1*H*-benzotriazol-1-yl)(dimethylamino)-methylene]-N-methylmethanaminium tetrafluoroborate N-oxide (TATU) plus DIPEA,
- 2-(1*H*-benzotriazol-1-yl)-1,1,3,3-tetramethyluronium hexafluorophosphate (HBTU) plus DIPEA, and
- N-[(7-Aza-1*H*-benzotriazol-1-yl)(dimethylamino)-methylene]-N-methylmethanaminium hexafluorophosphate N-oxide (HATU) plus DIPEA.

For example, if the further component C-(n+2) comprises a free carboxyl group, in step (f), 1.0-5.0 equivalents, preferably 2.0 equivalents of the further component C(n+2) of step (e) relative to the first cycle peptide P-1' of step (d) or the (n+1)-th- cycle peptide P-(n+1)' of step (g) of the preceding condensing cycle (whose molar amount constitutes 1.0 equivalent) may be used and the amide bond forming condensation reaction may be carried out at a temperature in the range of 10-90 °C, preferably 20-50 °C, wherein shorter condensing times may be used at elevated temperatures. For example, when performing the amide bond forming condensation reaction of step (f) at a temperature of about 35 °C, the condensing time may be in a range of 30-120 min, or 45-90 min. For example, when performing the amide bond forming condensation reaction of step (f) at a temperature of about 50 °C, the condensing time may be in a range of 5-60 min, or 20-40 min. The use of 1.0-6.0 equivalents of a carbodiimide coupling agent such as DIC alongside 1.0-6.0 equivalents an additive such as HOBt or Oxyma may be preferred, wherein again, all equivalents are given relative to the total molar amount of the first cycle peptide P-1' of step (d) or the (n+1)-th cycle peptide P-(n+1)' of step (g) of the preceding condensing cycle.

For example, if the further component C-(n+2) comprises a free carboxyl group, 1.0-5.0 equivalents, preferably 3.0 equivalents, of the further component C-(n+2) of step (e), 1.0-6.0 equivalents, preferably 3.0 equivalents of a carbodiimide coupling agent such as DIC, and 1.0-6.0 equivalents, preferably 3.0 equivalents, of an additive such as HOBt may be used in step (f), which may be performed at a temperature in the range of 20-50 °C and for a condensing time of 5-360 min or 20-180 min, for example at 35 °C for 15-60 min or at 50 °C for 10-30 min. As a further example, if the further component C-(n+2) comprises a free carboxyl group, 1.0-5.0 equivalents, preferably 2.0 equivalents, of the further component C-(n+2) of step (e), 1.0-6.0 equivalents, preferably 4.0 equivalents of a carbodiimide coupling agent such as DIC, and 1.0-6.0 equivalents, preferably 3.0 equivalents, of an additive such as Oxyma may be used in step (f), which may be performed at a temperature in the range of 20-50 °C, e.g. for a condensing time of 5-360 min or 20-180 min, for example at 35 °C, e.g. for 15-60 min or at 50 °C for 10-30 min. For example, if in an amide bond forming condensation reaction such as step (c) or step (f) of the method disclosed herein, the second component C-2 (for step (c)) or the further component C-(n+2) comprising a free carboxyl group is selected from the group consisting of histidine, cysteine, and alpha-phenylglycine, preferably histidine or cysteine, 1.0-3.0 equivalents, preferably 2.0 equivalents, of the second component C-2 or the further component C-(n+2), 1.0-6.0 equivalents, preferably 4.0 equivalents, of 2-HOPO, 1.0-5.0 equivalents, preferably 3.0 equivalents, of Oxyma, and 1.0-6.0 equivalents, preferably 4.0 equivalents, of a carbodiimide coupling agent, e.g. DIC, may be used at a temperature in the range of 20-50 °C, e.g. for a condensing time of 5-360 min or 20-180 min, for example at 35 °C, e.g. for 15-100 min or at 50 °C for 10-50 min. When relating to step (f), all equivalents are given relative to the total molar amount of the first cycle peptide P-1' of step (d) or the (n+1)-th cycle peptide P-(n+1)' of step (g) of the preceding condensing cycle. These may be approximated as explained with respect to the determination of equivalents with respect to step (c).

The conditions of step (c) of the method of the invention may for example be applied to step (f) of one or more further condensing cycles, with the proviso that the respective further component C-(n+2) comprises a free carboxyl group, preferably an alpha-carboxyl group of an alpha-amino acid moiety.

Herein, to say that the conditions of step (c) are applied to or in a step (f) may mean that the respective step (f) then is: "condensing the first cycle peptide P-1' obtained in step (d) or, if one or more iterations of further condensing cycles have already been performed, the (n+1)-th cycle peptide P-(n+1)' obtained in step (g) of the preceding condensing cycle, and the further component C-(n+2) of step (e) in the presence of a carbodiimide coupling agent, a first additive A-1, a second additive A-2, and an (n+1)-th cycle solvent, to form an amide bond between the free primary or secondary aliphatic amino group of the first cycle peptide P-1' of step (d) or the (n+1)-th cycle peptide P-(n+1)' of step (g) of the preceding condensing cycle and the free carboxyl group of the further component C-(n+2) of step (e), to obtain an (n+1)-th cycle peptide P-(n+1), wherein for the carbodiimide coupling agent, the first additive A-1, and the first additive A-2, the aforementioned definitions (and embodiments) apply, and wherein the (n+1)-th cycle solvent is defined similarly to the first cycle solvent." To a step (f) to which the conditions of step (c) apply, the embodiments provided herein related to the conditions of step (c) (e.g. temperature, condensing time, order of addition of species, ...) may also apply.

In some embodiments, in step (f) of each further condensing cycle, in which the further component C-(n+2) is a chiral alpha-amino acid selected from the group consisting of histidine, cysteine, and alpha-phenylgycine and comprises a free alpha-carboxyl group, the conditions of step (c) are applied. In some embodiments, in step (f) of each further condensing cycle, in which the further component C-(n+2) is a chiral alpha-amino acid selected from the group consisting of histidine and cysteine, and comprises a free alpha-carboxyl group, the conditions of step (c) are applied. In some embodiments, in step (f) of each further condensing cycle, in which the further component C-(n+2) is histidine and comprises a free alpha-carboxyl group, the conditions of step (c) are applied. In some embodiments, in step (f) of each further condensing cycle, in which the further component C-(n+2) is cysteine and comprises a free alpha-carboxyl group, the conditions of step (c) are applied.

It should be noted that even when stating that "each further condensing cycle" (or "further condensing cycles, each of which") comprises the steps (e) to (g), this may, unless indicated differently in the context of specific embodiments, mean that each completed further condensing cycle comprises the steps (e) to (g), wherein only the final (i.e. the last) condensing cycle may be an incomplete condensing cycle and not comprise a step (g).

Step (g) of the method of the present invention is: "deprotecting one protected primary or secondary aliphatic amino group in the (n+1)-th cycle peptide P-(n+1) obtained in step (f), to obtain a (n+1)-th cycle peptide P-(n+1)' comprising one free primary or secondary aliphatic amino group".

In step (g), the amino protecting group may be removed by any means used in peptide synthesis, which will depend on the structure of the respective protecting group. When stating that "one protected primary or secondary aliphatic amino group in the (n+1)-th cycle peptide P-(n+1)" is deprotected, this may mean that in the course of step (g), exactly one primary or secondary aliphatic amino group of the respective (n+1)-th cycle peptide P-(n+1) is deprotected, and thereafter available in free form.

In some embodiments, the one primary or secondary aliphatic amino group which is deprotected in step (g) is an alpha-amino group of an alpha-amino acid moiety. In some embodiments, the one primary or secondary aliphatic amino group which is deprotected in step (d) is the N-terminal amino group of the (n+1)-th cycle peptide P-(n+1) (i.e. the alpha-amino group of the N-terminal alpha-amino acid subunit of (n+1)-th cycle peptide P-(n+1)). It will be understood that, since the (n+1)-th cycle peptide P-(n+1) and the (n+1)-th cycle peptide P-(n+1)' still comprise the same amino acid subunits in the same order and since they only differ with regard to the protecting groups, they both comprise the same amino acid sequence (in other words peptide sequence or sequence) as defined herein.

In some embodiments, step (g) is: "deprotecting one protected primary or secondary aliphatic amino group in the (n+1)-th cycle peptide P-(n+1) obtained in step (f), to obtain a (n+1)-th cycle peptide P-(n+1)' comprising one free primary or secondary aliphatic amino group, wherein said free primary or secondary aliphatic amino group originates from the further component C-(n+2)".

The chemical structure of the (n+1)-th cycle peptide P-(n+1) is composed of one part which is derived from the first cycle peptide P-1' obtained in step (d) or the (n+1)-th cycle peptide P-(n+1)' obtained in step (g) of the preceding condensing cycle and another part which is derived from the further component C(n+2) of step (e), both of which are connected via the amide bond formed in step (f) of the method of the invention. When stating that the "free primary or secondary aliphatic amino group originates from the further component C-(n+2)", this may mean that said amino group was already comprised in the further component C-(n+2) of step (e) and is consequently comprised in the part of the (n+1)-th cycle peptide P-(n+1) which is derived from the further component C-(n+2).

In some embodiments, wherein the target peptide comprises a (n+1)-th cycle peptide P-(n+1) derived from the first cycle peptide P-1' obtained in step (d) by performing n iterations of further condensing cycles, each of which comprises the aforementioned steps (e) to (g):
- the one primary or secondary aliphatic amino group which is deprotected in step (d) is an alpha-amino group of an alpha-amino acid moiety,
- the one primary or secondary aliphatic amino group which is deprotected in step (g) of each further condensing cycle is an alpha-amino group of an alpha-amino acid moiety, and
- the one free or the one activated carboxyl group of each further component C-(n+2) is an alpha-carboxyl group of an alpha-amino acid moiety.

In the case that step (d) is followed by a step (e), the amino protecting group that is removed in step (d) may be regarded as a temporary protecting group. As used herein, a temporary protecting group may be a protecting group which is removed during a condensing cycle or in preparation of a condensing cycle. In contrast, as used herein, a permanent protecting group may be a protecting group which is not removed during a condensing cycle, but only after the target peptide has been assembled. Examples of temporary protecting groups comprise amino and carboxyl protecting groups which are removed prior to an amide bond forming condensation reaction to provide the functional groups for the amide bond formation. Examples of permanent protecting groups comprise a support and protecting groups of side chain residues. In the case that a step (g) is followed by a step (e) of another condensing cycle, the amino protecting group that is removed in this step (g) may be regarded as a temporary protecting group. It may be desirable to cleave such temporary amino protecting groups selectively, i.e. under conditions under which no further amino protecting groups, preferably no other protecting groups at all, are removed. A person skilled in the art knows how to selectively remove certain protecting groups and how to select suitable protecting groups in the first place, for example from what has been laid out herein, from F. Alberico et al., Chem. Rev. 2009., 109(6), 2455-2504, and from similar publications. For example, the temporary protecting groups which are to be selectively removed in step (d) and step(s) (g) may be selected so that they may be removed using a base, while all permanent protecting groups (including the support) may then be selected so that they may be (mostly or essentially or completely) stable (i.e. not removed) under the conditions of steps (d) and (g). These permanent protecting groups (including the support) may then for example be cleaved (i.e. removed) by treatment with an acid such as trifluoroacetic acid (TFA) or hydrochloric acid.

In some embodiments, wherein the target peptide comprises a (n+1)-th cycle peptide P-(n+1) derived from the first cycle peptide P-1' obtained in step (d) by performing n iterations of further condensing cycles, each of which comprises the aforementioned steps (e) to (g), the amino protecting group which is removed when deprotecting one protected primary or secondary aliphatic amino group in step (d) and step (g) is a fluorenylmethyloxycarbonyl-type protecting group, in particular the Fmoc group. The term "fluorenylmethyloxycarbonyl-type protecting group" has been explained above. In some embodiments, wherein the target peptide comprises a (n+1)-th cycle peptide P-(n+1) derived from the first cycle peptide P-1' obtained in step (d) by performing n iterations of further condensing cycles, each of which comprises the aforementioned steps (e) to (g), the amino protecting group which is removed when deprotecting one protected primary or secondary aliphatic amino group in step (d) and the amino protecting group which is removed when deprotecting one protected primary or secondary aliphatic amino group in each iteration of step (g) is a fluorenylmethyloxycarbonyl-type protecting group, in particular the Fmoc group. Thus, in some embodiments, the second component C-2 and each further component C-(n+2) comprise exactly one primary or secondary aliphatic amino group which is protected with a fluorenylmethyloxycarbonyl-type protecting group, in particular the Fmoc group. In some embodiments, the second component C-2 and each further component C-(n+2) comprise exactly one primary or secondary aliphatic amino group which is protected with a fluorenylmethyloxycarbonyl-type protecting group, in particular the Fmoc group, wherein said primary or secondary aliphatic amino group is an alpha-amino group of an alpha-amino acid moiety.

In the well-known Fmoc-SPPS, which constitutes a preferred way of putting the method of the invention into practice, the temporary amino protecting groups are typically protected using the Fmoc protecting group while the permanent protecting groups are typically selected so that they may be removed upon treatment with an acid such as TFA or hydrochloric acid, preferably TFA. Non-limiting examples of permanent protecting groups which are stable towards conditions under which the Fmoc group is commonly removed in Fmoc-SPPS comprise:
- the 2,2,4,6,7-pentamethyldihydrobenzofuran-5-sulfonyl (Pbf) group commonly employed for the protection of the guanidine group in the side chain residue of arginine,
- the tert-butyl ester group or the benzyl ester group commonly formed to protect the carboxyl groups in the side chain residues of aspartic acid and glutamic acid,
- the trityl (triphenylmethyl) group commonly employed to protect the N^{τ} atom (in 1 position of the imidazole ring) of the imidazole moiety in the histitine side chain and the sulfur atom in the sulfhydryl group of the cysteine side chain (the acetamidomethyl (Acm) group may also be used for cysteine protection) and the amide group in the side chain residues of asparagine and glutamine (the 4-methyltrityl (Mtt) group may also be used for asparagine and glutamine protection),
- the Boc group commonly employed to protect the amino group in the side chain residue of lysine (the Cbz group may also be employed for lysine protection) as well as the nitrogen atom comprised in the indole ring of tryptophan, and
- the tert-butyl ether group commonly formed from the hydroxyl groups in the side chain residues of serine (the benzyl ether may also be used for serine protection), threonine, and tyrosine.

Fluorenylmethyloxycarbonyl-type protecting groups, in particular the Fmoc group, may for example be removed by treating the respective peptide or support-bound peptide with a solution of piperidine in N,N-dimethylformamide (DMF) with a concentration of 20% (v/v) of piperidine. The temperature may for example be in a range of 10-90 °C, preferably 20-50 °C. For convenience, the deprotection may simply be performed at ambient temperature (i.e. (approximately) 20 °C). If the preceding and/or subsequent amide bond forming condensation reaction(s) are performed at a certain temperature, the deprotecting step may for practical reasons simply be performed at the same temperature. In general, shorter reaction times are to be expected at elevated temperatures. While these conditions may indeed be preferred, a person skilled in the art is familiar with manifold further reagents and conditions that allow for the removal of such base-labile protecting groups and that may be used to execute steps (d) and (g). For example, popular bases for the purpose of removing base-labile amino protecting groups comprise, e.g., secondary amines such as the aforementioned piperidine and 4methylpiperidine. Suitable solvents comprise, e.g., DMF, NMP, dimethyl sulfoxide, dichloromethane, tetrahydrofuran, acetonitrile, toluene, and mixtures thereof. If the reaction vessel in which the amide bond forming condensation reaction of step (c) and/or (f) is performed is heated to a certain temperature, the deprotecting reaction of step (d) and/or (g) may for practical reasons also be performed at the same temperature, for example at 35 °C or 50 °C. At such elevated temperatures, the concentration of the employed base may be reduced (e.g. 5-15% or 10% piperidine in DMF (v/v) may be used). Such a deprotecting step may comprise the repeated (e.g. twofold) treatment of the peptide or support-bound peptide with a suitable base. Usually, a base-labile amino protecting group such as the Fmoc group may be cleaved by a short treatment (1 to 20 minutes, e.g. 1, 2, 3, 4, 5, 6, 7, 8, 9, 10, 11, 12, 13, 14, 15, 16, 17, 18, 19 or 20 minutes) with 5-50%, preferably 10-20%, piperidine in DMF (v/v). It has been found that even slight variations of the reagent may considerably accelerate the deprotection, e.g. the use of: 1-5% DBU in DMF, 20% piperidine and 1-5% DBU in DMF, 20% piperidine in NMP, or 20% piperidine in DMF at 45 °C. Moreover, acceleration of the deprotection reaction may be achieved by microwave irradiation. On the other side, in some cases, the nature of the respective peptide may render the use of milder treatments advantageous. Particular mild conditions for removing a base-labile amino protecting group are, e.g., 0.1 M HOBt plus 20% piperidine in DMF, 50 % morpholine in DMF, 2% HOBt plus 2% hexamethyleneimine plus 25% N-methylpyrrolidine in 50% DMSO in NMP. A skilled artisan knows how to optimize and adapt deprotection conditions at each iteration of an iterative peptide synthesis, if necessary.

It should also be noted that in support-assisted peptide synthesis such as Fmoc-SPPS, one or more washing steps are typically performed after an amide bond forming condensation reaction such as steps (c) and (f) of the invention and after a deprotecting step such as steps (d) and (g) of the invention. It will be understood by a person skilled in the art without further notice that such washing steps may also form part of the method of the present invention. Such washing steps may for example be performed if the first component C-1 and thus the first cycle peptide P-1, the first cycle peptide P-1', the (n+1)-th cycle peptide P-(n+1), and the (n+1)-th cycle peptide P-(n+1)' are covalently linked to a support, preferably to a solid support. Such washing steps may in particular be performed after step (c), after step (d), after each iteration of step (f), and after each iteration of step (g). A washing step typically comprises contacting the support-bound peptide with a solvent or a solvent mixture (e.g. DMF and/or propan-2-ol (IPA)) for a certain time (e.g. stirring in a reaction vessel), followed by filtration. More than one washing step may typically be performed in a row. Additionally, for example, so-called capping steps may form part of the method disclosed herein. Such capping steps may in particular be performed after an amide bond forming condensation reaction of step (c) and each iteration of step (f), to block any unreacted (i.e. free) amino groups that may be present. Capping may for example be achieved by contacting the support-bound peptide with a solution of acetic anhydride and an organic base such as pyridine or sym-collidine in DMF, followed by filtration (e.g. draining the solvent through a membrane of the reaction vessel which retains the solid support carrying the growing peptide chains). A capping step may typically also be followed by one or more washing steps.

Additionally, for example, so-called capping steps may form part of the method disclosed herein. During a capping step, undesired free amino groups at the growing peptide chains are typically blocked from engaging in subsequent amide bond forming condensation reactions. Capping may usually be achieved by means of acylation, in particular acetylation, of these amino groups. Such capping steps may in particular be performed after an amide bond forming condensation reaction of step (c) and each iteration of step (f), to block any unreacted (i.e. free) amino groups that may be present. Capping may for example be achieved by contacting the support-bound peptide with a solution of acetic anhydride and optionally an organic base such as pyridine, sym-collidine, TEA or DIPEA, and optionally an additive such as HOBt, HOAt or Oxyma in DMF. For example, 1.0-8.0 equivalents of acetic anhydride may be used. For example, 1.0-8.0 equivalents of acetic anhydride and 1.0-10.0 eq of the organic base may be used. For example, 4.0-6.0 equivalents of acetic anhydride and 4.0-8.0 equivalents of a pyridine derivative may be used. Optionally, catalytic amounts of HOBt or HOAt may be added. Alternatively, catalytic amounts of Oxyma may be added. For example, 1.0-3.0 equivalents of acetic anhydride may be used in combination with 0.10-1.00 equivalents of an organic base such as pyridine, sym-collidine, TEA, and DIPEA. Optionally, HOBt or HOAt or Oxyma may be used in a molar amount of more than 1.0 equivalent. Alternatively, HOBt, HOAt or Oxyma may be added in a molar amount of less than 1.0 equivalent, for example in a molar amount of 0.01-0.50 equivalents, 0.01-0.50 equivalents, 0.01-0.10 equivalents or 0.01-0.05 equivalents. For example, 1.0-3.0 equivalents of acetic anhydride may be used in combination with 0.10-1.00 equivalents of an organic base such as pyridine, sym-collidine, TEA, and DIPEA, and 0.01-0.50 equivalents of HOBt. For example, 1.0-3.0 equivalents of acetic anhydride may be used in combination with 0.10-1.00 equivalents of an organic base such as pyridine, sym-collidine, TEA, and DIPEA, and 0.01-0.50 equivalents of HOAt. For example, 0.50-2.00 equivalents of acetic anhydride may be used in combination with 0.10-1.00 equivalents of an organic base such as pyridine, sym-collidine, TEA, and DIPEA, and 0.01-0.05 equivalents of Oxyma. Alternatively, capping may for example be achieved by contacting the support-bound peptide with a solution of acetic acid and a coupling agent such as a carbodiimide and optionally an organic base such as pyridine, sym-collidine, TEA or DIPEA, and optionally an additive such as Oxyma, HOBt or HOAt or in DMF. For example, acetic acid and DIC or DCC may be used. For example, 1.0-15.0 equivalents of acetic acid may be used. For example, 1.0-15.0 equivalents of acetic acid and 1.0-30.0 of DIC, DCC or EDC may be used. Optionally, a coupling additive such as HOBt, HOAt, and Oxyma may be added. For example, 1.0-15.0 equivalents of acetic acid and 1.0-30.0 of DIC, DCC or EDC, and 1.0-15.0 equivalents of HOBt, HOAt or Oxyma may be used. For example, about 2.0 equivalents of acetic acid, about 6.0 equivalents of DIC, and 2.0 equivalents of Oxyma in DMF may be used. A capping step may also be performed by adding the capping reagent(s) directly into the reaction mixture of the preceding amide bond forming condensation reaction. This is to say that the capping reagents may optionally be added directly into the reaction vessel once step (c) or an iteration of step (f) has been performed. For example, 1.0-3.0 equivalents of neat acetic anhydride may be added. Alternatively, 2.0-5.0 equivalents of neat acetic acid may be added. Alternatively, a solution of 1.0-3.0 equivalents of acetic anhydride in the solvent of the amide bond forming condensation reaction may be added. Alternatively, a solution of 2.0-5.0 equivalents of acetic acid in the solvent of the amide bond forming condensation reaction may be added. DMF may be used as solvent. In the context of a capping step, all equivalents are reported relative to the total molar amount of the first component C-1 as defined herein. The capping step may for example be performed at a temperature in the range of 10-90 °C, preferably 20-50 °C, and may for practical reasons simply be performed at (approximately) the same temperature at which the preceding amide bond forming condensation reaction was performed. A capping reaction may typically be performed for a few minutes, e.g. for 1-30 minutes or 5-30 minutes, typically for 3-15 minutes. A capping step may typically also be followed by one or more washing steps.

If for example the first component C-1 is covalently linked to a support, the first cycle peptide P-1, the first cycle peptide P-1', the (n+1)-th cycle peptide P-(n+1), the (n+1)-th cycle peptide P-(n+1)', and even the target peptide may typically also be covalently linked to said support. It may then be desired to cleave the target peptide from the support, which herein has the same meaning as to cleave the support from the target peptide.

In some embodiments, the method of the present invention further comprises a step of cleaving the support and optionally one or more protecting groups from the target peptide to obtain a support-cleaved target peptide, which may optionally be followed by a step of purifying the supportcleaved target peptide to obtain a purified supportcleaved target peptide. It will be understood that the conditions under which "the support and optionally one or more protecting groups" are cleaved from the target peptide may typically be selected so that the amino acid sequence remains intact. In this context, the term "intact" may mean that the amino acid sequence does not change and thus, the target peptide and the support-cleaved target peptide comprise the same amino acid sequence as defined herein. A support-cleaved target peptide obtained from cleaving a target peptide from an amide resin as defined herein may comprise a carboxamide group (-C(O)NH₂), wherein the amine part of the carboxamide group may be derived from the resin. For example, if the target peptide was covalently linked to an amino group at the amide resin via its C-terminal carboxyl group, said C-terminal carboxyl group may be a C-terminal carboxamide group in the support-cleaved target peptide.

As laid out above, when putting the method of the invention into practice, it may be preferred to select the permanent protecting groups, including the support, so that they may be cleaved by treatment with an acid such as TFA or hydrochloric acid. Thus, in some embodiments, the "step of cleaving the support and optionally one or more protecting groups from the target peptide" comprises treating the target peptide (the support-bound target peptide) with an acid, preferably an acid selected from the group consisting of TFA and hydrochloric acid, in particular with TFA. For example, TFA/water (95:5 v/v) may be used. As known to those skilled in the art a target peptide from which a support and one or more protecting groups are to be removed may typically be treated with a so-called cleavage composition (herein also referred to as cleavage cocktail) comprising the respective acid and usually also one or more scavengers which are supposed to trap any reactive species potentially formed during a cleavage step, for example from the cleaved protecting group(s). Such scavengers are known to those skilled in the art and may for example comprise thiols or silanes. Non-limiting examples of cleavage compositions (also cleavage cocktails) that may be employed to remove acid labile permanent protecting groups including the support comprise TFA/H₂O/1,2-ethanedithiol (EDT) (90:5:5, v/v/v) or TFA/triisoprpopyl silane (TIS)/H₂O/EDT (92.5:2.5:2.5:2.5, v/v/v/v). Thus, in some embodiments, the "step of cleaving the support and optionally one or more protecting groups from the target peptide" comprises treating the target peptide (the support-bound target peptide) with a cleavage composition (or cleavage cocktail) comprising an acid selected from the group consisting of TFA and hydrochloric acid, preferably TFA, and a scavenger selected from the group consisting of a thiol and a silane, preferably EDT and TIS. Such a "step of cleaving the support and optionally one or more protecting groups from the target peptide" may for convenience simply be performed at room temperature (i.e. (approximately) 20 °C), but may for example also be performed under cooling or heating, for example at a temperature in the range of 0-50 °C or 10-30 °C. It should be noted that such conditions may not just be applied to the cleavage of a solid support, but also to tag-assisted LPPS as defined herein (see for example: D. Takahashi et al. Angew. Chem. Int. Ed. 2017, 56, 7803-7807; EP3778621A1).

A "step of cleaving the support and optionally one or more protecting groups from the target peptide" may initially afford a solution of the support-cleaved target peptide. Thus, the step of "cleaving the support and optionally one or more protecting groups from the target peptide to obtain a support-cleaved target peptide" may typically comprise a step of precipitating the support-cleaved target peptide after treatment with the cleavage cocktail. Thus, in some embodiments the "step of cleaving the support and optionally one or more protecting groups from the target peptide to obtain a support-cleaved target peptide" comprises precipitating the support-cleaved target peptide, optionally followed by filtration. Such precipitation may typically be achieved by addition of the reaction mixture comprising the support-cleaved target peptide (once the cleavage has been completed) or a filtrate thereof , if a solid support has been removed, into an antisolvent (i.e. a solvent, in which the peptide that is to be precipitated is insoluble or poorly soluble) or by adding an antisolvent to the reaction mixture (once the cleavage has been completed) or filtrate (when a solid support has been removed) of the cleavage step. A person skilled in the art will be able to select an antisolvent for peptide precipitation. Non-limiting examples of such antisolvents comprise hydrophobic solvents such as ethers, in particular diisopropylether and the like. Precipitation of the support-cleaved target peptide may typically be followed by filtration.

In some embodiments, the method of the invention further comprises a step of cleaving the support and optionally one or more protecting groups from the target peptide, wherein said step comprises:
- treating the target peptide (the support-bound target peptide) with a cleavage composition (or cleavage cocktail) comprising an acid selected from the group consisting of TFA and hydrochloric acid, preferably TFA, and optionally a scavenger selected from the group consisting of a thiol and a silane, preferably EDT and TIS, and
- precipitating the support-cleaved target peptide, followed by filtration, to obtain a support-cleaved target peptide.

As laid out above, the method of the invention may further comprise "a step of purifying the support-cleaved target peptide to obtain a purified support-cleaved target peptide". It will be understood that said "purified support-cleaved target peptide will have the same sequence and may typically also have the same chemical structure as the support-cleaved target peptide, wherein these two peptides may for example differ in that the support-cleaved target peptide may typically be present in form of a salt of the acid used for the cleavage from the support (e.g. the TFA salt or the hydrochloride), while the purified support-cleaved target peptide may or may not be a salt and, if it is a salt, it may have one or more different kinds of counter ions, in particular counter ions that may be pharmaceutically acceptable or desirable. A person skilled in the art knows how to purify a support-cleaved target peptide of the invention to arrive at a purified support-cleaved target peptide. The term "purification" may herein refer to the complete or partial removal of one or more unwanted components form the peptide that is to be purified, in this case the support-cleaved target peptide. Such unwanted components may for example be peptides which may differ from the support-cleaved target peptide in that they lack one or more amino acid subunits in their sequence. Epimerization may also lead to unwanted components. Purification may also comprise the replacement of certain counter ions attached to the support-cleaved target peptide. Non-limiting examples of methods for the purification of the support-cleaved target peptide comprise reversed phase high performance liquid chromatography (RP-HPLC) and desalting. RP-HPLC is a standard technique for peptide purification and forms part of the common knowledge of those skilled in the art. "Desalting" is also known to those skilled in the art. RP-HPLC and/or desalting may typically be followed by a step of removing the solvent, in particular by lyophilization.

Thus, in some embodiments, the method of the invention further comprises a step of cleaving the support and optionally one or more protecting groups from the target peptide, wherein said step comprises:
- treating the target peptide (the support-bound target peptide) with a cleavage composition (or cleavage cocktail) comprising an acid selected from the group consisting of TFA and hydrochloric acid, preferably TFA, and optionally a scavenger selected from the group consisting of a thiol and a silane, preferably EDT and TIS, and
- precipitating the support-cleaved target peptide, followed by filtration to obtain a support-cleaved target peptide,
- purifying the support cleaved target peptide by means of RP-HPLC and/or desalting, and
- removing the solvent, preferably by means of lyophilization, to obtain a purified support-cleaved target peptide.

Once obtained, the support-cleaved target peptide or the purified target peptide may be further modified.

In some embodiments, the method of the invention further comprises a step of chemically modifying the target peptide, the support-cleaved target peptide or the purified support-cleaved target peptide to obtain a modified target peptide, which may optionally be followed by a step of purifying the modified target peptide to obtain a purified modified target peptide. As used herein, the term "chemically modifying" may refer to subjecting the species which is to be chemically modified to one or more chemical reactions. Non-limiting examples of such chemical modifications comprise conjugation as laid out above, e.g. with a carbohydrate residue or a fatty acid residue, and cyclization, e.g. via the formation of one or more disulfide bridges. Such disulfide bridges may for example be formed between the side chain sulfhydryl groups of cysteine subunits of the support-cleaved target peptide or the purified support cleaved target peptide. Another chemical modification may be the introduction or removal of one or more protecting groups. The modified target peptide may for example be purified by means of RP-HPLC, typically followed by lyophilization.

In some embodiments, in which the first component C-1 is covalently linked to a support, said support is insoluble in any of the solvents used or is soluble in the solvents used for the condensing, deprotecting, and cleavage steps. In some embodiments, in which the first component C-1 is covalently linked to a support, said support is insoluble in any of the solvents used. In some embodiments, in which the first component C-1 is covalently linked to a support, said support is insoluble in any of the solvents used soluble in the solvents used for the condensing, deprotecting, and cleavage steps.

A further aspect of the invention pertains to a composition comprising:
- a first component C-1 being selected from the group consisting of an amino acid and a peptide and comprising one free primary or secondary aliphatic amino group, wherein any other primary or secondary aliphatic amino groups and any carboxyl groups in C-1 are protected,
- a second component C-2 being selected from the group consisting of a chiral alpha-amino acid and a peptide and comprising one free carboxyl group, which is the alpha-carboxyl group of a chiral alpha-amino acid moiety comprising one hydrogen-substituent bonded to the alpha-carbon atom, wherein any primary or secondary aliphatic amino groups and any other carboxyl groups in C-2 are protected,
- a carbodiimide coupling agent,
- a first additive A-1 of formula A-I: 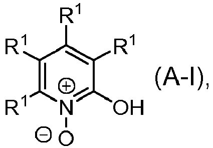 wherein in formula A-I,
   R¹ is at each occurrence independently selected from the group consisting of hydrogen, C₁-C₅-alkyl, and an electron-withdrawing substituent,
- a second additive A-2 selected from the group consisting of ethyl 2-cyano-2-(hydroxyimino)acetate (Oxyma), 1-hydroxybenzotriazole (HOBt), 1-hydroxy-7-azabenzotriazole (HOAt), 5-(hydroxyimino)-1,3-dimethylpyrimidine-2,4,6(1H,3H,5H)-trione (Oxyma-B), and ethyl 1-hydroxy-1 ,2,3-triazole-4-carboxylate (HOCt), or mixtures thereof, and
- one or more solvents,
wherein:
- the second component C-2 is present in a total molar amount of 1.0 to 5.0 equivalents,
- the carbodiimide coupling agent is present in a total molar amount of 1.0 to 6.0 equivalents,
- the first additive A-1 is present in a total molar amount of 1.0 to 6.0 equivalents, and
- the second additive A-2 is present in a total molar amount of 1.0 to 5.0 equivalents,
wherein all equivalents are given relative to the total molar amount of the first component C-1.

Based on what has been laid out so far, it will be understood that such a composition may be used in the method of the present invention, in particular in step (c) of the method of the invention. Such a composition may allow for the synthesis of a peptide by means of an amide bond forming condensation reaction between the free primary or secondary aliphatic amino group of the first component C-1 and the free alpha-carboxyl group of the second component C-2, thereby suppressing (i.e. preventing or reducing) epimerization and/or double insertion of the second component C-2. With regard to the composition of the present invention, the same definitions and embodiments may apply to the first component C-1, the second component C-2, the carbodiimide coupling agent, the first additive A-1, and the second additive A-2 as described for the method of the invention. Furthermore, for the one or more solvents of the composition the same definitions as for the first cycle solvent of step (c) of the method of the invention may apply.

In some embodiments of the composition of the invention, said composition comprises:
- a first component C-1 being selected from the group consisting of an amino acid and a peptide and comprising one free primary or secondary aliphatic amino group, which is the alpha-amino group of an alpha-amino acid moiety, wherein any other primary or secondary aliphatic amino groups and any carboxyl groups in C-1 are protected,
- a second component C-2 being a chiral alpha-amino acid selected from the group consisting of histidine, cysteine, and alpha-phenylglycine, wherein said chiral alpha-amino acid comprises a free alpha-carboxyl group and a protected alpha-amino group, wherein the amino protecting group is the Fmoc group,
- a carbodiimide coupling agent selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof,
- a first additive A-1, which is 2-HOPO,
- a second additive A-2 selected from the group consisting of ethyl 2-cyano-2-(hydroxyimino)acetate (Oxyma), 1-hydroxybenzotriazole (HOBt), 1-hydroxy-7-azabenzotriazole (HOAt), 5-(hydroxyimino)-1,3-dimethylpyrimidine-2,4,6(1H,3H,5H)-trione (Oxyma-B), and ethyl 1-hydroxy-1,2,3-triazole-4-carboxylate (HOCt), or mixtures thereof, and
- one or more solvents,
wherein:
- the second component C-2 is present in a total molar amount of 1.00-5.00 equivalents, 1.00-4.00 equivalents, 1.00-3.00 equivalents, 1.50-2.50 equivalents, or 2.00 equivalents,
- the carbodiimide coupling agent is present in a total molar amount of 1.00-6.00 equivalents, 2.00-5.00 equivalents, 3.00-5.00 equivalents, 3.50-4.50 equivalents, or 4.00 equivalents,
- the first additive A-1 is present in a total molar amount of 1.00-6.00 equivalents, 2.00-5.00 equivalents, 3.00-5.00 equivalents, 3.50-4.50 equivalents, or 4.00 equivalents, and
- the second additive A-2 is present in a total molar amount of 1.00-5.00 equivalents, 1.50-4.00 equivalents, 2.00-4.00 equivalents, 2.50-3.50 equivalents, or 3.00 equivalents,
wherein all equivalents are given relative to the total molar amount of the first component C-1.

The Examples, including the experiments conducted and the results achieved, are provided for illustrative purposes only and are not to be construed as limiting the scope of the claims.

### Examples

### General protocol for Fmoc-SPPS

Unless stated otherwise, Fmoc-SPPS was performed manually as stepwise SPPS. Standard, commercially available Fmoc-protected amino acids such as Fmoc-His(1-Trt)-OH, Fmoc-Cys(Trt)-OH, Fmoc-Ala-OH, Fmoc-Phe-OH, Fmoc-Pro-OH, Fmoc-N-Me-Ala-OH (N-Me-Ala = N-methylalanine), Fmoc-Phg-OH (Phg = alpha-phenylglycine), and Fmoc-Aib-OH (Aib = 2-aminoisobutyric acid = 2methylalanine) were used. The employed amino acids typically displayed an enantiomeric excess (ee) of the L-isomer equal to or larger than 99.8%. The syntheses were carried out using different solid supports (i.e. different solid phase materials), wherein said solid supports typically had the form of crosslinked polystyrene beads functionalized with various linkers.

A Fmoc-SPPS comprised one or more condensing cycles, wherein a typical condensing cycle comprised Fmoc-removal (i.e. a deprotecting step), followed by washing and a condensing step, again followed by washing.

The deprotecting step was typically performed by stirring (for example when using a reaction vessel equipped with a mechanical stirrer) or shaking/agitating (for example when using a reaction vessel which does not have a mechanical stirrer such as a syringe or an Erlenmeyer flask) the polymer beads to which the growing peptide chains were covalently linked in a solution of 20% (v/v) piperidine in N,N-dimethylformamide (DMF) at room temperature (i.e. (approximately) 20 °C), followed by filtration. 1-methylpyrrolidin-2-one (NMP) was used instead of DMF to enhance deprotection rates in some steps as indicated. The deprotection time typically varied between 15 minutes and 60 minutes. The completion of Fmoc-deprotection was optionally confirmed by means of UV/Vis-monitoring or by means of one or more of the well-known Kaiser-ninhydrin, TNBS, bromophenol blue or chloranil tests. Typically, two consecutive deprotection steps were carried out for each condensing cycle to assure complete Fmoc-deprotection of all growing peptide chains.

The deprotecting step was typically followed by washing using DMF and/or propan-2-ol (IPA) as solvents, unless indicated differently. Said washing comprised stirring or shaking/agitating the polymer beads to which the growing peptide chains were covalently linked in the respective solvent(s) at room temperature (i.e. (approximately) 20 °C), followed by filtration.

A condensing step was typically performed by stirring or shaking/agitating the polymer beads to which the growing peptide chains were covalently linked in a solution of the indicated coupling agent(s) and additive(s) in DMF at the indicated temperature and for the indicated time. The condensation reaction was optionally monitored by means of one or more of the well-known Kaiser-ninhydrin, TNBS, bromophenol blue or chloranil tests. If a condensing step did not go to completion (i.e. did not occur on all growing peptide chains comprising a free primary or secondary aliphatic amino group) as judged by reaction monitoring, the respective condensing step was repeated, wherein typically, washing (as stated above) was performed in between the two iterations of the condensing step. The repetition of a specific condensing step is herein indicated where applicable.

A condensing step was typically followed by washing using DMF and/or IPA as stated above, unless indicated differently.

In order to block any unreacted free amino groups at the growing peptide chains, one or more capping steps were typically performed after a condensing step. Capping was typically achieved by stirring or shaking/agitating the polymer beads to which the growing peptide chains were covalently linked at room temperature (i.e. (approximately) 20 °C) in a solution of acetic anhydride and an organic base such as pyridine or sym-collidine in DMF, followed by filtration. If performed, the one or more capping steps were typically followed by washing using DMF and/or IPA as stated above.

At the end of the synthesis, cleavage from the support and concomitant cleavage of (side-chain) protecting groups was typically performed by stirring or shaking/agitating the polymer beads to which the fully assembled target peptide chains were covalently linked at room temperature (i.e. (approximately) 20 °C) in a cleavage composition (also referred to as cleavage cocktail) consisting of trifluoroacetic acid (TFA)/H₂O/1,2-ethanedithiol (EDT) (90:5:5, v/v/v), TFA/triisoprpopyl silane (TIS)/H₂O/EDT (92.5:2.5:2.5:2.5, v/v/v/v) or TFA/H₂O (95:5), followed by filtration to remove the cleaved solid support. The support-cleaved target peptide was typically precipitated from the filtrate of the cleavage cocktail, followed by filtration. Precipitation was typically achieved by mixing of the filtrate of said cleavage step with an antisolvent such as diisopropyl ether. The precipitated crude comprising the support-cleaved target peptide (without further purification) was analyzed as described in a following section.

Typically, peptide syntheses started from commercially available solid supports, to which the first amino acid was already covalently linked, typically via its alpha-carboxyl group. The first condensing cycle typically started with a condensing step.

### Determination of molar amounts and equivalents

Unless indicated differently, molar amounts and equivalents are given relative to the (total) molar amount of the first amino acid covalently linked to the support, which was typically determined by multiplying the weight of initially utilized solid support carrying the first amino acid with the molar amount of available free (alpha-)amino groups per gram of the respective solid support carrying the first amino acid (loading factor). When using so-called amide resins (e.g. the Ramage amide resin, the Rink amide resin, or the TAL resin with TAL resin = 5-amino-10,11-dihydro-5H-dibenzo [a,d]cycloheptenyl-2-oxyacetyl-DL-Nle-4-methyl-benzhydrylamide resin, with Nle = norleucine), all molar amounts and equivalents are given relative to the (total) molar amount of the amino groups available at the support for amide bond formation, unless indicated differently. Said molar amount of the amino groups available at the support was typically determined by multiplying the weight amount of initially utilized solid support carrying the amino groups (determined by weighing the dry support carrying the free amino groups) with the molar amount of available free amino groups per gram of the respective solid support. Sometimes, for practical purposes, a larger batch of a support-bound peptide was produced, and aliquots thereof were used as a first component in several different condensation reactions. In such cases, the weight gain of the resin was taken into account for calculating the molar amount of the first component contained in each aliquot.

### Analysis of the target peptides with regard to epimerization and double insertion

Unless indicated differently, the precipitated crude comprising the support cleaved target peptide (without further purification) was subjected to separation and analysis by reversed phase analytical high-performance liquid chromatography (HPLC). Typically, a defined amount of about 1 mg of the precipitated crude comprising the support-cleaved target peptide was dissolved in a mixture of 0.5 mL eluent A (0.05% TFA in H2O/ACN 99:1 (v/v)) and 0.5 mL eluent B (0.05% TFA in H2O/ACN 99:1 (v/v); Eluent B: 0.05% TFA in ACN), for HPLC analysis using a Aquity UPLC CSH Phenyl-Hexyl column (1.7 um, 2.1×150 mm). The latter was performed on an Ultimate 3000RS Dionex HPLC system by Thermo Fisher equipped with a diode array detector.

Unless indicated differently, the areas underneath the peaks of the support-cleaved target peptide, the support-cleaved epimerization product, the support-cleaved double-insertion product, and the support-cleaved peptide which did not engage in the condensation reaction at all in a chromatogram (λ = 220 nm) obtained from the above-mentioned HPLC analysis were determined by means of integration. These compounds constituted the major components of the precipitated crude comprising the support-cleaved target peptide. Other peaks were not of relevance, unless indicated differently. In selected cases, additional relevant peaks were observed in the chromatogram. In such cases, all peaks in the chromatogram were integrated, which is indicated where applicable. In the following examples, area percentages (area %) are provided for selected species. Unless indicated differently, these area percentages have been determined by dividing the area underneath the peak of the respective species by the sum of areas underneath all integrated peaks, followed by multiplication with 100% (to arrive at a value in per cent, %).

### Example 1: Synthesis of solid phase-bound peptides used as the first component C-1

### Synthesis of H-Pro-Phe-Ala-2-CT resin

The solid-phase bound tripeptide H-Pro-Phe-Ala-2-chlorotrityl resin (H-Pro-Phe-Ala-2-CT resin) was synthesized by means of stepwise SPPS according to the general protocol for Fmoc-SPPS, starting from a H-Ala-2-chlorotrityl resin (H-Ala-2-CT resin, 100-200 mesh). Fmoc-Phe-OH was condensed using TBTU/DIPEA in DMF at ambient temperature (i.e. (approximately) 20 °C). Fmoc-Pro-OH was condensed using DIC/Oxyma in DMF at ambient temperature (i.e. (approximately) 20 °C). These first two condensing steps were each followed by washing, a capping step, and again by washing. In this fashion, the support-bound Fmoc-protected tripeptide Fmoc-Pro-Phe-Ala-2-CT resin was obtained and subsequently submitted to two iterations of a deprotecting step, followed washing to yield the support-bound tripeptide H-Pro-Phe-Ala-2-CT resin, which was washed, dried under reduced pressure, and stored at -20 °C.

### Synthesis of H-(N-Me-Ala)-Phe-Ala-2-CT resin

The solid phase-bound tripeptide H-(N-Me-Ala)-Phe-Ala-2-CT resin, was synthesized by means of stepwise SPPS according to the general protocol for Fmoc-SPPS, starting from a H-Ala-2-CT resin (100-200 mesh). Fmoc-Phe-OH was condensed using TBTU/DIPEA in DMF at ambient temperature (i.e. (approximately) 20 °C). Fmoc-(N-Me-Ala)-OH was condensed using DIC/Oxyma in DMF at ambient temperature (i.e. (approximately) 20 °C). These first two condensing steps were each followed by washing, a capping step, and again by washing. In this fashion, the support-bound Fmoc-protected tripeptide Fmoc-(N-Me-Ala)-Phe-Ala-2-CT resin was obtained and subsequently submitted to two iterations of a deprotecting step, followed by washing, to yield the support-bound tripeptide H-(N-Me-Ala)-Phe-Ala-2-CT, which was washed, dried under reduced pressure, and stored at -20 °C.

### Synthesis of H-Aib-Phe-Ala-TAL resin

The solid-phase bound tripeptide H-Aib-Phe-Ala-TAL resin (TAL resin = commercially available tricyclic amide linker resin, 5-amino-10,11-dihydro-5H-dibenzo[a,d]cycloheptenyl-2-oxyacetyl-DL-Nle-4-methyl-benzhydrylamide resin with Nle = norleucine) was synthesized by means of stepwise SPPS according to the general protocol for Fmoc-SPPS, starting from the commercially available Fmoc-protected TAL resin (200-400 mesh).The Fmoc groups were removed from the TAL resin 's amino groups in a deprotecting step. Fmoc-Ala-OH, Fmoc-Phe-OH, and Fmoc-Aib-OH were condensed using DIC/Oxyma in DMF at ambient temperature (i.e. (approximately) 20 °C). These condensing steps were each followed by washing, a capping step, and again by washing. In this fashion, the support-bound Fmoc-protected tripeptide Fmoc-Aib-Phe-Ala-TAL resin was obtained and subsequently submitted to two iterations of a deprotecting step, to yield the support-bound tripeptide H-Aib-Phe-Ala-TAL resin, which was washed, dried under reduced pressure, and stored at -20 °C.

### Example 2: Effect of the first and the second additive

A syringe, equipped with a polyethylene (PE)-filter was charged with a defined weight amount of the dried H-Pro-Phe-Ala-2-CT resin tripeptide (see Example 1), followed by washing with several syringe-volumes of DMF, so that the resin was swollen with said solvent. Fmoc-His(1-Trt)-OH (2.0 eq) and the indicated additive(s) were dissolved in DMF (9-10 mL DMF per gram of H-Pro-Phe-Ala-2-CT resin). The resulting solution was added into the syringe containing H-Pro-Phe-Ala-2-CT resin (0.11 mmol, 1.0 eq), followed directly by the addition of DIC in one portion. The syringe was then shaken in a ThermoMixer^{®} C (Eppendof) at a temperature of 50 °C for 60 min. This was followed by filtration through the syringe filter, washing using DMF and IPA, a cleavage step using TFA/H₂O/1,2-ethanedithiol (EDT) (90:5:5, v/v/v), and precipitation according to the general protocol for Fmoc-SPPS. The precipitated crude comprising the support-cleaved target peptide was analyzed by means of analytical HPLC as laid out above. The results of the analysis are given in Table 1.

**Table 1: Effect of the first and second additive.**

| entry | coupling agent and additives (equivalents, eq) | support-cleaved target peptide^{a} (area %)^{d} | D-His^{b} (area %)d | endo-His^{c} (area %)d |
|---|---|---|---|---|
| 1^{e,f} | DIC (3.0 eq), Oxyma (4.0 eq) | 95.69 | 4.20 | 0.08 |
| 2^{f} | DIC (3.0 eq), 2-HOPO (4.0 eq) | 97.76 | 0.10 | 2.10 |
| 3^{g} | DIC (3.0 eq), 2-HOPO (4.0 eq), Oxyma (1.0 eq) | 99.40 | 0.13 | 0.42 |
| 4^{f} | DIC (3.0 eq), 2-HOPO (4.0 eq), pyridine-HCl (1.0 eq) | 91.81 | 0.37 | 0.12 |
| 5^{f} | DIC (3.0 eq), 2-HOPO (4.0 eq), pyridine-HCl (0.75 eq) | 95.35 | 0.29 | 0.16 |
| 6^{f} | DIC (3.0 eq), 2-HOPO (4.0 eq), pyridine-HCl (0.50 eq) | 98.78 | 0.31 | 0.65 |

| | | | | |
|---|---|---|---|---|
| a: The term "support-cleaved target peptide" refers to Fmoc-His-Pro-Phe-Ala-OH (Fmoc protected peptide of SEQ ID NO: 1); b: The term "D-His" refers to the support-cleaved His-epimerization product, i.e. to Fmoc-D-His-Pro-Phe-Ala-OH (enantiomer of Fmoc protected peptide of SEQ ID NO: 1); c: The term "endo-His" refers to the support-cleaved His-double insertion product, i.e. to Fmoc-His-His-Pro-Phe-Ala-OH (Fmoc protected peptide of SEQ ID NO: 2); d: Area percentages have been determined by dividing the area underneath the peak of the respective species by the sum of the areas underneath the peaks of Fmoc-His-Pro-Phe-Ala-OH (Fmoc protected peptide of SEQ ID NO: 1), Fmoc-D-His-Pro-Phe-Ala-OH (enantiomer of (Fmoc protected peptide of SEQ ID NO: 1), Fmoc-His-His-Pro-Phe-Ala-OH (Fmoc protected peptide of SEQ ID NO: 2), and H-Pro-Phe-Ala-OH; e: 30 minutes reaction time; f: comparative; g: according to the invention. | | | | |

As can be concluded from Table 1, the combination of a carbodiimide coupling agent (e.g. DIC) with a first additive A-1 (e.g. 2-HOPO) and a second additive A-2 (e.g. Oxyma) according to the present invention (entry 3) gives an improved overall result as compared to the coupling mixtures of entries 1 and 2, lacking the first or the second additive, respectively. Low levels of (His-)epimerization and (His-)double insertion are achieved at the same time. The reduced reaction time of entry 1 is irrelevant in view of the already high content of the His-epimerization product D-His. Additionally, the comparison of entries 3 to 6 demonstrates that the second additive of the present invention may not simply be replaced by any acidic species such as pyridinium hydrochloride (pyridine-HCl). While pyridinium hydrochloride may be suitable to suppress (His-)double insertion, its use in amounts sufficient to achieve said effect is accompanied by a decrease of the overall purity of the support-cleaved target peptide.

### Example 3: optimization studies related to the stoichiometry of the carbodiimide coupling agent, the first, and the second additive

A syringe, equipped with a PE-filter was charged with a defined weight amount of the dried H-Pro-Phe-Ala-2-CT resin tripeptide (representing a first component C-1, see Example 1), followed by washing with several syringe-volumes of DMF, so that the resin was swollen with said solvent. Fmoc-His(1-Trt)-OH (2.0 eq; representing a second component C-2) and the respective additive(s) were dissolved in DMF (9-10 mL DMF per gram of H-Pro-Phe-Ala-2-CT). The resulting solution was added into the syringe containing H-Pro-Phe-Ala-2-CT resin (0.11 mmol, 1.0 eq), followed directly by the addition of DIC in one portion. The syringe was then shaken in a ThermoMixer^{®} C (Eppendof) at a temperature of 50 °C for 40 min. This was followed by filtration through the syringe filter, washing using DMF and IPA, a cleavage step using TFA/H₂O/1,2-ethanedithiol (EDT) (90:5:5, v/v/v), and precipitation according to the general protocol for Fmoc-SPPS. The precipitated crude comprising the support-cleaved target peptide was analyzed by means of analytical HPLC as laid out above. The results of the analysis given in Table 2

**Table 2: Optimization studies related to the stoichiometry of the carbodiimide coupling agent, the first, and the second additive.**

| entry | coupling agent and additives (equivalents, eq) | support-cleaved target peptide^{a} (area %)^{d} | D-His^{b} (area %)^{d} | endo-His^{c} (area %)^{d} |
|---|---|---|---|---|
| 1^{e} | DIC (2.0 eq), 2-HOPO (1.0 eq), Oxyma (1.0 eq) | 99.29 | 0.21 | 0.44 |
| 2^{e} | DIC (4.0 eq), 2-HOPO (1.0 eq), Oxyma (1.0 eq) | 99.43 | 0.24 | 0.25 |
| 3^{e} | DIC (2.0 eq), 2-HOPO (5.0 eq), Oxyma (1.0 eq) | 99.38 | 0.13 | 0.44 |
| 4^{e} | DIC (4.0 eq), 2-HOPO (5.0 eq), Oxyma (1.0 eq) | 99.53 | 0.11 | 0.31 |
| 5^{e} | DIC (2.0 eq), 2-HOPO (1.0 eq), Oxyma (3.0 eq) | 99.27 | 0.46 | 0.20 |
| 6^{e} | DIC (4.0 eq), 2-HOPO (1.0 eq), Oxyma (3.0 eq) | 99.29 | 0.51 | 0.14 |
| 7^{e} | DIC (4.0 eq), 2-HOPO (4.0 eq), Oxyma (3.0 eq) | 99.58 | 0.15 | 0.18 |

| | | | | |
|---|---|---|---|---|
| a: The term "support-cleaved target peptide" refers to Fmoc-His-Pro-Phe-Ala-OH (Fmoc protected peptide of SEQ ID NO: 1); b: The term "D-His" refers to the support-cleaved His-epimerization product, i.e. to Fmoc-D-His-Pro-Phe-Ala-OH (enantiomer of Fmoc protected peptide of SEQ ID NO: 1); c: The term "endo-His" refers to the support-cleaved His-double insertion product, i.e. to Fmoc-His-His-Pro-Phe-Ala-OH (Fmoc protected peptide of SEQ ID NO: 2); d: Area percentages have been determined by dividing the area underneath the peak of the respective species by the sum of the areas underneath the peaks of Fmoc-His-Pro-Phe-Ala-OH (Fmoc protected peptide of SEQ ID NO: 1), Fmoc-D-His-Pro-Phe-Ala-OH (enantiomer of Fmoc protected peptide of SEQ ID NO: 1), Fmoc-His-His-Pro-Phe-Ala-OH (Fmoc protected peptide of SEQ ID NO: 2), and H-Pro-Phe-Ala-OH; e: according to the invention. | | | | |

The comparison between entries 1 and 3, as well as 2 and 4 of Table 2 demonstrates that increasing the amount of the first additive A-1 (e.g. 2-HOPO) above 1.0 equivalent (and exemplarily to 5.0 equivalents) leads to an improved suppression of (His-)epimerization.

### Example 4: Effect of the reaction (condensing) temperature

A syringe, equipped with a PE-filter was charged with a defined weight amount of the dried H-Pro-Phe-Ala-2-CT resin tripeptide (representing a first component C-1, see Example 1), followed by washing with several syringe-volumes of DMF, so that the resin was swollen with said solvent. Fmoc-His(1-Trt)-OH (2.0 eq; representing a second component C-2), 2-HOPO (4.0 eq), and Oxyma (3.0 eq) were dissolved in DMF (10 mL DMF per gram of H-Pro-Phe-Ala-2-CT). The resulting solution was added into the syringe containing H-Pro-Phe-Ala-2-CT (0.11 mmol, 1.0 eq), followed directly by the addition of DIC (4.0 eq) in one portion. The syringe was then shaken in a ThermoMixer^{®} C (Eppendof) at the indicated temperature for the indicated time. This was followed by filtration through the syringe filter, washing using DMF and IPA, a cleavage step using TFA/H₂O/EDT (90:5:5, v/v/v), and precipitation according to the general protocol for Fmoc-SPPS. The precipitated crude comprising the support-cleaved target peptide was analyzed by means of analytical HPLC as laid out above. The results of the analysis are given in Table 3.

**Table 3: Investigating the effect of the reaction temperature.**

| entry | condensing time^{e} (min) | condensing temperature^{f} (°C) | support-cleaved target peptide^{a} (area %)^{d} | D-His^{b} (area %)^{d} | endo-His^{c} (area %)^{d} |
|---|---|---|---|---|---|
| 1^{g} | 60 | 50 | 99.57 | 0.16 | 0.20 |
| 2^{g} | 180 | 25 | 99.75 | 0.11 | 0.09 |

| | | | | | |
|---|---|---|---|---|---|
| a: The term "support-cleaved target peptide" refers to Fmoc-His-Pro-Phe-Ala-OH (Fmoc protected peptide of SEQ ID NO: 1); b: The term "D-His" refers to the support-cleaved His-epimerization product, i.e. to Fmoc-D-His-Pro-Phe-Ala-OH (enantiomer of Fmoc protected peptide of SEQ ID NO: 1); c: The term "endo-His" refers to the support-cleaved His-double insertion product, i.e. to Fmoc-His-His-Pro-Phe-Ala-OH (Fmoc protected peptide of SEQ ID NO: 2); d: Area percentages have been determined by dividing the area underneath the peak of the respective species by the sum of the areas underneath the peaks of Fmoc-His-Pro-Phe-Ala-OH (Fmoc protected peptide of SEQ ID NO: 1), Fmoc-D-His-Pro-Phe-Ala-OH, Fmoc-His-His-Pro-Phe-Ala-OH (Fmoc protected peptide of SEQ ID NO: 2), and H-Pro-Phe-Ala-OH (see general information and methods); e: The term "condensing time" refers to the time for which the above-mentioned components are reacted to condense Fmoc-His(1-Trt)-OH as first component C-1 and H-Pro-Phe-Ala-2-CT as second component C-2; f: The term "condensing temperature" refers to the temperature at which the above-mentioned components are reacted to condense Fmoc-His(1-Trt)-OH as first component C-1 and H-Pro-Phe-Ala-2-CT as second component C-2; g: according to the invention. | | | | | |

As can be concluded from Table 3, the method of the present invention gives good results at condensing temperatures of both 25 °C and 50 °C, as judged based on the overall purity of the support-cleaved target peptide and the levels of (His-)epimerization and (His-)double insertion impurities. In general, the experimental data suggests that the formation of side products is reduced at lower condensing temperatures. Prolonged condensing times may not always be desirable, especially when considering industrial applications.

### Example 5: Variation of the first and second additive

### Variation of the first additive A-1

A syringe, equipped with a PE-filter was charged with a defined weight amount of the dried H-Pro-Phe-Ala-2-CT resin tripeptide (representing a first component C-1, see Example 1), followed by washing with several syringe-volumes of DMF, so that the resin was swollen with said solvent. Fmoc-His(1-Trt)-OH (2.0 eq; representing a second component C-2), the indicated first additive (4.0 eq), and Oxyma (3.0 eq) were dissolved in DMF (10 mL DMF per gram of H-Pro-Phe-Ala-2-CT). The resulting solution was added into the syringe containing H-Pro-Phe-Ala-2-CT resin (0.11 mmol, 1.0 eq), followed directly by the addition of DIC (4.0 eq) in one portion. The syringe was then shaken in a ThermoMixer^{®} C (Eppendof) at 25 °C for 180 min, unless indicated differently. This was followed by filtration through the syringe filter, washing using DMF and IPA, a cleavage step using TFA/H₂O/EDT (90:5:5, v/v/v), and precipitation according to the general protocol for Fmoc-SPPS. The precipitated crude comprising the support-cleaved target peptide was analyzed by means of analytical HPLC as laid out above. The results of the analysis are compiled in Table 4a, alongside the employed first additives.

**Table 4a: Variation of the first additive.**

| entry | first additive | support-cleaved target peptide^{a} (area %)^{d} | D-His^{b} (area %)^{d} | endo-His^{c} (area %)^{d} |
|---|---|---|---|---|
| 1ⁱ | 2-HOPO | 99.80 | 0.09 | 0.08 |
| 2^{k} | 3-HOPO^{e} | 99.27 | 0.62 | 0.07 |
| 3^{k} | 4-HOPO^{f} | 99.32 | 0.60 | 0.05 |
| 4^{k} | none | 98.29 | 1.64 | 0.04 |
| 5^{k} | HOBt×H₂O | 97.85 | 2.10 | 0.02 |
| 6ⁱ | 4-Br-2-HOPO^{g,h} | 99.62 | 0.20 | 0.14 |
| 7ⁱ | 4-Br-2-HOPO^{g} | 99.81 | 0.13 | 0.06 |

| | | | | |
|---|---|---|---|---|
| a: The term "support-cleaved target peptide" refers to Fmoc-His-Pro-Phe-Ala-OH (Fmoc protected peptide of SEQ ID NO: 1); b: The term "D-His" refers to the support-cleaved His-epimerization product, i.e. to Fmoc-D-His-Pro-Phe-Ala-OH (enantiomer of Fmoc protected peptide of SEQ ID NO: 1); c: The term "endo-His" refers to the support-cleaved His-double insertion product, i.e. to Fmoc-His-His-Pro-Phe-Ala-OH (Fmoc protected peptide of SEQ ID NO: 2); d: Area percentages have been determined by dividing the area underneath the peak of the respective species by the sum of the areas underneath the peaks of Fmoc-His-Pro-Phe-Ala-OH, (Fmoc protected peptide of SEQ ID NO: 1) Fmoc-D-His-Pro-Phe-Ala-OH (enantiomer of Fmoc protected peptide of SEQ ID NO: 1), Fmoc-His-His-Pro-Phe-Ala-OH (Fmoc protected peptide of SEQ ID NO: 2), and H-Pro-Phe-Ala-OH; e: 3-HOPO = 3-hydroxypyridine N-oxide; f: 4-HOPO = 4-hydroxypyridine N-oxide; g: 4-Br-2-HOPO = 4-bromo-2-hydroxypyridine N-oxide; h: the condensing time was 60 min and the condensing temperature was 50 °C; i: according to the invention; k: comparative. | | | | |

The results summarized in Table 4a demonstrate that the 2-HOPO is best suited to reduce (His-)epimerization, and indeed affords the best overall result, reflected in a high overall purity of the support-cleaved target peptide and low levels of (His-)epimerization and (His-)double insertion. When replacing the first additive A-1 (e.g. 2-HOPO) by another second additive A-2 (e.g. HOBt), the level of (His-)epimerization increases (compare entries 1 and 5). The use of 4-Br-2-HOPO as first additive gave results comparable to the use of 2-HOPO at 50 °C and at 25 °C. As compared to 2-HOPO, 4-Br-2-HOPO seems to be slightly more effective in suppressing (His-) double-insertion, but slightly less effective in suppressing (His-)epimerization.

### Variation of the second additive A-2

A syringe, equipped with a PE-filter was charged with a defined weight amount of the dried H-Pro-Phe-Ala-2-CT resin tripeptide (representing a first component C-1, see Example 1), followed by washing with several syringe-volumes of DMF, so that the resin was swollen with said solvent. Fmoc-His(1-Trt)-OH (2.0 eq; representing a second component C-2), 2-HOPO (4.0 eq), and the indicated second additive in the indicated amount were dissolved in DMF (10 mL DMF per gram of H-Pro-Phe-Ala-2-CT resin). The resulting solution was added into the syringe containing H-Pro-Phe-Ala-2-CT resin (0.11 mmol, 1.0 eq), followed directly by the addition of DIC (4.0 eq) in one portion. The syringe was then shaken in a ThermoMixer^{®} C (Eppendof) at 25 °C for 180 min. This was followed by filtration through the syringe filter, washing using DMF and IPA, a cleavage step using TFA/H₂O/EDT (90:5:5, v/v/v), and precipitation according to the general protocol for Fmoc-SPPS. The precipitated crude comprising the target peptide was analyzed by means of analytical HPLC as laid out above. The results of the analysis are given in Table 4b.

**Table 4b: Variation of the second additive.**

| entry | second additive and equivalents (eq) | support-cleaved target peptide^{a} (area %)^{d} | D-His^{b} (area %)^{d} | endo-His^{c} (area %)^{d} |
|---|---|---|---|---|
| 1^{h} | Oxyma (3.0 eq) | 99.78 | 0.10 | 0.09 |
| 2^{g} | Oxyma (0.12 eq) | 99.59 | 0.08 | 0.33 |
| 3^{h} | HOAt (3.0 eq) | 99.70 | 0.14 | 0.11 |
| 4^{g} | HOAt (0.12 eq) | 99.51 | 0.10 | 0.36 |
| 5^{h} | HOBt×H₂O (3.0 eq) | 99.78 | 0.11 | 0.08 |
| 6^{g} | HOBt×H₂O (0.12 eq) | 99.56 | 0.08 | 0.36 |
| 7^{h} | Oxyma-B (3.0 eq) | 99.77 | 0.08 | 0.10 |
| 8^{g} | Oxyma-B (0.12 eq) | 99.56 | 0.08 | 0.33 |
| 9^{h} | HOCt (3.0 eq) | 99.34 | 0.15 | 0.05 |
| 10^{g} | HOCt (0.12 eq) | 99.61 | 0.09 | 0.27 |
| 11^{g} | HOSu^{e} (3.0 eq) | 99.36 | 0.08 | 0.53 |
| 12^{g} | HOSu^{e} (0.12 eq) | 99.26 | 0.09 | 0.62 |
| 13^{g} | HONB^{f} (3.0 eq) | 99.30 | 0.07 | 0.60 |
| 14^{g} | HONB^{f} (0.12 eq) | 99.28 | 0.08 | 0.61 |
| 15^{g} | no second additive used | 99.29 | 0.07 | 0.61 |

| | | | | |
|---|---|---|---|---|
| a: The term "support-cleaved target peptide" refers to Fmoc-His-Pro-Phe-Ala-OH (Fmoc protected peptide of SEQ ID NO: 1); b: The term "D-His" refers to the support-cleaved His-epimerization product, i.e. to Fmoc-D-His-Pro-Phe-Ala-OH (enantiomer of Fmoc protected peptide of SEQ ID NO: 1); c: The term "endo-His" refers to the support-cleaved His-double insertion product, i.e. to Fmoc-His-His-Pro-Phe-Ala-OH (Fmoc protected peptide of SEQ ID NO: 2); d: Area percentages have been determined by dividing the area underneath the peak of the respective species by the sum of the areas underneath the peaks of Fmoc-His-Pro-Phe-Ala-OH (Fmoc protected peptide of SEQ ID NO: 1), Fmoc-D-His-Pro-Phe-Ala-OH (enantiomer of Fmoc protected peptide of SEQ ID NO: 1), Fmoc-His-His-Pro-Phe-Ala-OH (Fmoc protected peptide of SEQ ID NO: 2), and H-Pro-Phe-Ala-OH; e: HOSu = N-hydroxysuccinimide; f: HONB = N-hydroxy-5-norbornene-2,3-dicarboximide; g: comparative; h: according to the invention. | | | | |

The conditions in examples 1, 3, 5, 7, and 9 resulted in a high overall purity of the support-cleaved target peptide as well as low levels of (His-)epimerization and (His-)double insertion impurities. By comparison, examples 2, 4, 6, 8, and 10, which differ from the inventive experiments only in that 0.12 equivalents instead of 3.0 equivalents of the respective second additive A-2 have been used, resulted in more (His-)double insertion. Examples 11 and 13 differ from the examples according to the present invention only in that the second additive (HOSu or HONB) is a known coupling additive having a N-OH functional group, but does not fulfill the structural requirements of a second additive A-2 of the present invention. It is evident that these additives are less efficient in suppressing (His-)double insertion. When not using a second additive at all (see example 15), the rate of (His-)double insertion is higher than in the experiments according to the present invention.

### Example 6: Synthesis of Fmoc-Cys-(N-Me-Ala)-Phe-Ala-OH (Fmoc protected peptide of SEQ ID NO: 3) under varying conditions

A syringe, equipped with a PE-filter was charged with a defined weight amount of the dried H-(N-Me-Ala)-Phe-Ala-2-CT resin tripeptide (representing a first component C-1, see Example 1), followed by washing with several syringe-volumes of DMF, so that the resin was swollen with said solvent. Fmoc-Cys(Trt)-OH (2.0 eq; representing a second component C-2), 2-HOPO (4.0 eq), and the indicated second additive in the indicated molar amount were dissolved in DMF (10 mL DMF per gram of H-(N-Me-Ala)-Phe-Ala-2-CT resin). The resulting solution was added into the syringe containing H-(N-Me-Ala)-Phe-Ala-2-CT resin (0.11 mmol, 1.0 eq), followed directly by the addition of DIC (4.0 eq) in one portion. The syringe was then shaken in a ThermoMixer^{®} C (Eppendof) at 35 °C for 60 min. This was followed by filtration through the syringe filter, washing using DMF and IPA, a cleavage step using TFAH₂O/EDT (92.5:2.5:2.5:2.5, v/v/v/v), and precipitation according to the general protocol for Fmoc-SPPS. The precipitated crude comprising the target peptide was analyzed by means of analytical HPLC as laid out above. The results of the analysis are given in Table 5.

**Table 5: Synthesis of Fmoc-Cys-(N-Me-Ala)-Phe-Ala-OH under varying conditions.**

| entry | coupling agent and additives and equivalents (eq) | support-cleaved target peptide^{a} (area %)^{d} | D-Cys^{b} (area %)^{d} | endo-Cys^{c} (area %)^{d} |
|---|---|---|---|---|
| 1^{f} | DIC (4.0 eq), 2-HOPO (4.0 eq), Oxyma (3.0 eq) | > 99^{g} | 0.09 | < 0.20^{g} |
| 2^{e} | DIC (4.0 eq), 2-HOPO (4.0 eq) | > 99^{g} | 0.12 | ≈ 0.35^{g} |
| 3^{e} | DIC (3.9 eq), Oxyma (3.1 eq) | > 98^{g} | 1.00 | < 0.20^{g} |

| | | | | |
|---|---|---|---|---|
| a: The term support-cleaved target peptide refers to Fmoc-Cys-(N-Me-Ala)-Phe-Ala-OH (Fmoc protected peptide of SEQ ID NO: 3); b: The term "D-Cys" refers to the support-cleaved Cys-epimerization product, i.e. to Fmoc-D-Cys-(N-Me-Ala)-Phe-Ala-OH (enantiomer of Fmoc protected peptide of SEQ ID NO: 3); c: The term "endo-Cys" refers to the support-cleaved Cys-double insertion product, i.e. to Fmoc-Cys-Cys-(N-Me-Ala)-Phe-Ala-OH (Fmoc protected peptide of SEQ ID NO: 4); d: Area percentages have been determined by dividing the area underneath the peak of the respective species by the sum of the areas underneath Fmoc-Cys-(N-Me-Ala)-Phe-Ala-OH (Fmoc protected peptide of SEQ ID NO: 3), Fmoc-D-Cys-(N-Me-Ala)-Phe-Ala-OH (enantiomer of Fmoc protected peptide of SEQ ID NO: 3), Fmoc-Cys-Cys-(N-Me-Ala)-Phe-Ala-OH (Fmoc protected peptide of SEQ ID NO: 4), and H-(N-Me-Ala)-Phe-Ala-OH; e: comparative; f: according to the invention; g: Due to minor overlap of the peaks of Fmoc-Cys-Cys-(N-Me-Ala)-Phe-Ala-OH (Fmoc protected peptide of SEQ ID NO: 4) ("endo-Cys") and Fmoc-Cys-(N-Me-Ala)-Phe-Ala-OH (Fmoc protected peptide of SEQ ID NO: 3), it was refrained from providing exact values for the corresponding area percentages (area %). The provided values suffice to point out the observed trend. | | | | |

As can be concluded from Table 5, the combination of a carbodiimide coupling agent (e.g. DIC) with the first additive A-1 (e.g. 2-HOPO) and the second additive A-2 (e.g. Oxyma) according to the present invention (entry 1) affords an improved result as compared to the coupling mixtures of entries 2 and 3, lacking the second or the first additive, respectively. Said improved result is reflected in low levels of (Cys-epimerization and (Cys-)double insertion.

### Example 7: Synthesis of Fmoc-Phg-(N-Me-Ala)-Phe-Ala-OH (Fmoc protected peptide of SEQ ID NO: 5) under varying conditions

A syringe, equipped with a PE-filter was charged with a defined weight amount of the dried H-(N-Me-Ala)-Phe-Ala-2-CT resin tripeptide (representing a first component C-1, see Example 1), followed by washing with several syringe-volumes of DMF, so that the resin was swollen with said solvent. Fmoc-Phg-OH (2.0 eq; representing a second component C-2), and the indicated additive(s) in the indicated amount(s) were dissolved in DMF (10 mL DMF per gram of H-(N-Me-Ala)-Phe-Ala-2-CT). The resulting solution was added into the syringe containing H-(N-Me-Ala)-Phe-Ala-2-CT (0.11 mmol, 1.0 eq), followed directly by the addition of the indicated amount of DIC in one portion. The syringe was then shaken in a ThermoMixer^{®} C (Eppendof) at 25 °C for 90 min. This was followed by filtration through the syringe filter, washing using DMF and IPA, and a cleavage step using TFA/H₂O (95:5, v/v). The filtrate of the cleavage step was diluted (1:19) with a 1:1 mixture of eluent A and eluent B of the HPLC analysis and analyzed by means of analytical HPLC as laid out above. The results of the analysis are compiled in Table 6.

**Table 6: Synthesis of Fmoc-Phg-(N-Me-Ala)-Phe-Ala-OH under varying conditions.**

| entry | coupling agent and additives and equivalents (eq) | support-cleaved target peptide^{a} (area %)^{c} | D-Phg^{b} (area %)^{c} |
|---|---|---|---|
| 1^{e} | DIC (4.0 eq), 2-HOPO (4.0 eq), Oxyma (3.0 eq) | 72.8 | 8.8 |
| 2^{d} | DIC (3.9 eq), Oxyma (3.1 eq) | 57.5 | 24.0 |

| | | | |
|---|---|---|---|
| A: The term support-cleaved target peptide refers to Fmoc-Phg-(N-Me-Ala)-Phe-Ala-OH (Fmoc protected peptide of SEQ ID NO: 5); b: The term "D-Phg" refers to the support-cleaved Phg-epimerization product, i.e. to Fmoc-D-Phg-(N-Me-Ala)-Phe-Ala-OH (enantiomer of Fmoc protected peptide of SEQ ID NO: 5); c: Area percentages have been determined by dividing the area underneath the peak of the respective species by the sum of the areas underneath all peaks in the chromatogram. In this particular case, the support-cleaved epimerization-product (D-Phg) was formed in significantly higher amounts than the support-cleaved double-insertion product (endo-Phg, Fmoc-Phg-Phg-(N-Me-Ala)-Phe-Ala-OH (Fmoc protected peptide of SEQ ID NO: 6), < 0.09 area % for entry 1 and 2); d: comparative; e: according to the invention. | | | |

As laid out above, the amino acid alpha-phenylglycine (Phg) is known to be particularly prone to epimerization. However, as can be concluded from entry 1, the level of Phg-epimerization is significantly reduced when applying the method of the present invention. In contrast, when for example not using the first additive A-1 (e.g. 2-HOPO) of the invention, the level of Phg-epimerization increases (entry 2).

### Example 8: Synthesis of Fmoc-His-Aib-Phe-Ala-NH₂ (Fmoc protected peptide of SEQ ID NO: 7) under varying conditions

A syringe, equipped with a PE-filter was charged with a defined weight amount of the dried H-Aib-Phe-Ala-TAL resin tripeptide (representing a first component C-1, see Example 1), followed by washing with several syringe-volumes of DMF, so that the resin was swollen with said solvent. Fmoc-His(1-Trt)-OH (2.0 eq; representing a second component C-2) and the indicated first and/or second additive in the indicated molar amount(s) were dissolved in DMF (10 mL DMF per gram of H-Aib-Phe-Ala-TAL resin). The resulting solution was added into the syringe containing H-Aib-Phe-Ala-TAL resin (0.11 mmol, 1.0 eq), followed directly by the addition of DIC (4.0 eq) in one portion. The syringe was then shaken in a ThermoMixer^{®} C (Eppendof) at 25 °C for 240 min. This was followed by filtration through the syringe filter, washing using DMF and IPA, a cleavage step using TFA/H₂O/EDT (90:5:5, v/v/v), and precipitation according to the general protocol for Fmoc-SPPS. The precipitated crude comprising the target peptide was analyzed by means of analytical HPLC as laid out above. The results of the analysis are compiled in Table 7.

**Table 7: Synthesis of Fmoc-His-Aib-Phe-Ala-NH₂ under varying conditions.**

| entry | additives and equivalents (eq) | support-cleaved target peptide^{a} (area %)d | D-His^{b} (area %)^{d} | endo-His^{c} (area %)^{d} |
|---|---|---|---|---|
| 1^{e} | Oxyma (3.0 eq) | 96.55 | 3.40 | 0.02 |
| 2^{e} | 2-HOPO (4.0 eq) | 97.53 | 0.04 | 2.40 |
| 3^{f} | 2-HOPO (4.0 eq), Oxyma (3.0 eq) | 96.98 | 0.22 | 0.10 |
| 4^{e} | 2-HOPO (4.0 eq), Oxyma (0.12 eq) | 99.42 | 0.05 | 0.50 |

| | | | | |
|---|---|---|---|---|
| a: The term "support-cleaved target peptide" refers to Fmoc-Aib-Pro-Phe-Ala-NH₂ (Fmoc protected peptide of SEQ ID NO: 7); b: The term "D-His" refers to the support-cleaved His-epimerization product, i.e. to Fmoc-D-His-Aib-Phe-Ala-NH₂; c: The term "endo-His" refers to the support-cleaved His-double insertion product, i.e. to Fmoc-His-His-Aib-Phe-Ala-NH₂ (Fmoc protected peptide of SEQ ID NO: 8); d: Area percentages have been determined by dividing the area underneath the peak of the respective species by the sum of the areas underneath the peaks of Fmoc-His-Aib-Phe-Ala-NH₂, Fmoc-D-His-Aib-Phe-Ala-NH₂ Fmoc-His-His-Aib-Phe-Ala-NH₂ (enantiomer of Fmoc protected peptide of SEQ ID NO: 7), Fmoc-His-His-Aib-Phe-Ala-NH₂ (Fmoc protected peptide of SEQ ID NO: 8), and H-Aib-Phe-Ala-NH₂; e: comparative; f: according to the invention. | | | | |

As can be concluded from Table 7, the combination of a carbodiimide coupling agent (e.g. DIC, 4.0 eq) with the first additive A-1 (e.g. 2-HOPO, 4.0 eq) and the second additive A-2 (e.g. Oxyma, 3.0 eq) affords an improved result as compared to the condensing mixtures of entries 1 and 2, lacking the first or the second additive, respectively. Said improved result is reflected in low levels of (His-)epimerization and (His-)double insertion.

## Claims

1. A method for the synthesis of a target peptide comprising a first cycle peptide P-1, wherein the method comprises the following steps:
(a) providing a first component C-1 being selected from the group consisting of an amino acid and a peptide and comprising one free primary or secondary aliphatic amino group, wherein any other primary or secondary aliphatic amino groups and any carboxyl groups in C-1 are protected,
(b) providing a second component C-2 being selected from the group consisting of a chiral alpha-amino acid and a peptide and comprising one free carboxyl group, which is the alpha-carboxyl group of a chiral alpha-amino acid moiety comprising one hydrogen-substituent bonded to the alpha-carbon atom, wherein any primary or secondary aliphatic amino groups and any other carboxyl groups in C-2 are protected, and
(c) condensing the first component C-1 of step (a) and the second component C-2 of step (b) in the presence of:
- a carbodiimide coupling agent,
- a first additive A-1 of formula A-I: 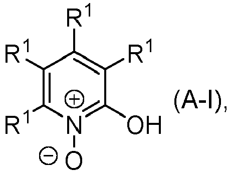 wherein in formula A-I,
R¹ is at each occurrence independently selected from the group consisting of hydrogen, C₁-C₅-alkyl, and an electron-withdrawing substituent,
- a second additive A-2 selected from the group consisting of ethyl 2-cyano-2-(hydroxyimino)acetate (Oxyma), 1-hydroxybenzotriazole (HOBt), 1-hydroxy-7-azabenzotriazole (HOAt), 5-(hydroxyimino)-1,3-dimethylpyrimidine-2,4,6(1H,3H,5H)-trione (Oxyma-B), and ethyl 1-hydroxy-1,2,3-triazole-4-carboxylate (HOCt), or mixtures thereof, and
- a first cycle solvent,
so as to form an amide bond between the free primary or secondary aliphatic amino group of the first component C-1 and the free alpha-carboxyl group of the second component C-2, to obtain a first cycle peptide P-1,
wherein in step (c), the second additive A-2 is used in a total molar amount of 1.0 to 5.0 equivalents relative to the total molar amount of the first component C-1.

2. The method according to claim 1, wherein the first additive A-1 is 2-hydroxypyridine N-oxide (2-HOPO).

3. The method according to any one of the preceding claims, wherein the carbodiimide coupling agent is selected from the group consisting of N,N'-diisopropylcarbodiimide (DIC), N,N'-dicyclohexylcarbodiimide (DCC), and N-(3-dimethylaminopropyl)-N'-ethylcarbodiimide (EDC), or mixtures thereof.

4. The method according to any one of the preceding claims, wherein the free primary or secondary aliphatic amino group of the first component C-1 is an alpha-amino group of an alpha-amino acid moiety.

5. The method according to any one of the preceding claims, wherein the second component C-2 is a chiral alpha-amino acid, preferably wherein the second component C-2 is selected from the group consisting of histidine, cysteine, an alpha-arylglycine, and an alpha-heteroarylglycine.

6. The method according to any one of claims 1 to 4, wherein the second component C-2 is a peptide selected from the group consisting of a dipeptide and a tripeptide.

7. The method according to any one of the preceding claims, wherein the second component C-2 comprises a primary or secondary aliphatic amino group which is protected by a fluorenylmethyloxycarbonyl-type protecting group.

8. The method according to any one of claims 1 to 5 and 7, wherein the second component C-2 is Fmoc-His(1-Trt)-OH.

9. The method according to any one of claims 1 to 5 and 7, wherein the second component C-2 is Fmoc-Cys(Trt)-OH.

10. The method according to any one of the preceding claims, wherein in step (c):
- the second component C-2 is used in a total molar amount of 1.0 to 5.0 equivalents,
- the carbodiimide coupling agent is used in a total molar amount of 1.0 to 6.0 equivalents,
- the first additive A-1 is used in a total molar amount of 1.0 to 6.0 equivalents, and
- the second additive A-2 is used in a total molar amount of 1.0 to 5.0 equivalents,
wherein all equivalents are given relative to the total molar amount of the first component C-1.

11. The method according to any one of the preceding claims, wherein the first component C-1 is covalently linked to a support.

12. The method according to any one of the preceding claims, wherein the method further comprises the following step (d):
(d) deprotecting one protected primary or secondary aliphatic amino group in the first cycle peptide P-1 obtained in step (c), to obtain a first cycle peptide P-1' comprising one free primary or secondary aliphatic amino group.

13. The method according to claim 12, wherein the target peptide comprises a (n+1)-th cycle peptide P-(n+1) derived from the first cycle peptide P-1' obtained in step (d) by performing n iterations of further condensing cycles, wherein n is an integer from 1 to 100, and wherein each further condensing cycle comprises the following steps (e) to (g):
(e) providing a further component C-(n+2) selected from the group consisting of an amino acid and a peptide comprising one free carboxyl group or one activated carboxyl group, and comprising at least one protected primary or secondary aliphatic amino group, wherein any primary or secondary aliphatic amino groups and any other carboxyl groups in C-(n+2) are protected,
(f) condensing the first cycle peptide P-1' obtained in step (d) or, if one or more iterations of further condensing cycles have already been performed, the (n+1)-th cycle peptide P-(n+1)' obtained in step (g) of the preceding condensing cycle, and the further component C-(n+2) of step (e) under suitable conditions, to form an amide bond between the free primary or secondary aliphatic amino group of the first cycle peptide P-1' of step (d) or of the (n+1)-th cycle peptide P-(n+1)' of step (g) of the preceding condensing cycle and the free or activated carboxyl group of the further component C-(n+2) of step (e), to obtain an (n+1)-th cycle peptide P-(n+1), and
(g) deprotecting one protected primary or secondary aliphatic amino group in the (n+1)-th cycle peptide P-(n+1) obtained in step (f), to obtain a (n+1)-th cycle peptide P-(n+1)' comprising one free primary or secondary aliphatic amino group,
wherein, the further component C-(n+2) is selected independently for each iteration n of further condensing cycles, and
wherein in the final condensing cycle, step (g) may be omitted.

14. The method according to claim 13, wherein
- the one primary or secondary aliphatic amino group which is deprotected in step (d) is an alpha-amino group of an alpha-amino acid moiety,
- the one primary or secondary aliphatic amino group which is deprotected in step (g) of each further condensing cycle is an alpha-amino group of an alpha-amino acid moiety, and
- the one free or the one activated carboxyl group of each further component C-(n+2) is an alpha-carboxyl group of an alpha-amino acid moiety.

15. The method according to any one of claims 13 and 14, wherein the amino protecting group which is removed when deprotecting one protected primary or secondary aliphatic amino group in step (d) and step (g) is a fluorenylmethyloxycarbonyl-type protecting group.

16. The method according to any one of claims 11 to 15, wherein the method further comprises a step of cleaving the support and optionally one or more protecting groups from the target peptide to obtain a support-cleaved target peptide, which may optionally be followed by a step of purifying the support-cleaved target peptide to obtain a purified support-cleaved target peptide.

17. The method according to claim 16, wherein the method further comprises a step of chemically modifying the target peptide, the support-cleaved target peptide or the purified support-cleaved target peptide to obtain a modified target peptide, which may optionally be followed by a step of purifying the modified target peptide to obtain a purified modified target peptide.

18. The method according to any of claims 11 to 17, wherein the support is insoluble in any of the solvents used or wherein the support is soluble in the solvents used for the condensing, deprotecting, and cleavage steps.

19. A composition comprising:
- a first component C-1 being selected from the group consisting of an amino acid and a peptide and comprising one free primary or secondary aliphatic amino group, wherein any other primary or secondary aliphatic amino groups and any carboxyl groups in C-1 are protected,
- a second component C-2 being selected from the group consisting of a chiral alpha-amino acid and a peptide and comprising one free carboxyl group, which is the alpha-carboxyl group of a chiral alpha-amino acid moiety comprising one hydrogen-substituent bonded to the alpha-carbon atom, wherein any primary or secondary aliphatic amino groups and any other carboxyl groups in C-2 are protected,
- a carbodiimide coupling agent,
- a first additive A-1 of formula A-I: 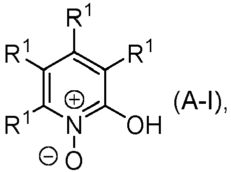 wherein in formula A-I,
R¹ is at each occurrence independently selected from the group consisting of hydrogen, C₁-C₅-alkyl, and an electron-withdrawing substituent,
- a second additive A-2 selected from the group consisting of ethyl 2-cyano-2-(hydroxyimino)acetate (Oxyma), 1-hydroxybenzotriazole (HOBt), 1-hydroxy-7-azabenzotriazole (HOAt), 5-(hydroxyimino)-1,3-dimethylpyrimidine-2,4,6(1H,3H,5H)-trione (Oxyma-B), and ethyl 1-hydroxy-1,2,3-triazole-4-carboxylate (HOCt), or mixtures thereof, and
- one or more solvents,
wherein:
- the second component C-2 is present in a total molar amount of 1.0 to 5.0 equivalents,
- the carbodiimide coupling agent is present in a total molar amount of 1.0 to 6.0 equivalents,
- the first additive A-1 is present in a total molar amount of 1.0 to 6.0 equivalents, and
- the second additive A-2 is present in a total molar amount of 1.0 to 5.0 equivalents,
wherein all equivalents are given relative to the total molar amount of the first component C-1.

## Patentansprüche

1. Verfahren zur Synthese eines Zielpeptids, umfassend ein Peptid des ersten Zyklus P-1, wobei das Verfahren die folgenden Schritte umfasst:
(a) Bereitstellen einer ersten Komponente C-1, die ausgewählt ist aus der Gruppe bestehend aus einer Aminosäure und einem Peptid, und eine freie primäre oder sekundäre aliphatische Aminogruppe umfasst, wobei jegliche anderen primären oder sekundären aliphatischen Aminogruppen und jegliche Carboxylgruppen in C-1 geschützt sind,
(b) Bereitstellen einer zweiten Komponente C-2, die ausgewählt ist aus der Gruppe bestehend aus einer chiralen Alpha-Aminosäure und einem Peptid, und die eine freie Carboxylgruppe umfasst, die die Alpha-Carboxylgruppe einer chiralen Alpha-Aminosäure-Einheit ist, die einen an das Alpha-Kohlenstoffatom gebundenen Wasserstoff-Substituenten umfasst, wobei jegliche primären oder sekundären aliphatischen Aminogruppen und jegliche anderen Carboxylgruppen in C-2 geschützt sind, und
(c) Kondensieren der ersten Komponente C-1 aus Schritt (a) und der zweiten Komponente C-2 aus Schritt (b) in Gegenwart von:
- einem Carbodiimid-Kupplungsmittel,
- einem ersten Additiv A-1 der Formel A-I: 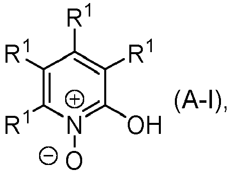 wobei in Formel A-I,
R¹ bei jedem Auftreten unabhängig ausgewählt ist aus der Gruppe bestehend aus Wasserstoff, C₁-C₅-Alkyl und einem elektronenziehenden Substituenten,
- einem zweiten Additiv A-2 ausgewählt aus der Gruppe bestehend aus Ethyl-2-cyano-2-(hydroxyimino)acetat (Oxyma), 1-Hydroxybenzotriazol (HOBt), 1-Hydroxy-7-azabenzotriazol (HOAt), 5(Hydroxyimino)-1,3-dimethylpyrimidin-2,4,6(1H,3H,5H)-trion (Oxyma-B) und Ethyl-1-hydroxy-1,2,3-triazol-4-carboxylat (HOCt) oder Mischungen davon, und
- einem Lösungsmittel des ersten Zyklus,
so dass eine Amidbindung zwischen der freien primären oder sekundären aliphatischen Aminogruppe der ersten Komponente C-1 und der freien alpha-Carboxylgruppe der zweiten Komponente C-2 gebildet wird, um ein Peptid P-1 des ersten Zyklus zu erhalten,
wobei in Schritt (c) das zweite Additiv A-2 in einer molaren Gesamtmenge von 1,0 bis 5,0 Äquivalenten bezogen auf die molare Gesamtmenge der ersten Komponente C-1 verwendet wird.

2. Verfahren gemäß Anspruch 1, wobei das erste Additiv A-1 2-Hydroxypyridin-N-oxid (2-HOPO) ist.

3. Verfahren gemäß einem beliebigen der vorhergehenden Ansprüche, wobei das Carbodiimid-Kupplungsmittel ausgewählt ist aus der Gruppe bestehend aus N,N'-Diisopropylcarbodiimid (DIC), N,N'-Dicyclohexylcarbodiimid (DCC) und N-(3-Dimethylaminopropyl)-N'-ethylcarbodiimid (EDC) oder Mischungen davon.

4. Verfahren gemäß einem beliebigen der vorhergehenden Ansprüche, wobei die freie primäre oder sekundäre aliphatische Aminogruppe der ersten Komponente C-1 eine alpha-Aminogruppe einer alpha-Aminosäure-Einheit ist.

5. Verfahren gemäß einem beliebigen der vorhergehenden Ansprüche, wobei die zweite Komponente C-2 eine chirale alpha-Aminosäure ist, bevorzugt wobei die zweite Komponente C-2 ausgewählt ist aus der Gruppe bestehend aus Histidin, Cystein, einem alpha-Arylglycin und einem alpha-Heteroarylglycin.

6. Verfahren gemäß einem beliebigen der Ansprüche 1 bis 4, wobei die zweite Komponente C-2 ein Peptid ausgewählt aus der Gruppe bestehend aus einem Dipeptid und einem Tripeptid ist.

7. Verfahren gemäß einem beliebigen der vorhergehenden Ansprüche, wobei die zweite Komponente C-2 eine primäre oder sekundäre aliphatische Aminogruppe umfasst, die durch eine Schutzgruppe des Fluorenylmethyloxycarbonyl-Typs geschützt ist.

8. Verfahren gemäß einem beliebigen der Ansprüche 1 bis 5 und 7, wobei die zweite Komponente C-2 Fmoc-His(1-Trt)-OH ist.

9. Verfahren gemäß einem beliebigen der Ansprüche 1 bis 5 und 7, wobei die zweite Komponente C-2 Fmoc-Cys(Trt)-OH ist.

10. Verfahren gemäß einem beliebigen der vorhergehenden Ansprüche, wobei in Schritt (c):
- die zweite Komponente C-2 in einer molaren Gesamtmenge von 1,0 bis 5,0 Äquivalenten verwendet wird,
- das Carbodiimid-Kupplungsmittel in einer molaren Gesamtmenge von 1,0 bis 6,0 Äquivalenten verwendet wird,
- das erste Additiv A-1 in einer molaren Gesamtmenge von 1,0 bis 6,0 Äquivalenten verwendet wird, und
- das zweite Additiv A-2 in einer molaren Gesamtmenge von 1,0 bis 5,0 Äquivalenten verwendet wird,
wobei alle Äquivalente in Bezug auf die molare Gesamtmenge der ersten Komponente C-1 angegeben sind.

11. Verfahren gemäß einem beliebigen der vorhergehenden Ansprüche, wobei die erste Komponente C-1 kovalent an einen Träger gebunden ist.

12. Verfahren gemäß einem beliebigen der vorhergehenden Ansprüche, wobei das Verfahren ferner den folgenden Schritt (d) umfasst:
(d) Entschützen einer geschützten primären oder sekundären aliphatischen Aminogruppe in dem in Schritt (c) erhaltenen Peptid P-1 des ersten Zyklus, um ein Peptid P-1' des ersten Zyklus zu erhalten, das eine freie primäre oder sekundäre aliphatische Aminogruppe umfasst.

13. Verfahren gemäß Anspruch 12, wobei das Zielpeptid ein Peptid P-(n+1) des (n+1)-ten Zyklus umfasst, das von dem in Schritt (d) erhaltenen Peptid P-1' des ersten Zyklus durch Durchführen von n Iterationen weiterer Kondensationszyklen abgeleitet ist, wobei n eine ganze Zahl von 1 bis 100 ist, und wobei jeder weitere Kondensationszyklus die folgenden Schritte (e) bis (g) umfasst:
(e) Bereitstellen einer weiteren Komponente C-(n+2), die ausgewählt ist aus der Gruppe bestehend aus einer Aminosäure und einem Peptid, das eine freie Carboxylgruppe oder eine aktivierte Carboxylgruppe umfasst und mindestens eine geschützte primäre oder sekundäre aliphatische Aminogruppe umfasst, wobei jegliche primären oder sekundären aliphatischen Aminogruppen und jegliche anderen Carboxylgruppen in C-(n+2) geschützt sind,
(f) Kondensieren des in Schritt (d) erhaltenen Peptids P-1' des ersten Zyklus oder, wenn bereits eine oder mehrere Iterationen weiterer Kondensationszyklen durchgeführt wurden, des in Schritt (g) des vorhergehenden Kondensationszyklus erhaltenen Peptids P-(n+1)' des (n+1)-ten Zyklus und der weiteren Komponente C (n+2) aus Schritt (e) unter geeigneten Bedingungen, um eine Amidbindung zwischen der freien primären oder sekundären aliphatischen Aminogruppe des Peptids P-1' des ersten Zyklus von Schritt (d) oder des Peptids P-(n+1)' des (n+1)-ten Zyklus von Schritt (g) des vorhergehenden Kondensationszyklus und der freien oder aktivierten Carboxylgruppe der weiteren Komponente C-(n+2) von Schritt (e) zu bilden, um ein Peptid P-(n+1) des (n+1)-ten Zyklus zu erhalten, und
(g) Entschützen einer geschützten primären oder sekundären aliphatischen Aminogruppe in dem in Schritt (f) erhaltenen Peptid P-(n+1) des (n+1)-ten Zyklus, um ein Peptid P-(n+1)' des (n+1)-ten Zyklus zu erhalten, das eine freie primäre oder sekundäre aliphatische Aminogruppe umfasst,
wobei die weitere Komponente C-(n+2) unabhängig für jede Iteration n von weiteren Kondensationszyklen ausgewählt wird, und
wobei im letzten Kondensationszyklus der Schritt (g) ausgelassen werden kann.

14. Verfahren gemäß Anspruch 13, wobei
- die eine primäre oder sekundäre aliphatische Aminogruppe, die in Schritt (d) entschützt wird, eine alpha-Aminogruppe einer alpha-Aminosäure-Einheit ist,
- die eine primäre oder sekundäre aliphatische Aminogruppe, die in Schritt (g) jedes weiteren Kondensationszyklus entschützt wird, eine alpha-Aminogruppe einer alpha-Aminosäure-Einheit ist, und
- die eine freie oder die eine aktivierte Carboxylgruppe jeder weiteren Komponente C-(n+2) eine alpha-Carboxylgruppe einer alpha-Aminosäure-Einheit ist.

15. Verfahren gemäß einem beliebigen der Ansprüche 13 und 14, wobei die Amino-Schutzgruppe, die bei der Entschützung einer geschützten primären oder sekundären aliphatischen Aminogruppe in Schritt (d) und Schritt (g) entfernt wird, eine Schutzgruppe vom Fluorenylmethyloxycarbonyl-Typ ist.

16. Verfahren gemäß einem beliebigen der Ansprüche 11 bis 15, wobei das Verfahren ferner einen Schritt der Abspaltung des Trägers und gegebenenfalls einer oder mehrerer Schutzgruppen vom Zielpeptid umfasst, um ein vom Träger abgespaltenes Zielpeptid zu erhalten, woran sich gegebenenfalls ein Schritt der Reinigung des vom Träger abgespaltenen Zielpeptids anschließen kann, um ein gereinigtes, vom Träger abgespaltenes Zielpeptid zu erhalten.

17. Verfahren gemäß Anspruch 16, wobei das Verfahren ferner einen Schritt des chemischen Modifizierens des Zielpeptids, des vom Träger abgespaltenen Zielpeptids oder des gereinigten vom Träger abgespaltenen Zielpeptids umfasst, um ein modifiziertes Zielpeptid zu erhalten, auf den gegebenenfalls ein Schritt der Reinigung des modifizierten Zielpeptids folgen kann, um ein gereinigtes modifiziertes Zielpeptid zu erhalten.

18. Verfahren gemäß einem der Ansprüche 11 bis 17, wobei der Träger in jeglichem der verwendeten Lösungsmittel unlöslich ist oder wobei der Träger in den für die Kondensations-, Entschützungs- und Spaltungs-Schritte verwendeten Lösungsmitteln löslich ist.

19. Zusammensetzung umfassend:
- eine erste Komponente C-1 ausgewählt aus der Gruppe bestehend aus einer Aminosäure und einem Peptid und die eine freie primäre oder sekundäre aliphatische Aminogruppe umfasst, wobei jegliche anderen primären oder sekundären aliphatischen Aminogruppen und jegliche Carboxylgruppen in C-1 geschützt sind,
- eine zweite Komponente C-2 ausgewählt aus der Gruppe bestehend aus einer chiralen alpha-Aminosäure und einem Peptid und die eine freie Carboxylgruppe umfasst, die die alpha-Carboxylgruppe einer chiralen alpha-Aminosäure-Einheit ist, die einen an das alpha-Kohlenstoffatom gebundenen Wasserstoff-Substituenten umfasst, wobei jegliche primären oder sekundären aliphatischen Aminogruppen und jegliche anderen Carboxylgruppen in C-2 geschützt sind,
- ein Carbodiimid-Kupplungsmittel,
- ein erstes Additiv A-1 der Formel A-I: 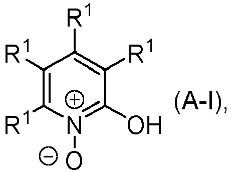 wobei in Formel A-I,
R¹ bei jedem Auftreten unabhängig ausgewählt ist aus der Gruppe bestehend aus Wasserstoff, C₁-C₅-Alkyl und einem elektronenziehenden Substituenten,
- ein zweites Additiv A-2 ausgewählt aus der Gruppe bestehend aus Ethyl-2-cyano-2-(hydroxyimino)acetat (Oxyma), 1-Hydroxybenzotriazol (HOBt), 1-Hydroxy-7-azabenzotriazol (HOAt), 5(Hydroxyimino)-1,3-dimethylpyrimidin-2,4,6(1H,3H,5H)-trion (Oxyma-B) und Ethyl-1-hydroxy-1,2,3-triazol-4-carboxylat (HOCt) oder Mischungen davon, und
- ein oder mehr Lösungsmittel,
wobei:
- die zweite Komponente C-2 in einer molaren Gesamtmenge von 1,0 bis 5,0 Äquivalenten vorhanden ist,
- das Carbodiimid-Kupplungsmittel in einer molaren Gesamtmenge von 1,0 bis 6,0 Äquivalenten vorhanden ist,
- das erste Additiv A-1 in einer molaren Gesamtmenge von 1,0 bis 6,0 Äquivalenten vorhanden ist, und
- das zweite Additiv A-2 in einer molaren Gesamtmenge von 1,0 bis 5,0 Äquivalenten vorhanden ist,
wobei alle Äquivalente in Bezug auf die molare Gesamtmenge der ersten Komponente C-1 angegeben sind.

## Revendications

1. Procédé pour la synthèse d'un peptide cible comprenant un peptide de premier cycle P-1, le procédé comprenant les étapes suivantes :
(a) la fourniture d'un premier composant C-1 qui est choisi dans le groupe constitué par un acide aminé et un peptide et qui comprend un groupe amino aliphatique primaire ou secondaire libre, tout autre quelconque groupe amino aliphatique primaire ou secondaire et tout groupe carboxyle dans C-1 étant protégés,
(b) la fourniture d'un deuxième composant C-2 qui est choisi dans le groupe constitué par un acide alpha-aminé chiral et un peptide et qui comprend un groupe carboxyle libre, qui est le groupe alpha-carboxyle d'un groupement d'acide alpha-aminé chiral comprenant un substituant hydrogène lié à l'atome de carbone alpha, tout groupe amino aliphatique primaire ou secondaire et tout autre groupe carboxyle dans C-2 étant protégés, et
(c) la condensation du premier composant C-1 de l'étape (a) et du deuxième composant C-2 de l'étape (b) en la présence de :
- un agent de couplage de type carbodiimide,
- un premier additif A-1 de formule A-I : 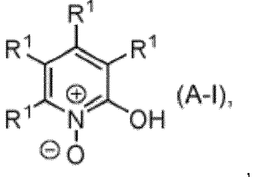 dans la formule A-I,
R¹ étant en chaque occurrence indépendamment choisi dans le groupe constitué par hydrogène, C₁₋₅-alkyle et un substituant électroattracteur,
- un deuxième additif A-2 choisi dans le groupe constitué par 2-cyano-2-hydroxyiminoacétate d'éthyle (Oxyma), 1-hydroxybenzotriazole (HOBt), 1-hydroxy-7-azabenzotriazole (HOAt), 5-(hydroxyimino)-1,3-diméthylpyrimidine-2,4,6(1H,3H,5H)-trione (Oxyma-B) et 1-hydroxy-1,2,3-triazole-4-carboxylate d'éthyle (HOCt) ou des mélanges correspondants, et
- un solvant de premier cycle,
de sorte à former une liaison amide entre le groupe amino aliphatique primaire ou secondaire du premier composant C-1 et le groupe alpha-carboxyle libre du deuxième composant C-2, pour obtenir un peptide de premier cycle B-1,
dans l'étape (c), le deuxième additif A-2 étant utilisé en une quantité molaire totale de 1,0 à 5,0 équivalents par rapport à la quantité molaire totale du premier composant C-1.

2. Procédé selon la revendication 1, le premier additif A-1 étant le N-oxyde de 2-hydroxypyridine (2-HOPO).

3. Procédé selon l'une quelconque des revendications précédentes, l'agent de couplage de type carbodiimide étant choisi dans le groupe constitué par N,N'diisopropylcarbodiimide (DIC), N,N'-dicyclohexylcarbodiimide (DCC) et N(3-diméthylaminopropyl)-N'-éthylcarbodiimide (EDC) ou des mélanges correspondants.

4. Procédé selon l'une quelconque des revendications précédentes, le groupe amino aliphatique primaire ou secondaire libre du premier composant C-1 étant un groupe alpha-amino d'un groupement d'acide alpha-aminé.

5. Procédé selon l'une quelconque des revendications précédentes, le deuxième composant C-2 étant un acide alpha-aminé chiral, préférablement le deuxième composant C-2 étant choisi dans le groupe constitué par l'histidine, la cystéine, une alpha-arylglycine et une alpha-hétéroarylglycine.

6. Procédé selon l'une quelconque des revendications 1 à 4, le deuxième composant C-2 étant un peptide choisi dans le groupe constitué par un dipeptide et un tripeptide.

7. Procédé selon l'une quelconque des revendications précédentes, le deuxième composant C-2 comprenant un groupe amino aliphatique primaire ou secondaire qui est protégé par un groupe protecteur de type fluorénylméthyloxycarbonyle.

8. Procédé selon l'une quelconque des revendications 1 à 5 et 7, le deuxième composant C-2 étant Fmoc-His(1-Trt)-OH.

9. Procédé selon l'une quelconque des revendications 1 à 5 et 7, le deuxième composant C-2 étant Fmoc-Cys(Trt)-OH.

10. Procédé selon l'une quelconque des revendications précédentes, dans l'étape (c) :
- le deuxième composant C-2 étant utilisé en une quantité totale molaire de 1,0 à 5,0 équivalents,
- l'agent de couplage de type carbodiimide étant utilisé en une quantité totale molaire de 1,0 à 6,0 équivalents,
- le premier additif A-1 étant utilisé en une quantité totale molaire de 1,0 à 6,0 équivalents,
- le deuxième additif A-2 étant utilisé en une quantité totale molaire de 1,0 à 5,0 équivalents,
tous les équivalents étant donnés par rapport à la quantité molaire totale du premier composant C-1.

11. Procédé selon l'une quelconque des revendications précédentes, le premier composant C-1 étant lié de manière covalente à un support.

12. Procédé selon l'une quelconque des revendications précédentes, le procédé comprenant en outre l'étape suivante (d) :
(d) déprotection d'un groupe amino aliphatique primaire ou secondaire protégé dans le peptide de premier cycle P-1 obtenu dans l'étape (c), pour obtenir un peptide de premier cycle P-1' comprenant un groupe amino aliphatique primaire ou secondaire libre.

13. Procédé selon la revendication 12, le peptide cible comprenant un peptide de (n + 1)^{ème} cycle P-(n + 1) issu du peptide de premier cycle P-1' obtenu dans l'étape (d) en réalisant n itérations de cycles de condensation supplémentaires, n étant un entier de 1 à 100, et chaque cycle de condensation supplémentaire comprenant les étapes suivantes (e) à (g) :
(e) fourniture d'un composant supplémentaire C-(n + 2) choisi dans le groupe constitué par un acide aminé et un peptide comprenant un groupe carboxyle libre ou un groupe carboxyle activé, et comprenant au moins un groupe amino aliphatique primaire ou secondaire protégé, tout groupe amino aliphatique primaire ou secondaire et tout autre groupe carboxyle dans C-(n + 2) étant protégés,
(f) condensation du peptide de premier cycle P-1' obtenu dans l'étape (d) ou, si une ou plusieurs itérations de cycles de condensation supplémentaires ont déjà été réalisés, du peptide de (n + 1)^{ème} cycle P-(n + 1)' obtenu dans l'étape (g) du cycle de condensation précédent, et du composant supplémentaire C-(n + 2) de l'étape (e) dans des conditions appropriées, pour former une liaison amide entre le groupe amino aliphatique primaire ou secondaire libre du peptide de premier cycle P-1' de l'étape (d) ou du peptide de (n + 1)^{ème} cycle P-(n + 1)' de l'étape (g) du cycle de condensation précédent et le groupe carboxyle libre ou activé du composant supplémentaire C-(n + 2) de l'étape (e), pour obtenir un peptide de (n + 1)^{ème} cycle P-(n + 1), et
(g) déprotection d'un groupe amino aliphatique primaire ou secondaire protégé dans le peptide de (n + 1)^{ème} cycle P-(n + 1) obtenu dans l'étape (f), pour obtenir un peptide de (n + 1)^{ème} cycle P-(n + 1)' comprenant un groupe amino aliphatique primaire ou secondaire libre,
le composant supplémentaire C-(n + 2) étant choisi indépendamment pour chaque itération n de cycles de condensation supplémentaires, et
dans le cycle de condensation final, l'étape (g) pouvant être omise.

14. Procédé selon la revendication 13,
- ledit groupe amino aliphatique primaire ou secondaire qui est déprotégé dans l'étape (d) étant un groupe alpha-amino d'un groupement d'acide alpha-aminé,
- ledit groupe amino aliphatique primaire ou secondaire qui est déprotégé dans l'étape (g) de chaque cycle de condensation supplémentaire étant un groupe alpha-amino d'un groupement d'acide alpha-aminé, et
- ledit groupe carboxyle libre ou ledit groupe carboxyle activé de chaque composant supplémentaire C-(n + 2) étant un groupe alpha-carboxyle d'un groupement d'acide alpha-aminé.

15. Procédé selon l'une quelconque des revendications 13 et 14, le groupe protecteur d'amino qui est éliminé lors de la déprotection d'un groupe amino aliphatique primaire ou secondaire protégé dans l'étape (d) et l'étape (g) étant un groupe protecteur de type fluorénylméthyloxycarbonyle.

16. Procédé selon l'une quelconque des revendications 11 à 15, le procédé comprenant en outre une étape de clivage du support et éventuellement d'un ou plusieurs groupes protecteurs du peptide cible pour obtenir un peptide cible clivé du support, qui peut éventuellement être suivie par une étape de purification du peptide cible clivé du support pour obtenir un peptide cible clivé du support purifié.

17. Procédé selon la revendication 16, le procédé comprenant en outre une étape de modification chimique du peptide cible, du peptide cible clivé du support ou du peptide cible clivé du support purifié pour obtenir un peptide cible modifié, qui peut éventuellement être suivie par une étape de purification du peptide cible modifié pour obtenir un peptide cible modifié purifié.

18. Procédé selon l'une quelconque des revendications 11 à 17, le support étant insoluble dans l'un quelconque des solvants utilisés ou le support étant soluble dans les solvants utilisés pour les étapes de condensation, de déprotection et de clivage.

19. Composition comprenant :
- un premier composant C-1 qui est choisi dans le groupe constitué par un acide aminé et un peptide et qui comprend un groupe amino aliphatique primaire ou secondaire libre, tout autre quelconque groupe amino aliphatique primaire ou secondaire et tout groupe carboxyle dans C-1 étant protégés,
- un deuxième composant C-2 qui est choisi dans le groupe constitué par un acide alpha-aminé chiral et un peptide et qui comprend un groupe carboxyle libre, qui est le groupe alpha-carboxyle d'un groupement d'acide alpha-aminé chiral comprenant un substituant hydrogène lié à l'atome de carbone alpha, tout groupe amino aliphatique primaire ou secondaire et tout autre groupe carboxyle dans C-2 étant protégés,
- un agent de couplage de type carbodiimide,
- un premier additif A-1 de formule A-I : 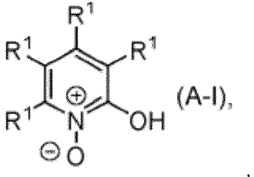 dans la formule A-I,
R¹ étant en chaque occurrence indépendamment choisi dans le groupe constitué par hydrogène, C₁₋₅-alkyle et un substituant électroattracteur,
- un deuxième additif A-2 choisi dans le groupe constitué par 2-cyano-2-hydroxyiminoacétate d'éthyle (Oxyma), 1-hydroxybenzotriazole (HOBt), 1-hydroxy-7-azabenzotriazole (HOAt), 5-(hydroxyimino)-1,3-diméthylpyrimidine-2,4,6(1H,3H,5H)-trione (Oxyma-B) et 1-hydroxy-1,2,3-triazole-4-carboxylate d'éthyle (HOCt) ou des mélanges correspondants, et
- un ou plusieurs solvants,
- le deuxième composant C-2 étant présent en une quantité totale molaire de 1,0 à 5,0 équivalents,
- l'agent de couplage de type carbodiimide étant présent en une quantité totale molaire de 1,0 à 6,0 équivalents,
- le premier additif A-1 étant présent en une quantité totale molaire de 1,0 à 6,0 équivalents,
- le deuxième additif A-2 étant présent en une quantité totale molaire de 1,0 à 5,0 équivalents,
tous les équivalents étant donnés par rapport à la quantité molaire totale du premier composant C-1.
